# EUROPEAN PATENT APPLICATION

(11) **EP 1 864 984 A1**
(43) Date of publication of application: **12.12.2007**
(21) Application number: 06730569.8
(22) Date of filing: 30.03.2006
(51) Int. Cl.: C07D 471/20, A61K 31/499, A61K 31/506, A61K 31/527, A61P 7/02, C07D 498/20, C07D 513/20

(54) **TRICYCLIC SPIRO COMPOUND COMPRISING ACYL GROUP BOUND TO NITROGEN ATOM IN THE RING**

(30) Priority: 31.03.2005 JP 2005105444
(71) Applicant: MOCHIDA PHARMACEUTICAL CO., LTD., Shinjuku-ku Tokyo 160-8515 (JP)
(72) Inventor: NISHIDA, Hidemitsu, Tokyo 1608515 (JP); OHKOUCHI, Munetaka, Tokyo 1608515 (JP)
(74) Representative: Grünecker, Kinkeldey, Stockmair & Schwanhäusser Anwaltssozietät
(86) International application number: PCT/JP2006/306621
(87) International publication number: WO 2006/106804

(57) **Abstract**

It is intended to provide an anticoagulant that has an extremely excellent FXa inhibitory action and an extremely weak hERG channel inhibitory action and can be orally administered, which is a compound represented by the following formula (I) or a pharmaceutically acceptable salt thereof.

## Description

### TECHNICAL FIELD

This invention relates to an orally administrable tricyclic compound having spiro union or a salt thereof which is useful as a drug, and in particular, as an inhibitor of activated blood coagulation factor X (hereinafter referred to as FXa), and which exhibits potent anticoagulation action with reduced side effects.

### BACKGROUND ART

With the westernization of the life style and the increase in the number of aged people, ischemic heart diseases and other pathology of heart and blood vessels are increasing year after year. In particular, thromboembolic diseases such as myocardial infarction, cerebral thrombosis, pulmonary embolism, and peripheral arterial and venous occlusive disease are increasing each year and treatment of such diseases has become a serious social issue. In the treatment and prevention of such thrombosis, anticoagulation therapy has been playing an important role in internal medicine together with antiplatelet therapy and fibrinolytic therapy. For the treatment and prevention of thrombosis, safety that permits long-term drug administration and the development of a reliable and appropriate anticoagulant activity are essential.

Heretofore, anticoagulants such as warfarin and heparin have been used in order to prevent and treat thrombosis caused by hypercoagulability. However, such use of the anticoagulants has been pointed to be associated with various demerits including the risk of bleeding and interactions with other drugs. Warfarin is extensively used in the world as the solo peroral anticoagulant. However, due to its characteristics based on the mechanism of action, the concentration range for the development of efficacy is narrow and yet it takes a long time to develop efficacy and the half-life in blood is as long as 36 hours; what is more, for several reasons such as the great individual difference of effective dose, it is difficult to control the anticoagulability of warfarin (N. Eng. J. Med. 324 (26) 1865-1875, 1991) and frequent monitoring is required in order to prevent bleeding as a side effect. In addition, warfarin also has many other side effects such as nausea, vomiting, diarrhea, and alopecia; thus, warfarin is a drug that involves considerable difficulty in clinical use. On the other hand, heparin is extensively used in the world as an intravenously administrable anticoagulant. However, since it is a direct inhibitor of thrombin, heparin has a high risk of bleeding and needs frequent monitoring as in the case of warfarin; what is more, due to its characteristics based on the mechanism of action, adequate coagulation inhibiting effect is not expected at a lowered antithrombin III level; thus, heparin is a drug that involves considerable difficulty in clinical use. In view of such situation, improved anticoagulants have been desired that has none of the defects inherent in warfarin and heparin.

The blood coagulation cascade is a chain reaction involving limited protein hydrolysis triggered by activation of the extrinsic coagulation cascade or the intrinsic coagulation cascade, and once the cascade is activated, the reaction is amplified like an avalanche. Since the final stage of the blood coagulation cascade is thrombin-mediated conversion of fibrinogen to fibrin, efforts have recently been made to develop thrombin inhibitors; however, drugs that directly inhibit thrombin are known to increase the risk of bleeding.

FXa is a key enzyme, which is located in the upstream of the thrombin in the coagulation cascade, and also at the junction of the extrinsic and the intrinsic coagulation cascade. One molecule of FXa is known to produce about a hundred molecules of thrombin per minute. Therefore, an FXa inhibitor can potentially inhibit the coagulation cascade more efficiently than a thrombin inhibitor (Thrombosis Research, vol. 19, pages 339-349, 1980; Mebio, vol. 14, No. 8, 1997).

Patent Documents 1 and 2 disclose use of tricyclic spiro compounds as an FXa inhibitor, in which these compounds are used in order to provide an effective FXa inhibitor which can be orally administered. These compounds have a structure wherein nitrogen atom in piperidine moiety of the tricyclic spiro skeleton (for example, nitrogen at position 1' of spiro[imidazo[1,2-a]pyrazine-2(3H),4'-piperidin]-5(1H)-one skeleton) is substituted with a heterocyclic group such as 4-pyridyl group, and these documents do not disclose a compound having an acyl group bonded to the nitrogen atom.

However, low molecular weight FXa inhibitors are still under development, and development of some inhibitors such as DX-9065a, YM-60868, JTV-803, and DPC-906 is slowing down. As a consequence, no low molecular weight FXa inhibitor has been commercialized.

In the development of pharmaceutical products, the desired pharmacological activity is not the sole requirement. Another requirement is that strict criteria be met in various aspects including absorption, distribution, metabolism and excretion. For example, the drugs are required to pass various examinations for drug interaction, desensitization or tolerance, absorption from digestive tract in the oral administration, transfer rate to small intestine, absorption rate and first pass effect, organ barrier, protein binding, induction of drug metabolizing enzyme, excretion pathway and body clearance, administration method (site, method, and purpose of administration), and the like, and a drug meeting all such requirements is seldom discovered.

In recent years, proarrhythmic activity due to a drug, in particular, the prolongation of QT interval on an electrocardiogram has been attracting attention. One method known in the art that can be used in estimating the QT prolongation effect is evaluation of actions on hERG (human ether-a-go-go related gene) channel. Discovery of drugs having extremely weak effects on the hERG current whose importance in the generation of side effects on the heart is recognized is one of the most important issues in the drug development.

The anticoagulants also share such general challenge of the drug development. In the case of the FXa inhibitor, circumvention of the problem of the side effects associated with the oral administration of the warfarin as well as risk of bleeding based on the thrombin inhibition as found in the case of heparin whose administration is only accomplished by intravenous injection is required.

Patent Document 1: WO 2001/02397
Patent Document 2: WO 2002/53568

### DISCLOSURE OF THE INVENTION

### PROBLEMS TO BE SOLVED BY THE INVENTION

In view of such situation, there is a demand for an anticoagulant drug which exhibits high safety and excellent effectivity with reduced side effects. To be more specific, there is a high demand for an anticoagulant which can be orally administered to mammals including humans, and in particular, which can be readily used in clinical practice, and which has realized at least one of avoidance of interaction with other drugs, reduced side effects including reduced risk of bleeding, improved dose response, extremely low inhibitory action of hERG current, and the like.

### MEANS TO SOLVE THE PROBLEMS

The present invention provides a pharmaceutical composition containing, as an effective component, a tricyclic spiro compound represented by the formula (I) and having an acyl group bonded to the ring member nitrogen atom, or a salt or a solvate thereof, or its derivative or a pharmaceutically acceptable salt or a solvate thereof.

### EFFECTS OF THE INVENTION

In order to solve the problems as described above, the inventors of the present invention conducted an intensive study to provide a compound having an excellent FXa inhibitory action, and found that the compound having spiro skeleton and represented by the formula (I) exhibits excellent FXa inhibitory action with extremely weak hERG channel inhibitory action, and that this compound is a potent candidate for use as an orally administerable anticoagulant.
Accordingly, the pharmaceutical composition of the present invention is an orally administerable anticoagulant exhibiting excellent FXa inhibitory action and having an extremely weak hERG channel inhibitory action.

### BEST MODE FOR CARRYING OUT THE INVENTION

As will be described in the following aspects, the present invention provides a tricyclic spiro compound represented by the formula (I) and having an acyl group bonded to the ring member nitrogen atom and its salts, a pharmaceutical composition containing such compound or its salt as an effective component, use of such compound or its salt for medical application.
Next, various aspects of the present invention are described. In the compound of the present invention, "C₁₋₆", for example, means that "the group is a straight chain or branched group containing 1 to 6 carbon atoms" unless otherwise noted. In the case of a cyclic group, "C₁₋₆" 6" denotes "the number of ring member carbon atoms".

The compound of the formula (I) according to the present invention is not particularly limited for its molecular weight. The molecular weight, however, is preferably up to 1000, and more preferably up to 700 (and otherwise stated, the total number of carbon atoms constituting the compound is less than 40). Such limitation of molecular weight is routinely employed for identifying the structure of the compound as a major limiting factor in addition to the pharmacologically characteristic basic skeleton in recent drug design. In particular, the molecular weight is preferably limited to the range of up to 1000 when oral absorbability of the drug is taken into consideration.

### ASPECTS OF THE INVENTION

### [1] First aspect of the present invention

The compound of the present invention is a compound represented by the following formula (I) or its pharmaceutically acceptable salt. 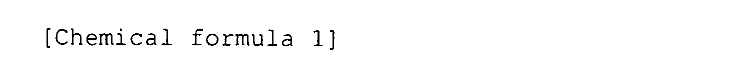 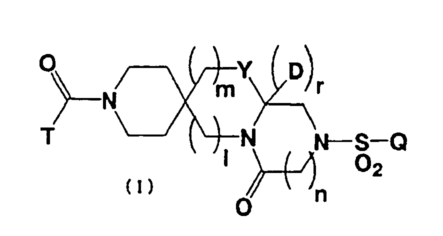 In the formula, T is an optionally substituted aliphatic hydrocarbon group; an optionally substituted 3-to 4-membered alicyclic hydrocarbon group, an optionally substituted C₂₋₆ alkanoyl group, trifluoromethyl group, furyl group, or pyrrolyl group; D is hydrogen atom, -CO-R⁵ (wherein R⁵ is hydrogen atom or a substituent), or an optionally substituted C₁₋₆ alkyl group; Y is oxygen atom, -S(O)_{y}-, or optionally substituted imino group (-NH-); Q is a hydrocarbon group or a heterocyclic group which optionally has a substituent; 1, m, n, and y are independently an integer selected from 0, 1, and 2 with the proviso that 1 and m are not simultaneously 0; and r is an integer of 0 or 1; and the three rings including the N-containing ring having the T-CO- bonded thereto, the Y-containing ring, and the N-containing ring having the -SO₂-Q bonded thereto may be optionally substituted.

Next, various groups included in the formula (I) are described in detail.
[1-1] In the compound of the formula (I), Q is a hydrocarbon group or a heterocyclic group, and these groups may optionally have substituents. Exemplary "hydrocarbon groups" within the definition of Q include aliphatic hydrocarbon groups, alicyclic hydrocarbon groups, and aryl groups, and the preferred is an aryl group.
Exemplary "aliphatic hydrocarbon groups" include straight- or branched-chain hydrocarbon groups, for example, alkyl groups, alkenyl groups, and alkynyl groups.

Exemplary "alkyl groups" include C₁₋₁₀ (and more preferably C₁₋₆) alkyl groups, for example, methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl, 1-methyl-heptyl, and n-nonyl.
Exemplary "alkenyl groups" include C₂₋₆ alkenyl groups, for example, vinyl, allyl, isopropenyl, 2-methylallyl, butenyl, pentenyl, and hexenyl.
Exemplary "alkynyl groups" include C₂₋₆ alkynyl groups, for example, ethynyl, 1-propynyl, 2-propynyl, butynyl, pentynyl, and hexynyl.

Exemplary "alicyclic hydrocarbon groups" include saturated and unsaturated alicyclic hydrocarbon groups, for example, cycloalkyl group, cycloalkenyl group, and cycloalkanedienyl group.
Exemplary "cycloalkyl groups" include C₃₋₉ cycloalkyl groups, for example, cyclopropyl, cyclobutyl, cyclopentyl, cyclohexyl, cycloheptyl, cyclooctyl, and cyclononyl.
Exemplary "cycloalkenyl groups" include C₃₋₆ cycloalkenyl groups, for example, 1-cyclopropen-1-yl, 1-cyclobuten-1-yl, 1-cyclopenten-1-yl, 2-cyclopenten-1-yl, 3-cyclopenten-1-yl, and 1-cyclohexen-1-yl.
Exemplary "cycloalkanedienyl groups" include C₄₋₆ cycloalkanedienyl groups, for example, 2,4-cyclopentadien-1-yl and 2,5-cyclohexadien-1-yl.
Exemplary "aryl groups" include C₆₋₁₄ aryl groups, for example, phenyl, naphthyl, biphenylyl, 2-anthryl, phenanthryl, acenaphthyl, 5,6,7,8-tetrahydronaphthalenyl, and partially hydrogenated fused aryl groups such as indanyl and tetrahydronaphtyl, and among these, the preferred are phenyl, 2-naphthyl, and 1-naphthyl.

Exemplary heterocyclic groups of the "optionally substituted heterocyclic groups" in Q include aromatic heterocyclic groups, and saturated and unsaturated non-aromatic heterocyclic groups. Exemplary such heterocyclic groups include those having a five- to fourteen-membered ring, and preferably, a five- to twelve-membered ring containing at least one heteroatom (preferably 1 to 4 heteroatoms) selected from N, O and S in addition to the carbon atoms.

Exemplary "aromatic heterocyclic groups" include monocyclic and fused heterocyclic groups. Preferable monocyclic aromatic heterocyclic groups are those containing 5 to 6 ring members, for example, pyrrolyl, furyl, thienyl, oxazolyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.
Preferable fused aromatic heterocyclic groups are those containing 8 to 12 ring members, for example, monovalent groups formed by condensation of the five- or six-membered aromatic ring as described above with one or more (preferably 1 to 2) aromatic rings (for example, benzene ring) followed by removal of hydrogen atom at an arbitrary position from the thus formed ring.

Exemplary such groups include indolyl, isoindolyl, 1H-indazolyl, benzofuranyl (-2-yl), isobenzofuranyl, benzothienyl(-2-yl), isobenzothienyl, benzindazolyl, benzoxazolyl(-2-yl), 1,2-benzisoxazolyl, benzothiazolyl(-2-yl), 1,2-benzisothiazolyl, 2H-benzopyranyl(-3-yl), (1H-)benzimidazolyl(-2-yl), 1H-benzotriazolyl, 4H-1,4-benzoxazinyl, 4H-1,4-benzothiazinyl, quinolyl, isoquinolyl, cinnolinyl, quinazolinyl, quinoxalinyl, phthalazinyl, naphthyridinyl, purinyl, pteridinyl, carbazolyl, carbolinyl, acridinyl, phenoxazinyl, phenothiazinyl, phenazinyl, phenoxazinyl, thianthrenyl, phenanthridinyl, phenanthrolinyl, indolizinyl, (9,5,6,7-) tetrahydrothiazolo(5, 4-c]pyridyl (-2-yl), (4,5,6,7-) tetrahydrothieno[3,2-c]pyridyl, (1,2,3,9-) tetrahydroisoquinolyl(-6-yl), thiazolo[5,4-c]pyridyl(-2-yl), pyrrolo[1,2-b]pyridazinyl, pyrazo[1,5-a]pyridyl, imidazo[1,2-a]pyridyl, imidazo[1,5-a]pyridyl, imidazo[1,2-b]pyridazinyl, imidazo[1,5-a]pyrimidinyl, 1,2,4-triazolo[4,3-a]pyridyl, 1,2,9-triazolo[9,3-b]pyridazinyl (Preferred embodiments are indicated in the brackets).
Exemplary "non-aromatic heterocyclic groups" include three- to eight-membered saturated and unsaturated non-aromatic heterocyclic groups, for example, azetidinyl, oxiranyl, oxetanyl, thietanyl, pyrrolidinyl, tetrahydrofuryl, thiolanyl, pyrazolinyl, pyrazolidinyl, piperidyl, tetrahydropyranyl, piperazinyl, morpholinyl, oxazolinyl, thiazolinyl, thiomorpholinyl, and quinuclidinyl.

Exemplary "substituents" of the "optionally substituted hydrocarbon group" or the "optionally substituted heterocyclic group" in Q include (a) alkyl, alkenyl, alkynyl, aryl, cycloalkyl, and cycloalkenyl, (b) heterocyclic groups, (c) amino, (d) imidoyl, amidino, hydroxy, thiol, and oxo, (e) halogen atoms such as fluorine, chlorine, bromine, and iodine, cyano, and nitro, (f) carboxyl, and (g) carbamoyl, thiocarbamoyl, sulfonyl, sulfinyl, sulfide and acyl. Of the (a) to (g) as described above, the groups excluding (e) may further comprise a substituent.
The "optionally substituted hydrocarbon group" and the "optionally substituted heterocyclic group" in Q may be arbitrarily substituted with 1 to 5 such substituents. Such substituents (a) to (g) are described in further detail.

(a) The alkyl, alkenyl, alkynyl, aryl, cycloalkyl, and cycloalkenyl groups may be any of the "alkyl groups", "alkenyl groups", "alkynyl groups", "aryl groups", "cycloalkyl groups" and "cycloalkenyl groups" mentioned as examples of the "hydrocarbon group" for Q, and the preferred are C₁₋₆ alkyl groups, C₂₋₆ alkenyl groups, C₂₋₆ alkynyl groups, C₆₋₁₄ aryl groups, C₃₋₇ cycloalkyl groups and C₃₋₆ cycloalkenyl groups.
These groups may further include an optional substituent RI (wherein RI represents a group selected from a C₁₋₆ alkoxy, a C₁₋₆ alkoxycarbonyl, carboxyl, carbamoyl which is optionally mono- or di-substituted with a C₁₋₆ alkyl, a halogen, a C₁₋₆ alkyl, a halogenated C₁₋₆ alkyl, amino which is optionally mono- or di-substituted with a C₁₋₆ alkyl, a C₂₋₆ alkenoylamino, nitro, hydroxy, phenyl, phenoxy, benzyl, oxo, cyano, and amidino).

(b) The heterocyclic group may be any of the "aromatic heterocyclic groups" and "non-aromatic heterocyclic groups" mentioned as examples of the "heterocyclic group" for Q, and the preferred are (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", which contain 1 to 4 heteroatoms selected from nitrogen atom, oxygen atom and sulfur atom in addition to the carbon atoms.
These groups may further include an optional substituent RII (wherein RII represents a halogen atom such as fluorine, chlorine, bromine, or iodine, a C₁₋₆ alkyl group, a C₁₋₆ alkanoyl group, or benzoyl group).

(c) The "optionally substituted amino group" may be, for example, amino group which is optionally mono- or di-substituted with substituent RIII (wherein RIII represents a group selected from a C₁₋₆ alkyl, a C₁₋₆ alkanoyl, a C₂₋₆ alkenoyl, benzoyl, and a C₁₋₆ alkoxycarbonyl which is optionally substituted with 1 to 5 halogen atoms), or three- to eight-membered monocyclic amino group which is optionally substituted with a group selected from a C₁₋₆ alkyl, a C₇₋₁₀ aralkyl, and a C₆₋₁₀ aryl.

(d) Exemplary substituents in the "optionally substituted imidoyl group, the optionally substituted amidino group, the optionally substituted hydroxy group, and the optionally substituted thiol group" include RIII (wherein RIII represents a group selected from a C₁₋₆ alkyl, a C₁₋₆ alkanoyl, a C₂₋₆ alkenoyl, benzoyl, and a C₁₋₆ alkoxycarbonyl which is optionally substituted with 1 to 5 halogen atoms) described in (c). Therefore, examples of (d) include a C₁₋₆ alkylimidoyl group, formimidoyl group or amidino group, a C₁₋₆ alkoxy group, benzyloxy group, a C₁₋₆ alkanoyloxy group, and oxo group.

(e) Halogen atoms such as fluorine, chlorine, bromine, and iodine, cyano group, and nitro group.

(f) The "optionally substituted carboxyl groups" include carboxyl group, C₁₋₆ alkoxycarbonyl groups, C₇₋₁₂ aryloxycarbonyl groups, and C₆₋₁₀ aryl-C₁₋₄ alkoxycarbonyl groups, and the aryl group in such (f) is optionally substituted with substituent RIV. RIV represents amino group which is optionally mono- or di-substituted with the substituent RII (wherein RII represents a halogen atom such as fluorine, chlorine, bromine, or iodine, a C₁₋₆ alkyl group, a C₁₋₆ alkanoyl group, or benzoyl group) of (b); a halogen atom; hydroxy group; nitro group; cyano group; a C₁₋₆ alkyl group which is optionally substituted with 1 to 5 halogen atoms; or an alkoxy group which is optionally substituted with 1 to 5 halogen atoms.

(g) The "optionally substituted carbamoyl group, the optionally substituted thiocarbamoyl group, the optionally substituted sulfonyl, the optionally substituted sulfinyl, the optionally substituted sulfide and the optionally substituted acyl group" are, for example, the groups represented by -CONR_{g}R_{g'}, -CSNR_{g}R_{g'}, -SO_{y}-R_{g}, or -CO-Rg, wherein:
Rg represents hydrogen atom or a substituent RV (wherein RV represents a C₁₋₆ alkyl, a C₃₋₆ cycloalkyl, a C₆₋₁₀ aryl, a C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered, fused aromatic heterocyclic group, and (iii) a three- to eight-membered saturated or unsaturated non-aromatic heterocyclic group, and the alkyl, the cycloalkyl, the aryl, the aralkyl, or the heterocyclic group is optionally further substituted with substituent RIV of (f));
R_{g'} is hydrogen atom or a group selected from C₁₋₆ alkyl groups, C₃₋₆ cycloalkyl groups, and C₇₋₁₀ aralkyl groups, and y is 0, 1, or 2.

In the compound of the formula (I), Q is preferably as described below.
[1-1-a] Examples of the "optionally substituted hydrocarbon group" or the "optionally substituted heterocyclic group" include:
(1) C₁₋₁₀ alkyl groups; (2) C₂₋₆ alkenyl groups; (3) C₂₋₆ alkynyl groups; (4) C₃₋₉ cycloalkyl groups; (5) C₃₋₆ cycloalkenyl groups; (6) C₄₋₆ cycloalkanedienyl groups; (7) C₆₋₁₄ aryl groups; and (8) a heterocyclic group containing 1 to 4 heteroatoms selected from oxygen atom, nitrogen atom and sulfur atom in addition to the carbon atoms which is any one of (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", and each group of the above (1) to (8) may be either unsubstituted or substituted with 1 to 5 substituents of the class selected from (a-1) to (g-1) as described below.

The classes are:
(a-1) : a C₁₋₆ alkyl group, a C₂₋₆ alkenyl group, a C₂₋₆ alkynyl group, a C₆₋₁₄ aryl group, a C₃₋₇ cycloalkyl group, and a C₃₋₆ cycloalkenyl group. These substituents are optionally further substituted with substituent RI (wherein RI represents a group selected from a C₁₋₆ alkoxy; a C₁₋₆ alkoxycarbonyl; carboxyl; carbamoyl which is optionally mono- or di-substituted with a C₁₋₆ alkyl; a halogen; a C₁₋₆ alkyl; a halogenated C₁₋₆ alkyl; amino which is optionally mono- or di-substituted with a C₁₋₆ alkyl; a C₂₋₆ alkenoylamino; nitro; hydroxy; oxo; cyano; and amidino).
(b-1) : a heterocyclic group containing 1 to 4 heteroatoms selected from oxygen atom, sulfur atom, and nitrogen atom in addition to the carbon atoms which is any one of (i) a "five- or six-membered, monocyclic aromatic heterocyclic group", (ii) an "eight- to twelve-membered, fused aromatic heterocyclic group", and (iii) a "three- to eight-membered, saturated or unsaturated, non-aromatic heterocyclic group". These heterocyclic groups are optionally further substituted with RII (wherein RII represents a halogen atom such as fluorine, chlorine, bromine, or iodine, a C₁₋₆ alkyl, a C₁₋₆ alkanoyl, or benzoyl).
(c-1): amino group which is optionally substituted with substituent RIII (wherein RIII represents a group selected from a C₁₋₆ alkyl, a C₁₋₆ alkanoyl, a C₂₋₆ alkenoyl, benzoyl, and a C₁₋₆ alkoxycarbonyl which is optionally substituted with 1 to 5 halogen atoms), and a three- to eight-membered monocyclic amino group which is optionally substituted with a group selected from a C₁₋₆ alkyl, a C₇₋₁₀ aralkyl and a C₆₋₁₀ aryl.
(d-1): an imidoyl group, amidino group, hydroxy group, and a thiol group. These substituents are optionally further substituted with groups selected from substituents RIII as described above in the (c-1).
(e-1): a halogen atom such as fluorine, chlorine, bromine, or iodine, cyano group, and nitro group.
(f-1): carboxyl group, a C₁₋₆ alkoxycarbonyl group, a C₇₋₁₂ aryloxycarbonyl group, and a C₆₋₁₀ aryl-C₁₋₄ alkoxycarbonyl group; and the aryl group in such (f-1) is optionally substituted with substituent RIV' (wherein RIV' represents amino which is optionally mono- or di-substituted with groups selected from RIII as described above in the (c-1) ; C₁₋₆ alkyl or C₁₋₆ alkoxy which is optionally substituted with 1 to 5 halogen atoms; a halogen atom; hydroxy; nitro; and cyano).
(g-1): a group -CONR_{g}R_{g'}, -CSNR_{g}R_{g'}, -CO-R_{g} or -SO_{y}-R_{g} wherein:
   Rg represents hydrogen atom or a substituent RV (wherein RV represents a C₁₋₆ alkyl, a C₃₋₆ cycloalkyl, a C₆₋₁₀ aryl, a C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered, fused aromatic heterocyclic group, and (iii) a three- to eight-membered, saturated or unsaturated, non-aromatic heterocyclic group, and the alkyl, the cycloalkyl, the aryl, the aralkyl, or the heterocyclic group is optionally further substituted with the substituent RIV as described in the (f-1)); Rg' is a group selected from hydrogen atom, C₁₋₆ alkyl groups, C₃₋₆ cycloalkyl groups, and C₇₋₁₀ aralkyl groups; and y is 0, 1, or 2.

Of the groups shown in (a-1) to (g-1) as described above, the "most preferable groups" are substituents including a C₁₋₆ alkyl, a C₂₋₆ alkenyl, a C₂₋₆ alkynyl, a halogen atom, a halogenated C₁₋₆ alkyl, cyano, amino, hydroxy, carbamoyl, a C₁₋₆ alkoxy, a C₂₋₆ alkenyloxy, a C₂₋₆ alkynyloxy, a C₁₋₆ alkylthio, a C₁₋₆ alkylsulfinyl, a C₁₋₆ alkylsulfonyl, a mono/di C₁₋₆ alkylamino, a C₁₋₆ alkoxycarbonyl, a C₂₋₆ alkanoyl, a C₂₋₆ alkanoylamino, a hydroxy-C₁₋₆ alkyl, a C₁₋₆ alkoxy-C₁₋₆ alkyl, a carboxy-C₁₋₆ alkyl, a C₁₋₆ alkoxycarbonyl-C₁₋₆ alkyl, a carbamoyl-C₁₋₆ alkyl, a N-(C₁₋₆)alkylcarbamoyl-C₁₋₆ alkyl, a N,N-di C₁₋₆ alkylcarbamoyl-C₁₋₆ alkyl, phenyl, phenoxy, phenylthio, phenylsulfinyl, phenylsulfonyl, benzyl, and benzoyl, and the aromatic ring in these substituents may be further substituted with 1 to 3 subustituents selected from a halogen atom, trifluoromethyl, cyano, hydroxy, amino, nitro, carboxyl, carbamoyl, a C₁₋₆ alkyl, a C₁₋₆ alkoxy, a mono/di C₁₋₆ alkylamino, a di-C₁₋₆ alkylcarbamoyl, a C₁₋₆ alkoxycarbonyl, a N-C₁₋₆ alkylcarbamoyl, a N,N-di C₁₋₆ alkylcarbamoyl, and a C₂₋₆ alkenoylamino.

[1-1-b] Preferably, Q is (1) a C₁₋₆ alkyl group, (2) a C₂₋₆ alkenyl group, (7) a C₆₋₁₄ aryl group, or (8) (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and each group in (1), (2), (7) and (8) is optionally further mono- or di-substituted at an arbitrary position with the substituent of the class selected from [1-1] (a-1) to (g-1) (and most preferably, from those listed as "most preferable groups").

[1-1-c] More preferably, Q is (1') or (2'): a C₁₋₆ alkyl group (most preferably a C₁₋₂ alkyl group) or a C₂₋₆ alkenyl group (most preferably a C₂ alkenyl group) substituted with a substituent selected from substituent (a-1): a C₆₋₁₄ aryl group and substituent (b-1): an aromatic group selected from (i) five- or six-membered monocyclic aromatic heterocyclic groups and (ii) eight- to twelve-membered, fused aromatic heterocyclic groups, which contain 1 to 4 heteroatoms selected from nitrogen atom, oxygen atom and sulfur atom in addition to the carbon atoms; (7'): a C₆₋₁₄ aryl group which is optionally substituted with 1 to 2 halogen atoms; or (8'): a heterocyclic group which is (i) a five- or six-membered, monocyclic, aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and wherein the carbon atoms are optionally mono- or di-substituted with a halogen atom.
The aromatic ring in the above substituent (1') or (2') is optionally further substituted with 1 to 3 substituents selected from halogen atoms, trifluoromethyl, cyano, hydroxy, amino, nitro, carboxyl, carbamoyl, a C₁₋₆ alkyl, a C₁₋₆ alkoxy, a mono/di C₁₋₆ alkylamino, a di C₁₋₆ alkylcarbamoyl, a C₁₋₆ alkoxycarbonyl, a N-C₁₋₆ alkylcarbamoyl, a N,N-di C₁₋₆ alkylcarbamoyl and a C₂₋₆ alkenoylamino.
The aromatic ring in the substituents (7') and (8') is also optionally mono- or di-substituted at arbitrary position with the substituent of the class selected from (a-1) to (g-1) (and most preferably, from those listed as "most preferable groups").

[1-1-d] Still more preferably, Q is phenyl group, benzyl group, phenethyl group, styryl group, 1-naphthyl group, 2-naphthyl group, benzofuran-2-yl group, benzo[b]thiophen-2-yl group, indol-2-yl group, quinolin-3-yl group, 1H-benzimidazol-2-yl group, benzoxazol-2-yl group, benzothiazol-2-yl group, 2H-benzopyran-3-yl group, 4-vinylphenyl group, 4-benzenesulfonyl-thiophen-2-yl group, 5-(2-pyridyl)thiophen-2-yl group, quinolin-6-yl, or (thiophen-2-yl)ethenyl group, and the aromatic ring in such group is optionally mono- or di-substituted with a halogen atom (most preferably chlorine atom or bromine atom) or a C₁₋₆ alkyl group (most preferably methyl group).

[1-1-e] Particularly preferably, Q is phenyl group, benzo[b]thiophen-2-yl group, indol-2-yl group, or (thiophen-2-yl)ethenyl group, and the aromatic ring in such group is optionally further mono- or di-substituted with a halogen atom (most preferably chlorine atom or bromine atom) or a C₁₋₆ alkyl group (most preferably methyl group).

[1-1-f] In the compound of the formula (I), Q is most preferably indol-2-yl group, and the aromatic ring in such group is optionally further mono- or di-substituted with a halogen atom (most preferably chlorine atom or bromine atom) or a C₁₋₆ alkyl group (most preferably methyl group).

[1-2] In the compound of the formula (I), T is preferably an optionally substituted aliphatic hydrocarbon group, an optionally substituted 3- to 4-membered alicyclic hydrocarbon group, an optionally substituted C₂₋₆ alkanoyl group, or trifluoromethyl group.

[1-2-a] Examples of the "aliphatic hydrocarbon group" and the "3- to 4-membered alicyclic hydrocarbon group" include those described in [1-1] which are optionally substituted with the substituent mentioned in (a) to (g) of the "optionally substituted hydrocarbon group" of [1-1].

[1-2-b] Preferable examples of the "aliphatic hydrocarbon group" and the "3- to 4-membered alicyclic hydrocarbon group" include an optionally substituted C₁₋₆ alkyl group, an optionally substituted C₂₋₆ alkenyl group, an optionally substituted C₂₋₆ alkynyl group, an optionally substituted C₃₋₄ cycloalkyl group, an optionally substituted C₃₋₄ cycloalkenyl group, and an optionally substituted C₄ cycloalkanedienyl group.
The substituent may be a substituent RI (wherein RI represents a group selected from a C₁₋₆ alkoxy, a C₁₋₆ alkoxycarbonyl, carboxyl, carbamoyl optionally mono- or di-substituted with a C₁₋₆alkyl, a halogen, a C₁₋₆alkyl, a halogenated C₁₋₆alkyl, amino mono- or di-substituted with a C₁₋₆alkyl, a C₂₋₆ alkenoylamino, nitro, hydroxy, phenyl, phenoxy, benzyl, oxo, cyano, and amidino).

[1-2-c] More preferable examples of the "aliphatic hydrocarbon group" and the "3- to 4-membered alicyclic hydrocarbon group" include "an optionally substituted C₁₋₆ alkyl group" such as methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, and n-hexyl which are optionally substituted with a group selected from a C₁₋₆ alkoxy, amino which is optionally mono- or di-substituted with a C₁₋₆ alkyl, and hydroxy.

Examples of the "optionally substituted C₂₋₆ alkenyl group" include vinyl, allyl, isopropenyl, 2-methylallyl, butenyl, pentenyl, and hexenyl which are optionally substituted with a group selected from a C₁₋₆ alkoxy, amino which is optionally mono- or di-substituted with a C₁₋₆ alkyl, and hydroxy.
Examples of the "optionally substituted C₂₋₆ alkynyl group" include ethynyl, 1-propynyl, 2-propynyl, butynyl, pentynyl, and hexynyl which are optionally substituted with a group selected from a C₁₋₆ alkoxy, amino which is optionally mono- or di-substituted with a C₁₋₆ alkyl, and hydroxy. Examples of the "optionally substituted C₃₋₄ cycloalkyl group" include cyclopropyl and cyclobutyl which are optionally substituted with a group selected from a C₁₋₆ alkoxy, amino which is optionally mono- or di-substituted with a C₁₋₆ alkyl, and hydroxy.
Examples of the "optionally substituted C₃₋₄ cycloalkenyl group" include C₃₋₄ cycloalkenyl groups such as 1-cyclopropen-1-yl and 1-cyclobuten-l-yl which are optionally substituted with a group selected from a C₁₋₆ alkoxy, amino which is optionally mono- or di-substituted with a C₁₋₆ alkyl, and hydroxy.
Examples of the "optionally substituted C₄ cycloalkanedienyl group" include C₄ cycloalkanedienyl groups such as 1,3-cyclobutadien-1-yl which are optionally substituted with a group selected from a C₁₋₆ alkoxy, amino which is optionally mono- or di-substituted with a C₁₋₆ alkyl, and hydroxy.

[1-2-d] Still more preferable examples of the "aliphatic hydrocarbon group" and the "3- to 4-membered alicyclic hydrocarbon group" include methyl, ethyl, propyl, isopropyl, butyl, isobutyl, sec-butyl, and tert-butyl; vinyl, allyl, isopropenyl, and 2-methylallyl; ethynyl, 1-propynyl, and 2-propynyl; cyclopropyl, and cyclobutyl; 1-cyclopropen-1-yl, 1-cyclobuten-1-yl, and 1,3-cyclobutadien-1-yl which are optionally substituted with a group selected from a C₁₋₆ alkoxy, amino which is optionally mono- or di-substituted with a C₁₋₆ alkoxy, and hydroxy.

[1-2-e] In the compound of the formula (I), exemplary "optionally substituted C₂₋₆ alkanoyl groups" include acetyl and propylonyl which are optionally substituted with a group selected from a C₁₋₆ alkoxy, amino which is optionally mono- or di-substituted with a C₁₋₆ alkoxy, and hydroxy.

[1-2-f] In the compound of the formula (I), preferable examples of T include methyl, ethyl, propyl, isopropyl, butyl, isobutyl, sec-butyl, tert-butyl, methoxymethyl, 2-methoxy-1-ethyl, 3-methoxy-1-propyl, hydroxymethyl, 2-hydroxy-1-ethyl, 3-hydroxy-1-propyl, aminomethyl, 2-amino 1-ethyl, 3-aminopropyl, (N-methyl-amino)methyl, 2-(N-methyl-amino)-1-ethyl, 3-(N-methyl-amino)propyl, (N,N-dimethyl-amino)methyl, 2-(N,N-dimethyl-amino)-1-ethyl, and 3-(N,N-dimethyl-amino)propyl; vinyl, allyl, isopropenyl, and 2-methylallyl; ethynyl, 1-propynyl, 2-propynyl, and 2-methoxy-1-vinyl; cyclopropyl and cyclobutyl; 1-cyclopropen-1-yl, 1-cyclobuten-1-yl, and 1,3-cyclobutadien-1-yl; acetyl; and trifluoromethyl.

[1-2-g] More preferable examples of T include methyl, ethyl, propyl, isopropyl, butyl, isobutyl, sec-butyl, tert-butyl, methoxymethyl, 2-methoxy-1-ethyl, hydroxymethyl, 2-hydroxy-1-ethyl, aminomethyl, 2-amino-1-ethyl, (N-methylamino)methyl, 2-(N-methyl-amino)-1-ethyl, (N,N-dimethylamino)methyl, and 2-(N,N-dimethyl-amino)-1-ethyl; vinyl and allyl; ethynyl and 2-methoxy-1-vinyl; cyclopropyl and cyclobutyl; acetyl; and trifluoromethyl.

[1-3] In the compound of the formula (I), D is [1-3-a] hydrogen atom, group -CO-R⁵ (wherein R⁵ is hydrogen atom or a substituent), or an optionally substituted C₁₋₆ alkyl group (preferably a C₁₋₆ alkyl group optionally substituted with R¹⁵ as described below).
R⁵ is preferably hydrogen atom; hydroxy group; a C₁₋₆ alkyl group; a C₁₋₆ alkoxy group; a C₁₋₆ alkoxycarbonylalkyl group; phenoxy group or benzyloxy group which is optionally substituted with a C₁₋₆ alkyl, a C₁₋₆ alkoxy, or a halogen atom; or optionally substituted amino group, and in particular, group -NR⁶R⁷ (wherein R⁶ and R⁷ are independently hydrogen atom, a C₁₋₆ alkyl, a C₄₋₇ cycloalkyl, or a C₂₋₆ alkenyl; or R⁶ and R⁷ may together form a five- to seven-membered heterocyclic ring with the nitrogen atom to which R⁶ and R⁷ are bonded, the heterocyclic ring optionally further comprising 1 or 2 heteroatoms selected from N, S, and O); and such substituent R⁵ is optionally further substituted with a group selected from hydroxy, amino, carboxyl, a C₁₋₆ alkoxycarbonyl, oxo, a C₁₋₆ alkyl, a hydroxy-C₁₋₆ alkyl, a C₁₋₆ alkoxy-C₁₋₆ alkyl, a carboxy C₁₋₆ alkyl, a C₁₋₆ alkyl-C₁₋₆ alkoxycarbonyl, and a carbamoyl C₁₋₆ alkoxy).

[1-3-b] More preferably, D is hydrogen atom; or
1) a group selected from carboxyl group; a C₁₋₆ alkylcarbonyl group; a C₁₋₆ alkoxycarbonyl group; a C₁₋₆ alkoxycarbonylalkylcarbonyl group; phenoxycarbamoyl group optionally substituted with a C₁₋₆ alkyl, a C₁₋₆ alkoxy, or a halogen atom; benzyloxycarbonyl group optionally substituted with a C₁₋₆ alkyl, a C₁₋₆ alkoxy, or a halogen atom;
2) carbamoyl group optionally mono- or di-substituted with a C₁₋₆ alkyl group; a C₁₋₆ alkoxycarbamoyl group; a C₁₋₆ alkoxycarbonylalkylcarbamoyl group; a cyclic aminocarbonyl group (in particular, pyrrolidin-1-ylcarbonyl group, piperidin-1-ylcarbonyl, piperazin-1-ylcarbonyl, 4-morpholinocarbonyl, thiomorpholinocarbonyl, or 1,1-dioxo-4-thiomorpholinocarbonyl) optionally substituted with oxo, hydroxy, amino, or carboxyl; or a group selected from N-phenylcarbamoyl group and a group represented by -CONH(CH₂)ₚS(O)_{q}R¹⁰ or -CONH(CH₂)ₜNR¹¹R¹² wherein R¹⁰, R¹¹, and R¹² independently represent hydrogen atom, a C₁₋₆ alkyl group, phenyl group, or a C₁₋₆ alkylphenyl group, p is an integer of 0 to 4, q is an integer of 0 to 2, and t is an integer of 1 to 4; or
3) a C₁₋₆ alkyl group (preferably methyl or ethyl) optionally substituted with R¹⁵ wherein R¹⁵ is carboxyl group; a C₁₋₆ alkoxycarbonyl group; hydroxy group; a C₁₋₆ alkoxy group; a C₁₋₆ alkanoyloxy group; amino group; amino group mono- or di-substituted with substituent RX (wherein the substituent RX is a group selected from a C₁₋₆ alkyl, a C₁₋₆ alkanoyl, a C₁₋₆ alkylsulfonyl, a C₁₋₆ alkoxycarbonyl, a C₁₋₆ alkoxycarbonyl-C₁₋₆ alkyl, and a carboxy-C₁₋₆ alkyl); a five- or six-membered cyclic amino group (and in particular, pyrrolidin-1-yl, piperidin-1-yl, piperazin-1-yl, 4-morpholino, thiomorpholino, or 1,1-dioxo-4-thiomorpholino) or N-hydroxyimino group (aldoxime group) optionally substituted with oxo, hydroxy, amino, or carboxyl; a hydroxy-C₁₋₆ alkoxy group; a C₁₋₆ alkoxy-C₁₋₆ alkoxy group; a carboxy-C₁₋₆ alkoxy group; a C₁₋₆ alkoxycarbonyl-C₁₋₆ alkoxy group; a carbamoyl-C₁₋₆ alkoxy group; a C₀₋₆ alkoxy group (and in particular, a C₀₋₂ alkoxy group in which the C₀ alkoxy group designates the group wherein the substituent is directly bonded to the oxygen atom) mono-substituted with a substituent selected from (i) a five- or six-membered, monocyclic aromatic heterocyclic group and (ii) an eight- to twelve-membered, fused aromatic heterocyclic group containing 1 to 4 heteroatoms selected from oxygen atom, sulfur atom, and nitrogen atom in addition to the carbon atoms.

[1-3-c] Still more preferably, D is hydrogen atom; or
1) a group selected from carboxyl group, a C₁₋₂ alkylcarbonyl group, a C₁₋₂ alkoxycarbonyl group, and a C₁₋₂ alkoxycarbonylalkylcarbonyl group; or a group selected from phenoxycarbonyl group which is optionally substituted with a C₁₋₂ alkyl, a C₁₋₂ alkoxy, or a halogen atom, or benzyloxycarbonyl group which is optionally substituted with a C₁₋₂ alkyl, a C₁₋₂ alkoxy, or a halogen atom;
2) carbamoyl group which is optionally mono- or di-substituted with a C₁₋₂ alkyl; a C₁₋₂ alkoxycarbamoyl group; a C₁₋₂ alkoxycarbonylalkylcarbamoyl group; a cyclic aminocarbonyl group which is optionally substituted with oxo, hydroxy, amino or carboxyl (and in particular, pyrrolidin-1-ylcarbonyl group, piperidin-1-ylcarbonyl, piperazin-1-ylcarbonyl, 4-morpholinocarbonyl group, thiomorpholinocarbonyl group, or 1,1-dioxo-4-thiomorpholinocarbonyl group); or
3) methyl group or ethyl group optionally substituted with R¹⁵' (wherein R¹⁵' represents carboxyl group; a C₁₋₂ alkoxycarbonyl group; hydroxy group; a C₁₋₂ alkoxy group; a C₁₋₃ alkanoyloxy group; amino group; amino group mono- or di-substituted with a substituent RX' wherein the substituent RX' is a group selected from a C₁₋₂ alkyl, a C₁₋₂ alkanoyl, a C₁₋₂ alkylsulfonyl, a C₁₋₂ alkoxycarbonyl, a C₁₋₂ alkoxycarbonyl-C₁₋₂ alkyl, and a carboxy-C₁₋₂ alkyl; pyrrolidin-1-yl group, piperidin-1-yl group, piperazin-1-yl group, 4-morpholino group, thiomorpholino group or 1,1-dioxo-4-thiomorpholino group which is optionally substituted with oxo, hydroxy, amino or carboxyl; hydroxy-methoxy group, 2-hydroxy-ethoxy group, 2-methoxy-ethoxy group, 2-ethoxy-ethoxy group, carboxy-methoxy group, 2-carboxy-ethoxy group, methoxycarbonyl-methoxy group, 2-methoxycarbonyl-ethoxy group, ethoxycarbonyl-methoxy group, 2-ethoxycarbonyl-emethoxy group, carbamoyl-methoxy group, 2-carbamoyl-ethoxy group; and a C₀₋₂ alkoxy group (in which the C₀ alkoxy group designates the group wherein the substituent is directly bonded to the oxygen atom, for example, pyridyloxy group, pyridylmethoxy group, pyridylethoxy group, pyrimidyloxy group, pyrimidylmethoxy group and pymidylethoxy group) mono-substituted with a five- or six-membered, monocyclic aromatic heterocyclic group containing 1 to 2 nitrogen atoms in addition to the carbon atoms.

[1-4] In the formula (I), Y is
[1-4-a] oxygen atom, -S(O)_{y}- (wherein y is an integer of 0 to 2), or optionally substituted imino group (-NH-) wherein the substituent for the imino group is 1) -CO-R⁵ (wherein R⁵ is a group selected from those as defined above); 2) a C₁₋₆ alkyl group optionally substituted with R¹⁵ (wherein R¹⁵ is a group selected from those as defined above); or 3) phenyl group which is optionally substituted with a C₁₋₆ alkyl, a C₁₋₆ alkoxy, or a halogen atom; or 4) a N-oxide group, as described for D in [1-3].

[1-4-b] Preferably, Y is oxygen atom, or

[1-4-c] alternatively, Y is preferably -S(O)_{y}- (wherein y is an integer of 0 to 2, and in particular, 0), or

[1-4-d] non-substituted imino group (-NH-). If substituted, the substituent may be, for example, methyl group.

[1-5] In the formula (I), 1, m, and n are
[1-5-a] independently an integer selected from 0, 1 and 2 with the proviso that 1 and m are not simultaneously 0, and
[1-5-b] more preferably, 1 is 1; m is 0 or 1; and n is 1.

[1-6] In the formula (I), r is 0 or 1.

[1-7] Exemplary substituents of the N-containing ring having the T-CO- bonded thereto, the Y-containing ring, and the N-containing ring having the -SO₂-Q bonded thereto include oxo group (=O), hydroxyimino group (=N~OH), alkoxyimino group (=N~ORi wherein Ri is a C₁₋₆ alkyl group which is optionally substituted with a substituent preferably selected from a halogen, hydroxyl, and carboxy), and the groups mentioned for D in [1-3]. Preferable substituents, include oxo group, hydroxy group, carboxyl group, a halogen atom, a C₁₋₆ alkyl group, a C₂₋₆ alkenyl group, and a C₂₋₆ alkynyl group, and among these, a C₁₋₆ alkyl group, a C₂₋₆ alkenyl group, and a C₂₋₆ alkynyl group may be further substituted with a substituent RI (wherein RI represents a group selected from a C₁₋₆ alkoxy, a C₁₋₆ alkoxycarbonyl, carboxyl, carbamoyl which is optionally mono- or di-substituted with a C₁₋₆ alkyl, a halogen, a C₁₋₆ alkyl, a halogenated C₁₋₆ alkyl, amino which is optionally mono- or di-substituted with a C₁₋₆ alkyl, a C₂₋₆ alkenoylamino, nitro, hydroxy, oxo, cyano, and amidino). More preferable substituents include oxo group, a C₁₋₆ alkoxy group, and carboxyl group.

More preferably,
[1-7-a] exemplary substituents for the N-containing ring having the T-CO- bonded thereto include oxo group, hydroxyl group, lower alkyl group, and lower alkoxyalkyl group,
[1-7-b] exemplary substituents for the Y-containing ring include oxo group, hydroxyimino group, substituted alkoxyimino group (=N~ORi wherein Ri is a C₁₋₆ alkyl group which is optionally substituted with a substituent preferably selected from a halogen, hydroxyl, and carboxy), and
[1-7-c] exemplary substituents for the N-containing ring having the -SO₂-Q bonded thereto include oxo group, hydroxyimino group, substituted alkoxyimino group (=N~ORi wherein Ri is a C₁₋₆alkyl group optionally substituted with a substituent which is preferably selected from a halogen, hydroxyl, and carboxy), and the position of the substitution also include the case wherein the oxo group is substituted with hydroxyimino group or a substituted alkoxyimino group.

Preferable compounds of the formula (I) may be defined by adequate combinations of the [1-1] to [1-7]. Exemplary preferable compounds are described in [1-8].

[1-8] In the formula (I), 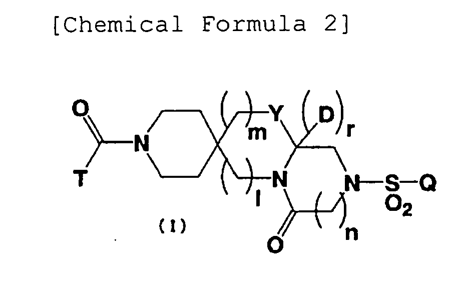 Q is (1) a C₁₋₁₀ alkyl group, (2) a C₂₋₆ alkenyl group, (3) a C₂₋₆ alkynyl group, (4) a C₃₋₉ cycloalkyl group, (5) a C₃₋₆ cycloalkenyl group, (6) a C₄₋₆ cycloalkadienyl group, (7) a C₆₋₁₄ aryl group, or (8) a heterocyclic group containing 1 to 4 heteroatoms selected from oxygen atom, nitrogen atom and sulfur atom in addition to the carbon atoms which is any one of (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", and the groups of the (1) to (8) may be either unsubstituted or substituted with 1 to 5 substituents of the class selected from (a) to (g) as described below.
(a) A C₁₋₆ alkyl group or a C₆₋₁₄ aryl group which is optionally further substituted with a substituent RI (wherein RI represents a C₁₋₆ alkoxy group, a halogen, a C₁₋₆ alkyl group, amino group, hydroxy group, cyano group, or amidino.group).
(b) A heterocyclic group containing 1 to 4 heteroatoms selected from oxygen atom, nitrogen atom and sulfur atom in addition to the carbon atoms which is any one of (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", and which may be further substituted with substituent RII (wherein RII represents a halogen atom such as fluorine, chlorine, bromine, or iodine, a C₁₋₆alkyl group, a C₁₋₆alkanoyl group, or benzoyl group).
(c) Amino group optionally substituted with a group selected from substituents RIII wherein RIII represents a C₁₋₆ alkyl group, a C₁₋₆alkanoyl group, benzoyl group, or an optionally halogenated C₁₋₆ alkoxycarbonyl group, a C₁₋₆ alkylimidoyl group, formimidoyl group, or amidino group.
(d) Imidoyl group, amidino group, hydroxy group, or thiol group, which is optionally further substituted with a substituent selected from a C₁₋₆alkyl group, a C₁₋₆alkanoyl group, benzoyl group, an optionally halogenated C₁₋₆ alkoxycarbonyl group.
(e) A halogen atom, cyano group, or nitro group.
(f) Carboxyl group, a C₁₋₆ alkoxycarbonyl group, a C₇₋₁₂ aryloxycarbonyl group, or a C₆₋₁₀ aryl-C₁₋₄ alkoxycarbonyl group. The aryl group is optionally further substituted with a substituent RIV (wherein RIV represents hydroxy, amino group optionally mono- or di-substituted with a group selected from those mentioned in the above (c) for the substituent RIII, a halogen atom, nitro group, cyano group, a C₁₋₆alkyl group optionally substituted with 1 to 5 halogen atoms, or an alkoxy group optionally substituted with 1 to 5 halogen atoms).
(g) -CO-RV wherein RV represents a C₁₋₆alkyl group, a C₃₋₆ cycloalkyl group, a C₆₋₁₀ aryl group, a C₇₋₁₀ aralkyl group, or a heterocyclic group. This heterocyclic group is a heterocyclic group containing 1 to 4 heteroatoms selected from oxygen atom, nitrogen atom and sulfur atom in addition to the carbon atoms which is any one of (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups".

More preferably, Q is phenyl group, benzyl group, phenethyl group, styryl group, 1-naphthyl group, 2-naphthyl group, benzofuran-2-yl group, benzo[b]thiophen-2-yl group, indol-2-yl group, quinolin-3-yl group, 1H-benzimidazol-2-yl group, benzoxazol-2-yl group, benzothiazol-2-yl group, 2H-benzopyran-3-yl group, 4-vinylphenyl group, 4-benzenesulfonyl-thiophen-2-yl group, 5-(2-pyridyl)thiophen-2-yl group, quinolin-6-yl group, or (thiophen-2-yl)ethenyl group wherein the aromatic ring is optionally further mono- or di-substituted with a halogen atom (in particular, chlorine atom or bromine atom) or a C₁₋₆alkyl group (in particular, methyl group), and
still more preferably, Q is phenyl group, benzo[b]thiophen-2-yl group, indol-2-yl group, or (thiophen-2-yl)ethenyl group wherein the aromatic ring is optionally further mono- or di-substituted with a halogen atom (in particular, chlorine atom or bromine atom) or a C₁₋₆alkyl group (in particular, methyl group).

T is an optionally substituted C₁₋₆ alkyl group, an optionally substituted C₂₋₆ alkenyl group, an optionally substituted C₂₋₆ alkynyl group, an optionally substituted C₃₋₄ cycloalkyl group, an optionally substituted C₃₋₄ cycloalkenyl group, an optionally substituted C₄ cycloalkanedienyl group, a C₂₋₆ alkanoyl group, or trifluoromethyl group wherein the substituent is a group selected from a C₁₋₆ alkoxy, amino optionally mono- or di-substituted with a C₁₋₆alkyl, and hydroxy;

preferably, T is methyl group, ethyl group, propyl group, isopropyl group, butyl group, isobutyl group, sec-butyl group, tert-butyl group, methoxymethyl group, 2-methoxy-1-ethyl group, 3-methoxy-1-propyl group, hydroxymethyl group, 2-hydroxy-1-ethyl group, 3-hydroxy-1-propyl group, aminomethyl group, 2-amino-1-ethyl group, 3-aminopropyl group, (N-methyl-amino)methyl group, 2-(N-methyl-amino)-1-ethyl group, 3-(N-methyl-amino)propyl group, (N,N-dimethyl-amino)methyl group, 2-(N,N-dimethyl-amino)-1-ethyl group, or 3-(N,N-dimethyl-amino)propyl group; vinyl group, allyl group, isopropenyl group, or 2-methylallyl group; ethynyl group, 1-propynyl group, 2-propynyl group, or 2-methoxy-1-vinyl group; cyclopropyl group, or cyclobutyl group; 1-cyclopropen-1-yl group, 1-cyclobuten-1-yl group, or 1,3-cyclobutadien-1-yl group; acetyl group; or trifluoromethyl group; and
more preferably, T is methyl group, ethyl group, propyl group, isopropyl group, butyl group, isobutyl group, sec-butyl group, tert-butyl group, methoxymethyl group, 2-methoxy-1-ethyl group, hydroxymethyl group, 2-hydroxy-1-ethyl group, aminomethyl group, 2-amino-1-ethyl group, (N-methyl-amino)methyl group, 2-(N-methyl-amino)-1-ethyl group, (N,N-dimethyl-amino)methyl group, 2-(N,N-dimethyl-amino)-1-ethyl group; vinyl group, allyl group; ethynyl group, 2-methoxy-1-vinyl group; cyclopropyl group, cyclobutyl group; acetyl group; or trifluoromethyl group.

D is hydrogen atom, group -CO-R⁵ (wherein R⁵ is hydrogen atom or a substituent), or an optionally substituted C₁₋₆ alkyl group (preferably a C₁₋₆ alkyl group optionally substituted with R¹⁵ as will be described below).
R⁵ is preferably hydrogen atom, hydroxy, a C₁₋₆ alkyl, a C₁₋₆ alkoxy, a C₁₋₆ alkoxycarbonylalkyl, or optionally substituted amino group, and in particular, -NR⁶R⁷ (wherein R⁶ and R⁷ are independently hydrogen atom, a C₁₋₆ alkyl, a C₄₋₇ cycloalkyl, a C₂₋₆ alkenyl, or may together form a 5-to 7-membered heterocycle ring with the nitrogen to which the R⁶ and R⁷ are bonded, the heterocycle optionally further containing 1 or 2 heteroatoms selected from N, S, and O). The substituent R⁵ is optionally further substituted with a group selected from hydroxy, amino, carboxyl, a C₁₋₆ alkoxycarbonyl, oxo, a C₁₋₆ alkyl, a hydroxy-C₁₋₆ alkyl, a C₁₋₆ alkoxy-C₁₋₆ alkyl, a carboxy-C₁₋₆ alkyl, a C₁₋₆ alkyl-C₁₋₆ alkoxycarbonyl, and a carbamoyl-C₁₋₆ alkoxy.

More preferably, D is hydrogen atom;
1) a group selected from carboxyl group, a C₁₋₆ alkylcarbonyl group, a C₁₋₆ alkoxycarbonyl group, and a C₁₋₆ alkoxycarbonylalkylcarbonyl group;
2) carbamoyl group optionally mono- or di-substituted with a C₁₋₆ alkyl group; a C₁₋₆ alkoxycarbamoyl group; a C₁₋₆ alkoxycarbonylalkylcarbamoyl group; a cyclic aminocarbonyl group (in particular, pyrrolidin-1-ylcarbonyl group, piperidin-1-ylcarbonyl, piperazin-1-ylcarbonyl, 4-morpholinocarbonyl, thiomorpholinocarbonyl, or 1,1-dioxo-4-thiomorpholinocarbonyl) optionally substituted with oxo, hydroxy, amino, or carboxyl; or a group selected from N-phenylcarbamoyl group and a group represented by -CONHCH₂)ₚS(O)_{q}R¹⁰ or -CONH(CH₂)ₜNR₁₁R₁₂ wherein R¹⁰, R¹¹, and R¹² independently represent hydrogen atom, a C₁₋₆ alkyl group, phenyl group, or a C₁₋₆ alkylphenyl group, p is an integer of 0 to 4, q is an integer of 0 to 2, and t is an integer of 1 to 4; or
3) a C₁₋₆ alkyl group (preferably methyl or ethyl) optionally substituted with R¹⁵ wherein R¹⁵ is carboxyl group; a C₁₋₆ alkoxycarbonyl group; hydroxy group; a C₁₋₆ alkoxy group; a C₁₋₆ alkanoyloxy group; amino group; amino group mono- or di-substituted with substituent RX (wherein the substituent RX is a group selected from a C₁₋₆ alkyl, a C₁₋₆ alkanoyl, a C₁₋₆ alkylsulfonyl, a C₁₋₆ alkoxycarbonyl, a C₁₋₆ alkoxycarbonyl-C₁₋₆ alkyl, and a carboxy-C₁₋₆ alkyl); a five- or six-membered cyclic amino group (and in particular, pyrrolidin-1-yl, piperidin-1-yl, piperazin-1-yl, 4-morpholino, thiomorpholino, or 1,1-dioxo-4-thiomorpholino) or N-hydroxyimino group (aldoxime group) optionally substituted with oxo, hydroxy, amino, or carboxyl; a hydroxy-C₁₋₆ alkoxy group; a C₁₋₆ alkoxy-C₁₋₆ alkoxy group; a carboxy-C₁₋₆ alkoxy group; a C₁₋₆ alkoxycarbonyl-C₁₋₆ alkoxy group; a carbamoyl-C₁₋₆ alkoxy group; a C₀₋₆ alkoxy group (and in particular, a C₀₋₂ alkoxy group in which the C₀ alkoxy group designates the group wherein the substituent is directly bonded to the oxygen atom) mono-substituted with a substituent selected from (i) a five- or six-membered, monocyclic aromatic heterocyclic group and (ii) an eight- to twelve-membered, fused aromatic heterocyclic group containing 1 to 4 heteroatoms selected from oxygen atom, sulfur atom, and nitrogen atom in addition to the carbon atoms.

Y is oxygen atom, -S(O)_{y}- (wherein y is an integer of 0 to 2, and in particular, 0) or NH, and
l, m, and n are independently an integer selected from 0, 1, and 2, with the proviso that 1 and m are not simultaneously 0, and r is an integer of 0 or 1.

Exemplary preferable compounds are:
(-)-1'-acetyl-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one (Example 1);
(-)-7-[(5-chloro-1H-indol-2-yl)sulfonyl]tetrahydro-8a-(methoxymethyl)-1-methyl-1'-propanoyl-spiro[imidazo[1,2-a]pyrazine-2(3H),4'-piperidin]-5(1H)-one (Example 2);
(-)-7-[(5-chloro-1H-indol-2-yl)sulfonyl]-1'-cyclobutanecarbonyl-tetrahydro-8a-(methoxymethyl)-1-methyl-spiro[imidazo[1,2-a]pyrazine-2(3H),4'-piperidin]-5(1H)-one (Example 3);
(-)-7-[(5-chloro-1H-indol-2-yl)sulfonyl]tetrahydro-8a-(methoxymethyl)-1-methyl-1'-propenoyl-spiro[imidazo[1,2-a]pyrazine-2(3H),9'-piperidin]-5(1H)-one (Example 4);
(-)-7-[(5-chloro-1H-indol-2-yl)sulfonyl]-1'-(2-furoylcarbonyl)-tetrahydro-8a-(methoxymethyl)-1-methyl-spiro[imidazo[1,2-a]pyrazine-2(3H),4'-piperidin]-5(1H)-one (Example 5);
(-)-7-[(5-chloro-1H-indol-2-yl)sulfonyl]-1'-(trifluoroacetyl)-tetrahydro-8a-(methoxymethyl)-1-methyl-spiro[imidazo[1,2-a]pyrazine-2(3H),4'-piperidin]-5(1H)-one (Example 6);
(-)-7-[(5-chloro-1H-indol-2-yl)sulfonyl]tetrahydro-1'-(methoxyacetyl)-8a-(methoxymethyl)-1-methyl-spiro[imidazo[1,2-a]pyrazine-2(3H),4'-piperidin]-5(1H)-one (Example 7);
(-)-7-[(5-chloro-1H-indol-2-yl)sulfonyl]tetrahydro-8a-(methoxymethyl)-1-methyl-1'-(dimethylaminoacetyl)-spiro[imidazo[1,2-a]pyrazine-2(3H),4'-piperidin]-5(1H)-one (Example 8);
(-)-1'-(aminoacetyl)-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one (Example 9);
(-)-7-[(5-chloro-1H-indol-2-yl)sulfonyl]tetrahydro-1'-(hydroxyacetyl)-8a-(methoxymethyl)-1-methyl-spiro[imidazo[1,2-a]pyrazine-2(3H),4'-piperidin]-5(1H)-one (Example 10);

(-)-7-[(5-chloro-1H-indol-2-yl)sulfonyl]tetrahydro-8a-(methoxymethyl)-1'-(2-methoxypropenoyl)-1-methyl-spiro[imidazo[1,2-a]pyrazine-2(3H),4'-piperidin]-5(1H)-one (Example 11);
(-)-7-[(5-chloro-1H-indol-2-yl)sulfonyl]tetrahydro-8a-(methoxymethyl)-1'-[(1Z)-(2-methoxypropenoyl)]-1-methyl-spiro[imidazo[1,2-a]pyrazine-2(3H),4'-piperidin]-5(1H)-one (Example 12);
(-)-7-[(5-chloro-1H-indol-2-yl)sulfonyl]tetrahydro-8a-(methoxymethyl)-1-methyl-1'-propinoyl-spiro[imidazo[1,2-a]pyrazine-2(3H),4'-piperidin]-5(1H)-one (Example 13);
(-)-1'-acetyl-7-[[(1E)-2-(5-chloro-2-thienyl)ethenyl]sulfonyl]tetrahydro-8a-(methoxymethyl)-1-methyl-spiro[imidazo[1,2-a]pyrazine-2(3H),9'-piperidin]-5(1H)-one (Example 14);
(-)-1'-acetyl-7-[(6-chloro-benzo[b]thiophen-2-yl)sulfonyl]tetrahydro-8a-(methoxymethyl)-1-methyl-spiro[imidazo[1,2-a]pyrazine-2(3H),4'-piperidin]-5(1H)-one (Example 15);
(-)-1'-acetyl-7-[(6-chloronaphthalen-2-yl)sulfonyl]tetrahydro-8a-(methoxymethyl)-1-methyl-spiro[imidazo[1,2-a]pyrazine-2(3H),4'-piperidin]-5(1H)-one (Example 16);
(-)-7-[(6-chloro-benzo[b]thiophen-2-yl)sulfonyl]tetrahydro-8a-(methoxymethyl)-1-methyl-1'-propinoyl-spiro[imidazo[1,2-a]pyrazine-2(3H),9'-piperidin]-5(1H)-one (Example 17);
(±)-1'-acetyl-7-[(5-chloro-1H-indol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-5(1H)-one (Example 18);
(±)-1'-acetyl-7-[(5-chloro-1H-indol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-5(1H)-one (Example 19);
(±)-1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one (Example 20);

optically active 1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one (Example 21);
(-)-1'-acetyl-7-[(5-chloro-1H-indol-2-yl)sulfonyl]tetrahydro-8a-(hydroxymethyl)-1-methyl-spiro[imidazo[1,2-a]pyrazine-2(3H),4'-piperidin]-5(1H)-one (Example 22);
(-)-1'-acetyl-7-[(5-chloro-1H-indol-2-yl)sulfonyl]tetrahydro-1-ethyl-8a-(methoxymethyl)-spiro[imidazo[1,2-a]pyrazine-2(3H),4'-piperidin]-5(1H)-one (Example 23);
(±)-1'-acetyl-7-[(5-chloro-1H-indol-2-yl)sulfonyl]tetrahydro-1-(hydroxyethyl)-8a-(methoxymethyl)-spiro[imidazo[1,2-a]pyrazine-2(3H),4'-piperidin]-5(1H)-one (Example 24);
(-)-1'-acetyl-7-[(5-chloro-1H-indol-2-yl)sulfonyl]tetrahydro-8a-(methoxymethyl)-spiro[imidazo[1,2-a]pyrazine-2(3H),4'-piperidin]-5(1H)-one (Example 25);
(-)-7-[(5-chloro-1H-indol-2-yl)sulfonyl]tetrahydro-8a-(methoxymethyl)-1'-propanoyl-spiro[imidazo[1,2-a]pyrazine-2(3H),4'-piperidin]-5(1H)-one (Example 26);
(-)-1'-acetyl-7-[[(1E)-2-(5-chloro-2-thienyl)ethenyl]sulfonyl]tetrahydro-8a-(methoxymethyl)-spiro[imidazo[1,2-a]pyrazine-2(3H),4'-piperidin]-5(1H)-one (Example 27);
(-)-1'-acetyl-7-[(6-chloro-benzo[b]thiophen-2-yl)sulfonyl]tetrahydro-8a-(methoxymethyl)-spiro[imidazo[1,2-a]pyrazine-2(3H),4'-piperidin]-5(1H)-one (Example 28);
(±)-1'-acetyl-7-[(6-chloronaphthalen-2-yl)sulfonyl]tetrahydro-8a-(methoxymethyl)-spiro[imidazo[1,2-a]pyrazine-2(3H),9'-piperidin]-5(1H)-one (Example 29);
(-)-7-[(5-chloro-1H-indol-2-yl)sulfonyl]tetrahydro-8a-(methoxymethyl)-1'-propanoyl-spiro[5H-oxazolo[3,2-a]pyrazine-2(3H),4'-piperidin]-5-one (Example 30);

(-)-7-[(6-chloro-benzo[b]thiophen-2-yl)sulfonyl]tetrahydro-8a-(methoxymethyl)-1'-propanoyl-spiro[5H-oxazolo[3,2-a]pyrazine-2(3H),4'-piperidin]-5-one (Example 31);
(-)-7-[(5-chloro-1H-indol-2-yl)sulfonyl]tetrahydro-8a-(methoxymethyl)-1'-propenoyl-spiro[5H-oxazolo[3,2-a]pyrazine-2(3H),4'-piperidin]-5-one (Example 32);
(-)-7-[(6-chloro-benzo[b]thiophen-2-yl)sulfonyl]tetrahydro-8a-(methoxymethyl)-1'-propenoyl-spiro[5H-oxazolo[3,2-a]pyrazine-2(3H),4'-piperidin]-5-one (Example 33);
(-)-1'-acetyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one (Example 34);
(-)-1'-acetyl-7-[(6-chloro-benzo[b]thiophen-2-yl)sulfonyl]tetrahydro-8a-(methoxymethyl)-spiro[5H-oxazolo[3,2-a]pyrazine-2(3H),4'-piperidin]-5-one (Example 35) ;
(-)-1'-acetyl-7-[[(1E)-2-(5-chloro-2-thienyl)ethenyl]sulfonyl]tetrahydro-8a-(methoxymethyl)-spiro[5H-oxazolo[3,2-a]pyrazine-2(3H),9'-piperidin]-5-one (Example 36);
(-)-1'-acetyl-7-[(6-chloronaphthalen-2-yl)sulfonyl]tetrahydro-8a-(methoxymethyl)-spiro[5H-oxazolo[3,2-a]pyrazine-2(3H),4'-piperidin]-5-one (Example 37);
(-)-7-[(6-chloro-benzo[b]thiophen-2-yl)sulfonyl]tetrahydro-8a-(methoxymethyl)-1'-propinoyl-spiro[5H-oxazolo[3,2-a]pyrazine-2(3H),4'-piperidin]-5-one (Example 38);
(-)-1'-n-butanoyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one (Example 39);
(-)-7-[(5-chloro-1H-indol-2-yl)sulfonyl]tetrahydro-1'-cyclopropanoyl-8a-(methoxymethyl)-spiro[5H-oxazolo[3,2-a]pyrazine-2(3H),4'-piperidin]-5-one (Example 40);

(-)-7-[(5-chloro-1H-indol-2-yl)sulfonyl]tetrahydro-1'-(methoxyacetyl)-8a-(methoxymethyl)-spiro[5H-oxazolo[3,2-a]pyrazine-2(3H),9'-piperidin]-5-one (Example 41);
(-)-7-[(5-chloro-1H-indol-2-yl)sulfonyl]tetrahydro-1'-(hydroxyacetyl)-8a-(methoxymethyl)-spiro[5H-oxazolo[3,2-a]pyrazine-2(3H),4'-piperidin]-5-one (Example 42);
(-)-7-[(5-chloro-1H-indol-2-yl)sulfonyl]tetrahydro-1'-(2-furanoyl)-8a-(methoxymethyl)-spiro[5H-oxazolo[3,2-a]pyrazine-2(3H),4'-piperidin]-5-one (Example 43);
(-)-7-[(5-chloro-1H-indol-2-yl)sulfonyl]tetrahydro-8a-(methoxymethyl)-1'-(2-methylpropanoyl)-spiro[5H-oxazolo[3,2-a]pyrazine-2(3H),4'-piperidin]-5-one (Example 44);
(-)-7-[(5-chloro-1H-indol-2-yl)sulfonyl]tetrahydro-8a-(methoxymethyl)-1'-(3-methoxypropanoyl)-spiro[5H-oxazolo[3,2-a]pyrazine-2(3H),4'-piperidin]-5-one (Example 45);
(-)-7-[(5-chloro-1H-indol-2-yl)sulfonyl]tetrahydro-8a-(methoxymethyl)-1'-(2-oxopropanoyl)-spiro[5H-oxazolo[3,2-a]pyrazine-2(3H),4'-piperidin]-5-one (Example 46) ;
(-)-7-[(5-chloro-1H-indol-2-yl)sulfonyl]tetrahydro-8a-(methoxymethyl)-1'-(2,2-dimethylpropanoyl)-spiro[5H-oxazolo[3,2-a]pyrazine-2(3H),9'-piperidin]-5-one (Example 97);
(±)-1'-acetyl-7-[(5-chloro-1H-indol-2-yl)sulfonyl]tetrahydro-8a-(hydroxymethyl)-spiro[5H-oxazolo[3,2-a]pyrazine-2(3H),9'-piperidin]-5-one (Example 48) ;
(±)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one (Example 49);
(-)-1'-acetyl-7-[(5-chloro-1H-indol-2-yl)sulfonyl]tetrahydro-8a-(hydroxymethyl)-spiro[5H-oxazolo[3,2-a]pyrazine-2(3H),9'-piperidin]-5-one (Example 50);

(-)-7-[(5-chloro-1H-indol-2-yl)sulfonyl]tetrahydro-8a-(hydroxymethyl)-1'-propanoyl-spiro[5H-oxazolo[3,2-a]pyrazine-2(3H),4'-piperidin]-5-one (Example 51);
(-)-7-[(5-chloro-1H-indol-2-yl)sulfonyl]tetrahydro-1'-cyclopropanoyl-8a-(hydroxymethyl)-spiro[5H-oxazolo[3,2-a]pyrazine-2(3H),4'-piperidin]-5-one (Example 52);
(-)-7-[(5-chloro-1H-indol-2-yl)sulfonyl]tetrahydro-1'-(2-furanoyl)-8a-(hydroxymethyl)-spiro[5H-oxazolo[3,2-a]pyrazine-2(3H),4'-piperidin]-5-one (Example 53);
(-)-1'-acetyl-7-[(5-chloro-1H-indol-2-yl)sulfonyl]tetrahydro-8a-(methoxycarbonylmethoxymethyl)-spiro[5H-oxazolo[3,2-a]pyrazine-2(3H),4'-piperidin]-5-one (Example 54);
(-)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one (Example 55);
(-)-1'-acetyl-7-[(5-chloro-1H-indol-2-yl)sulfonyl]tetrahydro-8a-(2-pyrimidyloxymethyl)-spiro[5H-oxazolo[3,2-a]pyrazine-2(3H),4'-piperidin]-5-one (Example 56);
(±)-1'-acetyl-8a-(aminomethyl)-7-[(5-chloro-1H-indol-2-yl)sulfonyl]tetrahydro-spiro[5H-oxazolo[3,2-a]pyrazine-2(3H),4'-piperidin]-5-one (Example 57);
(-)-1'-acetyl-8a-(aminomethyl)-7-[(5-chloro-1H-indol-2-yl)sulfonyl]tetrahydro-spiro[5H-oxazolo[3,2-a]pyrazine-2(3H),4'-piperidin]-5-one (Example 58);
(±)-1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),4'-piperidin]-5-one (Example 59);
optically active 1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),9'-piperidin]-5-one (Example 60);

(±)-8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one (Example 61);
optically active 8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one (Example 62) ;
(±)-7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one (Example 63);
optically active 7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one (Example 64);
(±)-N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine (Example 65);
optically active N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine (Example 66);
(±)-N-[[(1'-acetyl)-7-[(5-chloro-1H-indol-2-yl)sulfonyl]tetrahydro-5-oxo-spiro[5H-oxazolo[3,2-a]pyrazine-2(3H),4'-piperidin]-8a-yl]methyl]-N-methylglycine (Example 67);
(±)-1-acetyl-8'-[(5-chloro-1H-indol-2-yl)sulfonyl]tetrahydro-9'a-(methoxymethyl)-spiro[piperidine-4,3'(4'H)-[2H]pyrazino[1,2-a]pyrimidin]-6'(7'H)-one (Example 68);
(±)-1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one (Example 69) ;
optically active 1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one (Example 70);

(±)-1'-acetyl-7-[(5-chloro-1H-indol-2-yl)sulfonyl]tetrahydro-8a-(methoxymethyl)-1,1-dioxide-spiro[5H-thiazolo[3,2-a]pyrazine-2(3H),4'-piperidin]-5-one (Example 71); and
their (+) and (-) optical isomers and
pharmaceutically acceptable salts (such as methanesulphonates (mono- or di-salts).

[1-9] More preferable examples of the compound of the formula (I) include the compounds of the formula (Im) represented by

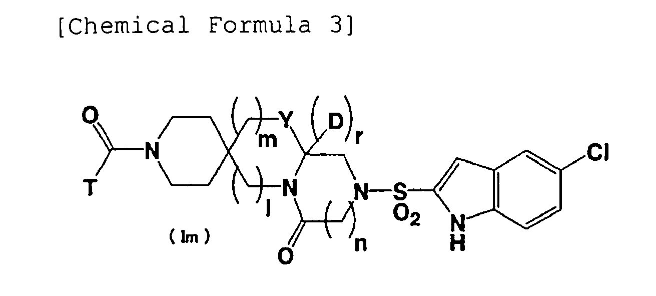

wherein T, Y, D, 1, m, n, and r are as defined above in [1-8], and the preferable compounds are described in [1-8].

[2] A second aspect of the present invention provides a pharmaceutical composition characterized by its inclusion of the compound represented by the formula (I) or (Im), the compound described in the above [1-1] to [1-9], or a pharmaceutically acceptable salt thereof as an effective component.
[2-a] More specifically, the pharmaceutical composition is
   1) an anticoagulant; or a prophylactic and/or therapeutic agent for diseases induced by thrombosis or embolism;
   2) a prophylactic and/or therapeutic agent for diseases wherein an anticoagulant is effective; or a prophylactic and/or therapeutic agent for diseases wherein inhibition of FXa is effective;
   3) a prophylactic agent against embolism associated with atrial fibrillation/artificial valve or valvular heart disease (and preferably, a prophylactic agent against the onset of cerebral embolism associated with these diseases); or a prophylactic and/or therapeutic agent for (and in particular, a prophylactic agent against the recurrence of) transient ischemic attack; or
   4) a prophylactic and/or therapeutic agent for DIC; a prophylactic and/or therapeutic agent for influenza virus infection; or a prophylactic and/or therapeutic agent for deep vein thrombosis.
[2-b] The second aspect of the present invention also provides a prophylactic and/or therapeutic process in which an effective amount of the pharmaceutical composition is administered to patients suffering from the diseases as mentioned above.
[2-c] The second aspect of the present invention also provides use of the compound represented by the formula (I) or (Im), the compound described in the above [1-1] to [1-9], or a pharmaceutically acceptable salt thereof for producing a drug for preventing and/or treating the diseases as mentioned above.

The pharmaceutical composition of the present invention contains at least one compound represented by the formula (I) (definition of the formula is as described above) or its salt as its effective component, and it may also contain a pharmaceutically acceptable carrier. Preferable examples of the compound of the formula (I) are as described in the above [1-1] to [1-9].

[3] A third aspect of the present invention provides an FXa inhibitor containing the compound represented by the formula (I) or (Im), the compound described in the above [1-1] to [1-9], or a pharmaceutically acceptable salt thereof; and
[3-a] More specifically, the third aspect of the present invention relates to a specific FXa inhibitor containing the compound represented by the formula (I) or (Im), the compound described in the above [1-1] to [1-9], or a pharmaceutically acceptable salt thereof as its effective component; and also, to an orally administerable FXa inhibitor, and an orally administerable specific FXa inhibitor.
[3-b] The third aspect of the present invention also provides a reagent using the compound represented by the formula (I) or (Im), the compound described in the above [1-1] to [1-9], or a pharmaceutically acceptable salt thereof. Exemplary such reagents include a reagent which diagnoses abnormal blood coagulability of a mammal by using the FXa inhibitory action, and a reagent used in physiological experiments which uses quantitative FXa inhibitory action.
[3-c] The third aspect of the present invention also provides a prophylactic and/or therapeutic method in which an effective amount of the FXa inhibitor is administered to a patient.
[3-d] The third aspect of the present invention also provides use of the compound represented by the formula (I) or (Im), the compound described in the above [1-1] to [1-9], or a pharmaceutically acceptable salt thereof for producing the FXa inhibitor.

In all of the aspects as described above, the expression "compound" should be deemed to also include "the pharmaceutically acceptable salt thereof". The compound of the present invention may include an asymmetric carbon, and the compound of the present invention may be a mixture or an isolation product of geometric isomer, tautomer, optical isomer or other stereoisomer. Isolation or purification of such stereoisomer may be accomplished by those skilled in the art using any of the techniques commonly used in the art, for example, by optical resolution using preferential crystallization or column chromatography, or by asymmetric synthesis.

The compound (I) of the present invention may be in the form of an acid addition salt, and depending on the type of the substituent, the compound (I) may also be in the form of a salt with a base. Such salt is not particularly limited as long as the salt is a pharmaceutically acceptable salt, and exemplary salts include acid addition salts with a mineral acid such as hydrochloric acid, hydrobromic acid, hydroiodic acid, sulfuric acid, nitric acid, or phosphoric acid; an organic carboxylic acid such as acetic acid, propionic acid, oxalic acid, malonic acid, succinic acid, fumaric acid, maleic acid, lactic acid, formic acid, malic acid, tartaric acid, citric acid, or mandelic acid; an organic sulfonic acid such as methanesulfonic acid, ethanesulfonic acid, p-toluenesulfonic acid, or 2-hydroxyethanesulfonic acid; or acidic amino acid such as aspartic acid or glutamic acid; and salts with a base of an alkaline metal or alkaline earth metal such as sodium, potassium, magnesium, calcium, or aluminum and an organic base such as methylamine, ethylamine, ethanolamine, pyridine, lysine, arginine, or ornithine; and ammonium salt.
Furthermore, the salts of the compound of the present invention also include mono-salts, di-salts, and tri-salts. Still further, the compound of the present invention may simultaneously form an acid addition salt and a salt with a base depending on the type of the substituent on the side chain.
Still further, the present invention also includes hydrates of the compound (I) as well as pharmaceutically acceptable solvates and crystalline polymorphic forms of the compound (I). It should also be taken for granted that the present invention is by no means limited to the compounds mentioned in the Examples as described below, and all of the tricyclic compounds having the spiro union as represented by the formula (I) and their pharmaceutically acceptable salts are within the scope of the present invention.
It would be understood that such situation also applies to the compounds of the formula (Im).

Next, the therapeutic and/or prophylactic agent and the pharmaceutical composition of the present invention are described. The pharmaceutical composition of the present invention contains at least one compound represented by the formula (I) or (Im) (definition of the formula is as described above) as its effective component, and it may also contain a pharmaceutically acceptable carrier. Preferable examples of the compound of the formula (I) are as described above.

### <FXa inhibitory action of the compound of the present invention>

The compound of the present invention has strong FXa inhibitory activity. In other words, the composition of the present invention is a strong FXa inhibitor, and more specifically, a highly specific FXa inhibitor which does not inhibit other enzymes.
The composition of the present invention is also a FXa inhibitor which can be orally administered, and more specifically, a specific FXa inhibitor which can be orally administered. The compound of the present invention specifically and strongly inhibits activity of the FXa among the many serine proteases. To be more specific, it does not inhibit trypsin or chymotrypsin at all, and in addition, it does not at all inhibit thrombin which is a serine protease in the same blood coagulation system. Thus, problems such as bleeding tendency associated with the use of a thrombin inhibitor have been overcome. Still further, the compound of the present invention is well absorbed from digestive tracts after oral administration with no activity reduced by the absorption, and it also exhibits favorable absorption, distribution, metabolism, and excretion characteristics. Its value as an orally administerable agent is quite high.

The composition containing the compound of the present invention is a prophylactic and/or therapeutic agent for diseases wherein an FXa inhibitor is useful. The composition containing the compound of the present invention is also an anticoagulant which is a prophylactic and/or therapeutic agent for diseases wherein the anticoagulant is useful. To be more specific, such agent is effective in prevention and/or treatment of diseases caused by thrombus or embolus. Specific examples of such diseases include: diseases from ischemic cerebrovascular disorders such as cerebral thrombosis, brain infarction, cerebral embolism, transient cerebral ischemic attack (TIA) and cerebrovascular spasm after subarachnoid hemorrhage; Alzheimer's disease, cerebrovascular dementia, asymptomatic cerebrovascular disorder, diseases associated with ischemic heart diseases such as acute and chronic myocardial infarction, sequelae of myocardial infarction, unstable angina pectoris, angina pectoris and coronary artery thrombolysis; thrombogenesis after artificial blood vessel or artificial valve replacement, reocclusion and restenosis after coronary artery bypass grafting, reocclusion and restenosis after PTCA or PTCA or stent placement, pulmonary infarction, pulmonary thrombosis/pulmonary embolism, diseases associated with pulmonary vascular disorder (for example, drug-induced pneumonia), acute respiratory distress syndrome (ARDS), acute nephritis, acute progressive nephritis, chronic nephritis (for example, diabetic nephropathy, chronic glomerulonephritis, and IgA nephropathy), acute arterial occlusion, thromboangiitis obliterans (Buerger's disease), arteriosclerosis obliterans, peripheral arterial occlusive disease, peripheral venous occlussive disease, deep vein thrombosis, thrombophlebitis, disseminated intravascular coagulation (DIC), organ failures induced with the progress of the shock or DIC, thrombotic microangiopathy (TMA), systemic inflammatory response syndrome (SIRS), thrombotic thrombocytopenic purpura, hemolytic uremic syndrome (HUS), diseases associated with various vascular disorders such as thrombogenesis in extracorporeal circulation, thrombocytopenia in major operation, arterial sclerosis, cancer metastasis, rejection in transplantation, and organ protection or functional improvement in transplantation. Also included are prophylaxis of vascular endothelial cell injury associated with diabetes, hypercoagulability associated with transplantation or activated protein C (APC) resistance, excessive blood coagulation associated with vascular disease, injury after operation, obesity, pregnancy, use of oral contraceptive, sustained depression, heparin-induced thrombocytopenia, collagen disease (for example, antiphospholipid antibody syndrome, polyarteritis, and systemic lupus erythematosus), Bechet's disease, ischemic reperfusion injury, cancer or the like, and toxemia of pregnancy.
The agent of the present invention is particularly adapted for use in prevention of embolism associated with atrial fibrillation/artificial valve or valvular heart disease, and preferably for prevention of onset of cerebral embolism, prevention of transient cerebral ischemic attack and especially for prevention of recurrence of the transient cerebral ischemic attack, and prevention/treatment of deep vein thrombosis or DIC.

When the agent of the present invention is used as a drug for these diseases, preventive administration is recommended and such use is particularly important since the agent of the present invention is neither a direct thrombolytic agent nor a direct platelet aggregation inhibitor. In other words, the agent of the present invention is adapted for preventive use in patients suffering from thrombophilia or patients having the risk factor of thrombosis/embolism for the purpose of preventing thrombus/embolus. In the case of the patients with atrial fibrillation/artificial valve or valvular heart disease, a thrombus is easily generated at the site of the lesion or the transplantation, and such thrombus often triggers cerebral infarction, which is more than often a fatal attack. The agent of the present invention has a good potential to be a potent drug for preventing onset of the thrombosis/embolism, and in particular, cerebral embolism induced in such patients.
Such therapy is continued for a long time. The agent of the present invention can be orally administered with fewer side effects such as bleeding, and therefore, the agent of the present invention can be reliably used for a long time with no need of frequent monitoring.

In other words, the agent of the present invention is a prophylactic and/or therapeutic agent for embolism associated with atrial fibrillation/artificial valve or valvular heart disease. The agent of the present invention is preferably a prophylactic agent against the onset of cerebral embolism associated with such disease. The agent of the present invention is also a prophylactic and/or therapeutic agent for transient cerebral ischemic attack, and in particular, a prophylactic agent for preventing the recurrence of the onset of transient cerebral ischemic attack; and a prophylactic and/or therapeutic agent for deep vein thrombosis or DIC.
In addition, some compounds of the present invention are easily metabolized in the course of the absorption and secretion of the pharmaceutical substance by the substituent in D, and some of the thus produced metabolites are within the scope of the compound of the present invention as represented by the formula (I), and exhibit a potent FXa inhibitory activity. This is a finding quite interesting in pharmacological/pharmacokinetical point of view.
The composition containing the compound of the present invention as an active ingredient is also effective as a veterinary drug and has high value of use. The composition is also useful as a reagent adapted for use in measuring various blood coagulation functions and as a laboratory reagent.
Owing to the FXa inhibitory action of the compound of the present invention, such composition is also useful as a prophylactic/therapeutic agent against infection with influenza virus based on the inhibitory activity against the propagation of the influenza virus, and also, as a prophylactic/therapeutic agent against periodontal disease.

### [Production method of the compound of the present invention]

Next, the production method for the derivative of the formula (I) of the present invention is described.
The derivative of the formula (I) and the salt and solvate thereof according to the present invention can be produced by a combination of chemical processes commonly known to the art, and in particular, by the processes similar to those described in WO 01/02397 and WO 02/053568. Typical production methods are as described below.
Unless otherwise noted, D, Q, T, Y, l, m, n, and r in the formulae shown in the description of the production method are as defined above for the formula (I). The alkylene group in the side chain or ring of the compound may be substituted with the substituents defined for the formula (I).
Unless otherwise noted, P¹ and P² in the production method independently designate protecting group of the imino group (-NH-), and exemplary appropriate protecting groups include typical acyl protecting groups, namely, an alkanoyl group such as acetyl group; an alkoxycarbonyl group such as methoxycarbonyl group, ethoxycarbonyl group, or t-butoxycarbonyl group; an arylmethoxycarbonyl group such as benzyloxycarbonyl group, paramethoxybenzyloxycarbonyl group, or para(ortho)nitrobenzyloxycarbonyl group; an arylmethyl group such as benzyl group or triphenylmethyl group; or an aroyl group such as benzoyl group. The method used for deprotecting such protecting group differs depending on the chemical nature of the protecting group employed, and in the case of an acyl protecting group such as an alkanoyl group, an alkoxycarbonyl group, or aroyl group, the deprotection can be accomplished by the hydrolysis using an appropriate base such as an alkaline metal hydroxide such as lithium hydroxide, sodium hydroxide, or potassium hydroxide.

The substituted methoxycarbonyl protecting group such as t-butoxycarbonyl group or paramethoxybenzyloxycarbonyl group can be removed by an appropriate acid such as acetic acid, hydrochloric acid, hydrobromic acid, sulfuric acid, phosphoric acid, trifluoroacetic acid, trifluoromethanesulfonic acid, or a combination thereof. The arylmethoxycarbonyl group such as benzyloxycarbonyl group, paramethoxybenzyloxycarbonyl group, or para(ortho)nitrobenzyloxycarbonyl group and the arylmethyl group such as benzyl group can be removed by the hydrolysis using a palladium-carbon catalyst. The benzyl group can be removed by Birch reduction using metal sodium in liquid ammonia for conversion into nitrogen-hydrogen bond. The triphenylmethyl group can be removed by using an appropriate acid such as acetic acid, hydrochloric acid, hydrobromic acid, sulfuric acid, phosphoric acid, trifluoroacetic acid, trifluoromethanesulfonic acid, or a combination thereof, or alternatively, by Birch reduction using metal sodium in liquid ammonia, or hydrolysis using a palladium-carbon catalyst.
The protecting groups P¹ and P² of the imino group (-NH-) can be independently or simultaneously deprotected by adequately selecting the type of the protecting group and deprotection conditions, and if desired, the protecting group can be re-introduced.

Unless otherwise noted, "W" in the production method designates a leaving group such as a halogen atom (for example, fluorine, chlorine, bromine, or iodine), methanesulfonyloxy group, or p-toluenesulfonyloxy group, or a replaceable substituent such as hydroxy group or an alkoxy group.
"J" designates a thiol protecting group such as p-methoxybenzyl group.
It should be noted that, when the derivative of the formula (I) of the present invention synthesized has a reactive group such as hydroxy group, amino group, carboxyl group, or thiol group as its substituent, such group may be adequately protected with a protective group in each reaction step and the protective group may be removed at an adequate stage. The process of such introduction and removal of the protective group may be adequately determined depending on the group to be protected and the type of the protective group, and such introduction and removal are conducted, for example, by the process described in the review section of "Protective Groups in Organic Synthesis", Second edition, 1991, John Wiley & Sons, Inc. The required starting materials are either commercially available, or capable of being readily synthesized by the method commonly used in the organic chemistry from commercially available products. Unless otherwise noted, the reaction conditions employed in the production method are as described below: Reaction temperature is in the range of -78°C to the solvent-reflux temperature, and reaction time is the time sufficient for required progress of the reaction. Solvent which is not involved in the reaction may be any of the aromatic hydrocarbon solvents such as toluene and benzene; polar solvents such as water, methanol, DMF, and DMSO; basic solvents such as triethylamine and pyridine; halogen solvents such as chloroform, methylene chloride, and 1,2-dichloroethane; ethereal solvent such as diethylether, tetrahydrofuran, and dioxane; and mixed solvents thereof; and the solvent used may be adequately selected depending on the reaction conditions. Base may be any of inorganic bases such as potassium carbonate, cesium carbonate, sodium hydroxide, potassium hydroxide, and sodium hydride; and organic bases such as triethylamine, pyridine, N,N-dialkylaniline, and lithium diisopropylamide; and acid may be any of mineral acids such as hydrochloric acid, and sulfuric acid; and organic acids such as methanesulfonic acid and p-toluenesulfonic acid. The base and the acid are not necessarily limited to those mentioned above.

Next, the production method is described. The present invention, however, is by no means limited to the processes as described below.

### (1) Next, the method for producing the derivative of the formula (I) is described.

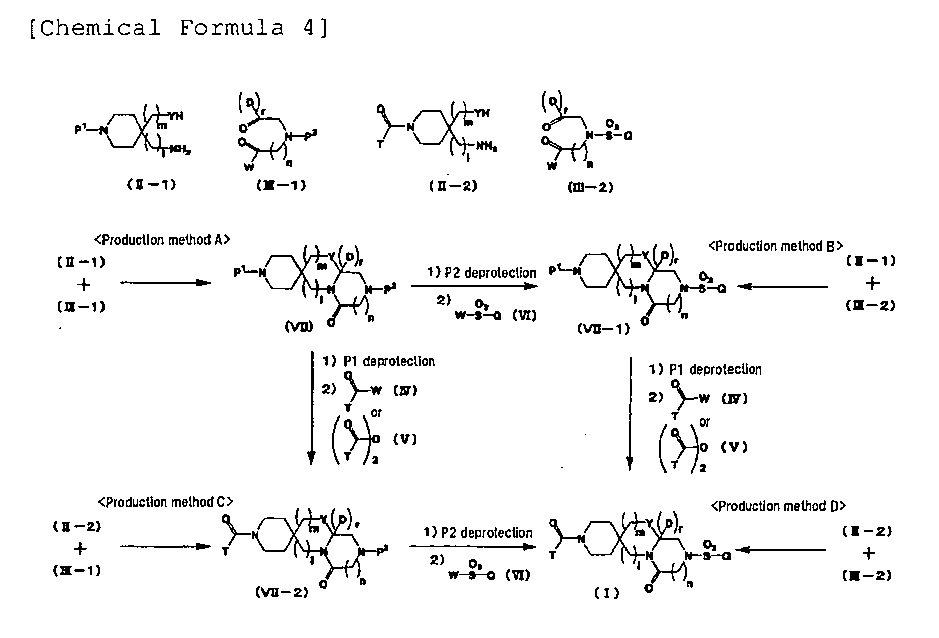

### <Production method A>

The compound represented by the formula (I) or its salt can also be produced by a method wherein the compound of the formula (VII): 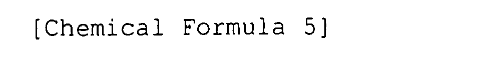 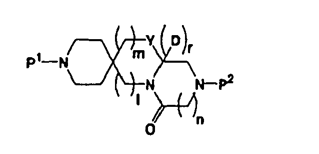 (wherein D, P¹, P², Y, l, m, n, r and substitution of the alkylene chain are as defined above) or its salt is produced as an intermediate.
In this method, the compounds represented by the formula (II-1) and the formula (III-1):

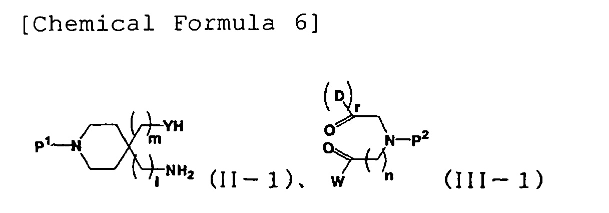

(wherein D, P¹, P², Y, l, m, n, r, and W, and substitution of each alkylene chain are as defined above) or their salts which are commercially available or readily derived from commercially available compounds may be reacted in accordance with a known process described in a document (for example, Journal of Medicinal Chemistry, vol. 19, page 436, 1976; Journal of American Chemical Society, vol. 107, page 7776, 1985; or Journal of Organic Chemistry, vol. 63, page 1732, 1998) preferably by using toluene for the solvent in the presence or absence of an acid catalyst, and preferably, in the presence of p-toluenesulfonic acid. The reaction may be promoted at a temperature in the range of 0°C to the solvent reflux temperature, and preferably at the solvent reflux temperature for a time sufficient for the progress of the required reaction, and preferably for 2 to 6 hours to produce the compound represented by the formula (VII) or its salt.

The protecting group P1 of the compound represented by the formula (VII) or its salt is deprotected by the method appropriate for the type of the protecting group, and the deprotected product is then reacted with the compound represented by the formula (IV) or (V): 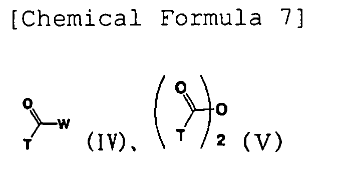 (wherein T and W are as defined above) or its salt which is commercially available or readily derived from a commercially available compound for formation of an amide to thereby produce the compound represented by the formula (VII-2) or its salt.

The protecting group P2 of the compound represented by the formula (VII-2) or its salt is deprotected by the method appropriate for the type of the protecting group, and the deprotected product is then reacted with the compound represented by the formula (VI): 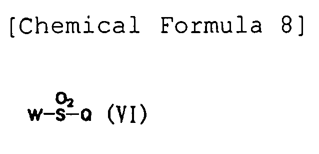 (wherein Q and W are as defined above) or its salt which is commercially available or readily derived from a commercially available compound for formation of a sulfonamide to thereby produce the compound represented by the formula (I) or its salt.

The protecting group P2 of the compound represented by the formula (VII) or its salt is deprotected by the method appropriate for the type of the protecting group, and the deprotected product is then reacted with the compound represented by the formula (VI): 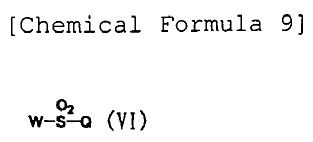 (wherein Q and W are as defined above) or its salt which is commercially available or readily derived from a commercially available compound for formation of a sulfonamide to thereby produce the compound represented by the formula (VII-1) or its salt.

The protecting group P1 of the compound represented by the formula (VII-1) or its salt is deprotected by the method appropriate for the type of the protecting group, and the deprotected product is then reacted with the compound represented by the formula (IV) or the formula (V) : 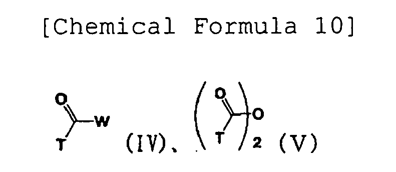 (wherein T and W are as defined above) or its salt which is commercially available or readily derived from a commercially available compound for formation of an amide to thereby produce the compound represented by the formula (I) or its salt.

### <Production method B>

The compound represented by the formula (I) or its salt can also be produced by a method wherein the compound of the formula (VII-1): 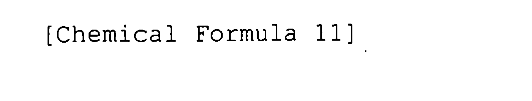 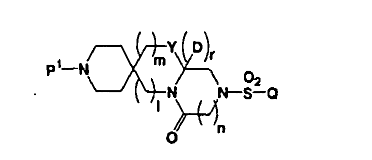 (wherein D, P¹, Q, Y, l, m, n, r, and substitution of the alkylene chain are as defined above) or its salt is produced as an intermediate.

In this method, the compounds represented by the formula (II-1) and the formula (III-2): 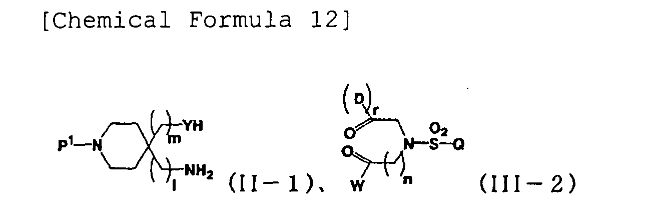 (wherein D, P¹, Q, Y, l, m, n, r, W, and the substitution of the alkylene chain are as defined above) or their salts which are commercially available or readily derived from commercially available compounds may be used according to the method described in <Production method A> to produce the compound represented by the formula (VII-1) or its salt.

The protecting group P¹ of the compound represented by the formula (VII-1) or its salt is deprotected by the method appropriate for the type of the protecting group, and the deprotected product is then reacted with the compound represented by the formula (IV) or (V):

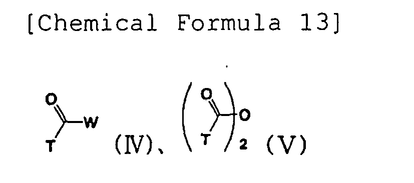

(wherein T and W are as defined above) or its salt which is commercially available or readily derived from a commercially available compound for formation of an amide to thereby produce the compound represented by the formula (I) or its salt. When W is a halogen atom, hydroxyl group, or an alkoxy group in the compound represented by the formula (IV), amidation in normal peptide is carried out. For example, when W is hydroxyl group, a phenol such as 2,4,5-trichlorophenol, pentachlorophenol, 2-nitrophenol, or 4-nitrophenol, or a N-hydroxy compound such as N-hydroxysuccinimide, N-hydroxy-5-norbornene-endo-2,3-dicarboxyimide or N-hydroxypiperidine is condensed in the presence of a condensing agent such as N,N-dicyclohexylcarbodiimide for conversion into active ester form, and allowed for reaction. Alternatively, the reaction may be conducted after producing a mixed acid anhydride by reacting with a halogenated acyl compound such as isobutyl chloroformate. The reaction may be also promoted by using a peptide condensation reagent such as N,N-dicyclohexylcarbodiimide, diphenylphosphoric acid azide or diethyl cyanophosphate alone.

### <Production method C>

The compound represented by the formula (I) or its salt can also be produced by a method wherein the compound of the formula (VII-2): 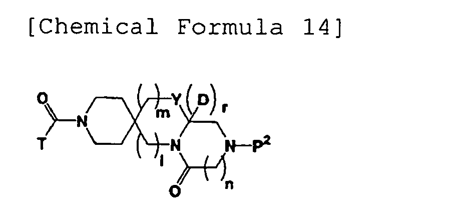 (wherein D, P², T, Y, l, m, n, r, and substitution of the alkylene chain are as defined above) or its salt is produced as an intermediate.

In this method, the compounds represented by the formula (II-2) and the formula (III-1): 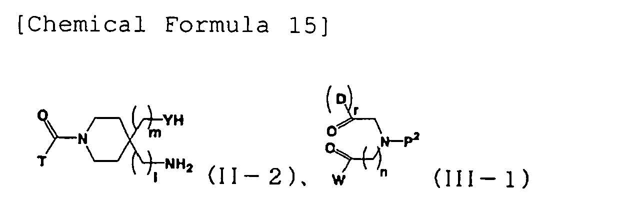 (wherein D, P², T, Y, l, m, n, r, W, and substitution of the alkylene chain are as defined above) or their salts which are commercially available or readily derived from commercially available compounds may be used according to the method described in <Production method A> to produce the compound represented by the formula (VII-2) or its salt.

The protecting group P² of the compound represented by the formula (VII-2) or its salt is deprotected by the method appropriate for the type of the protecting group, and the deprotected product is then reacted with the compound represented by the formula (VI): 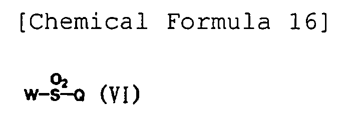 (wherein Q and W are as defined above) or its salt which is commercially available or readily derived from a commercially available compound for formation of a sulfonamide to thereby produce the compound represented by the formula (I) or its salt. When W is chlorine atom, reaction may be conducted in methylene chloride in the presence of triethylamine at 0°C to room temperature and preferably at room temperature for 2 to 12 hours.

### <Production method D>

The compound represented by the formula (I): 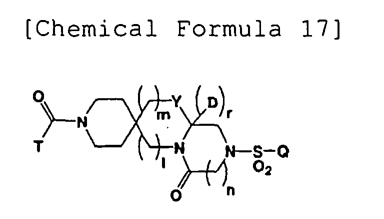 (wherein D, Q, T, Y, l, m, n, r, and substitution of the alkylene chain are as defined above) or its salt can also be produced by the method as described below.

In this method, the compounds represented by the formula (II-2) and the formula (III-2): 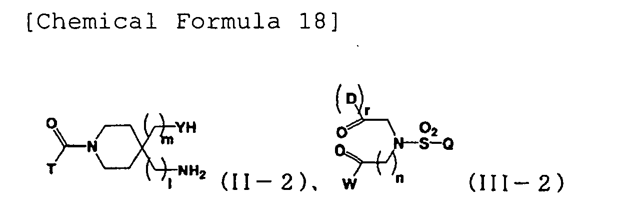 (wherein D, Q, T, Y, l, m, n, r, W, and substitution of the alkylene chain are as defined above) or their salts which are commercially available or readily derived from commercially available compounds may be used according to the method described in <Production method A> to produce the compound represented by the formula (I) or its salt.

In the Production methods A to D, a compound represented by the formula (VIII), (VIII-1), (VIII-2), or (VIII-3) or its salt can often be isolated, and in such case, the isolated compound can be converted to the corresponding compound represented by the formula (VII), (VII-1), (VII-2), or (I) or its salt by the amide formation reaction described in the <Production method A>.

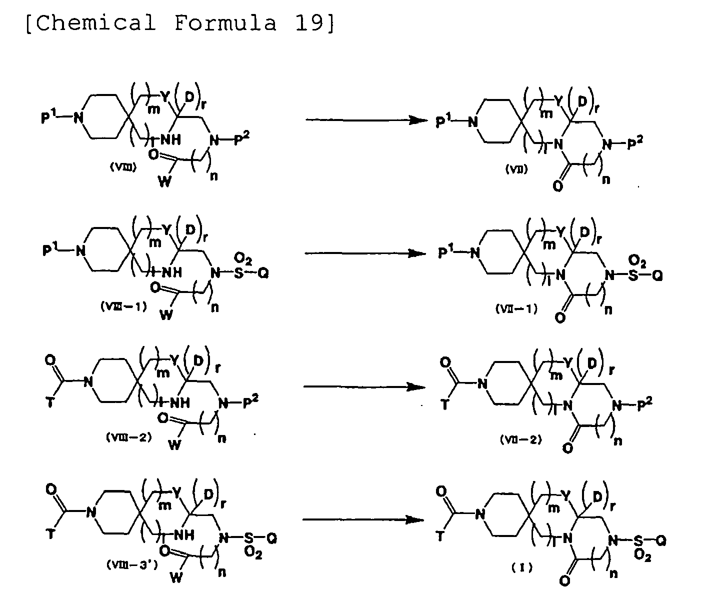

### <Production method E>

The compound represented by the formula (I) wherein r is 0 and D is H (hydrogen atom), namely, the compound represented by the formula (I-a): 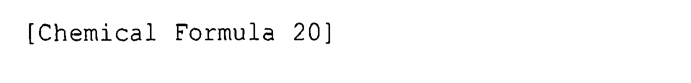 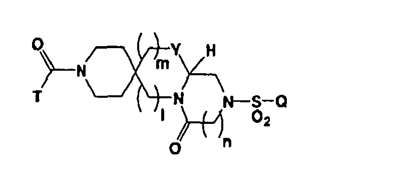 (wherein Q, T, Y, l, m, n, and substitution of the alkylene chain are as defined above) or its salt can also be produced by the method as described below.

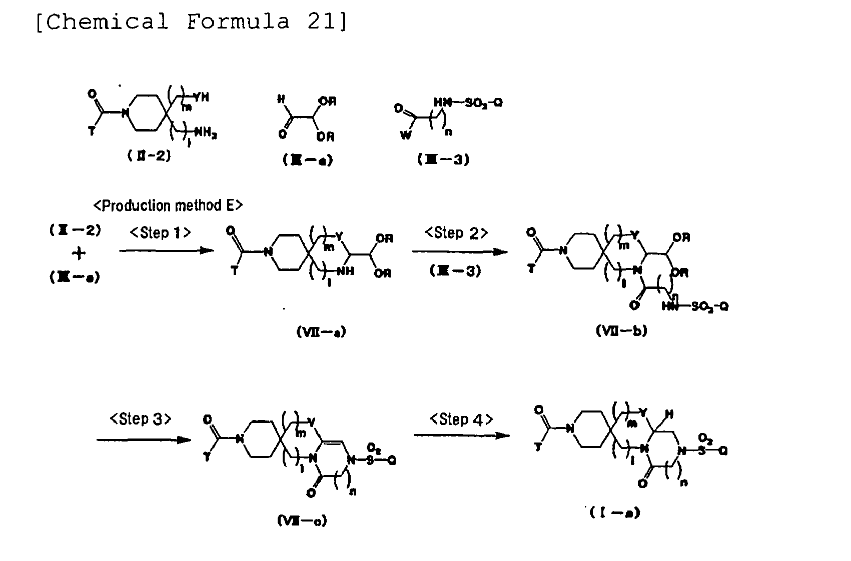

### <Step 1>

In this method, the compounds represented by the formula (II-2) and the formula (III-a): 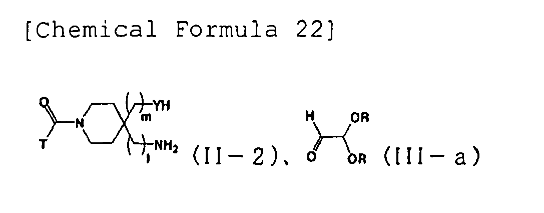 (wherein T, Y, l, m, and substitution of the alkylene chain are as defined above; and R is independently a C₁₋₆ alkyl group (and in particular, methyl group or ethyl group) optionally substituted, for example, with hydrogen atom, a C₁₋₆ alkyl, a C₁₋₆ alkoxy, hydroxy, or a halogen atom, or two Rs together representing a C₂₋₄ alkylene group (and in particular, 1,2-ethylene group or 1,3-propylene group) optionally substituted with a C₁₋₆ alkyl, a C₁₋₆ alkoxy, hydroxy, or a halogen atom) or their salts which are commercially available or readily derived from commercially available compounds may be used according to the method described in <Production method A> to produce the compound represented by the formula (VIIa) or its salt.

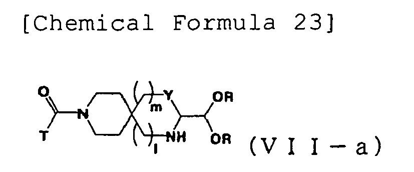

### <Step 2>

Next, a reaction is allowed to proceed by using the compound represented by the formula (VII-a) or its salt obtained in <Step 1> and the compound represented by the formula (III-3): 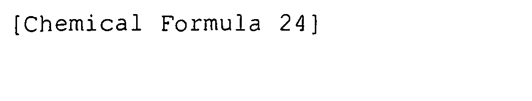 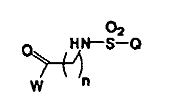 (wherein Q, W, n, and substitution of the alkylene chain are as defined above) or its salt to thereby produce the compound represented by the formula (VII-b) or its salt.

When W is a halogen atom, hydroxyl group, or an alkoxy group in the compound represented by the formula (III-3) or its salt, amidation in normal peptide is carried out. For example, when W is hydroxyl group, a phenol such as 2,4,5-trichlorophenol, pentachlorophenol, 2-nitrophenol, or 4-nitrophenol or a N-hydroxy compound such as N-hydroxysuccinimide, N-hydroxy-5-norbornene-endo-2,3-dicarboxyimide, or N-hydroxypiperidine may be condensed in the presence of a condensing agent such as N,N-dicyclohexylcarbodiimide for conversion into active ester form, and used in the reaction.
Alternatively, the reaction may be conducted after producing an acid anhydride mixture by the reaction with a halogenated acyl compound such as isobutyl chloroformate. The reaction may also be promoted by using a peptide condensation reagent such as N,N-dicyclohexylcarbodiimide, diphenylphosphoric acid azide, or diethyl cyanophosphate alone.
When W is hydroxyl group, the compound represented by the formula (III-3) is activated by using a phosphorus compound such as triphenylphosphine or tributylphosphine and an azodicarboxylate such as diethyl azodicarboxylate, and the reaction may be conducted in a solvent which will not be involved in the reaction.

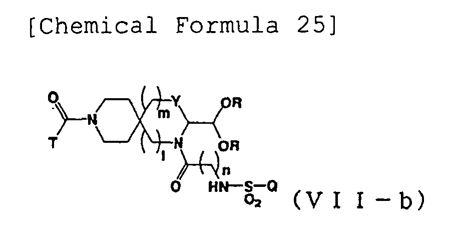

### <Step 3>

Next, the compound represented by the formula (VII-b) (wherein Q, T, Y, l, m, n, and substitution of the alkylene chain are as defined above, and R is as defined above for R of the formula (III-a)) or its salt produced in the <Step 2> is used in a process according to the method disclosed in a publication, for example, JP 09-316059 A in a solvent which will not be involved in the reaction, and is preferably toluene in the presence of an acid catalyst, and preferably p-toluenesulfonic acid to thereby produce the compound represented by the formula (VII-c) or its salt. The reaction is preferably conducted at a temperature of 70°C to 80°C for a reaction time of 1 to 2 hours.

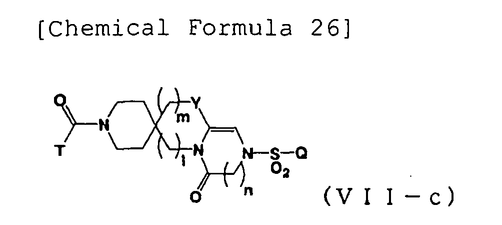

### <Step 4>

Next, the compound represented by the formula (VII-c) (wherein Q, T, Y, l, m, n, and substitution of the alkylene chain are as defined above) or its salt produced in the <Step 3> may be reacted for reduction of the double bond in the formula to produce the compound represented by the formula (I-a) or its salt. Exemplary reduction processes include reduction by a metal such as sodium, calcium, or aluminum, or a metal salt; reduction by a metal hydride such as diisopropylaluminum hydride; and reduction by a metal hydride complex such as sodium borohydride; electrophilic reduction by diborane or substituted borane; and catalytic hydrogenation using a metal catalyst. The reaction solvent used is a solvent which is not involved in the reaction, for example, tetrahydrofuran, toluene, methylene chloride, or methanol, or a mixture thereof, and the reaction is conducted at a temperature of -78°C to reflux temperature for a time sufficient for required progress of the reaction.

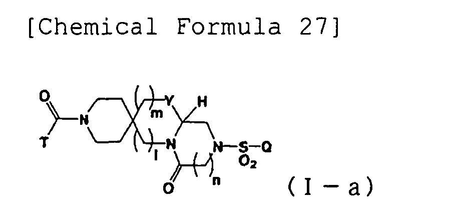

The compounds used for the starting materials in the Production methods A to E, namely, the compounds of the formula (II-1), (II-2), (III-1), and (III-2) may be produced, for example, by the methods known in the art such as those described below or similar methods.

### (2) Next, the production method of the compounds represented by the formula (II-1) and the formula (II-2) is described.

The compounds represented by the formula (II-1) and the formula (II-2): 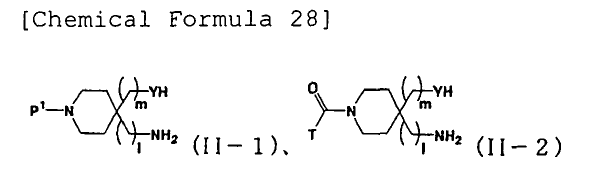 (wherein P¹, T, Y, l, m, and substitution of the alkylene chain are as defined above) or their salts can be produced by a method known in a publication, for example, by a method described in WO 01/02397 or WO 02/053568.

As described below, the protecting group P¹ is deprotected by the method appropriate for the type of the protecting group, and the deprotected product is then reacted with the compound represented by the formula (IV) or (V): 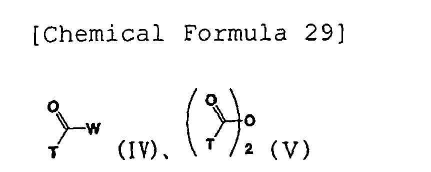 (wherein T and W are as defined above) or its salt which is commercially available or readily derived from a commercially available compound for amide formation. Under the condition under which the reaction is not affected, compounds or their salts in which the protecting group P¹ has been converted to T-CO- can be produced.

### <Production method F>

When m is 0, l is 1, and Y is O (oxygen atom), the compound represented by the formula (II-a) or its salt can be produced according to the production method as described below: 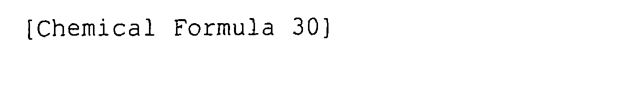 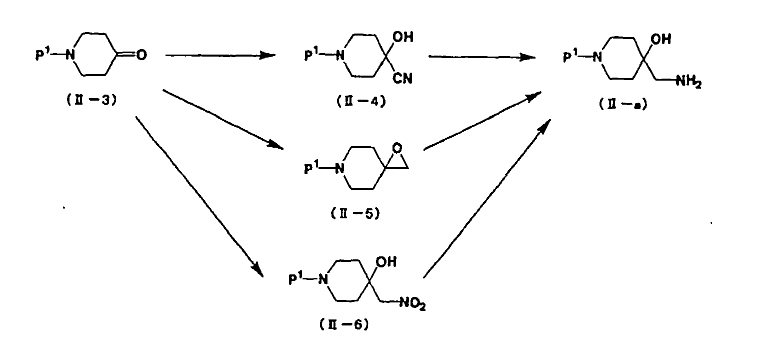 (wherein P¹ is as defined above).

### <Production method G>

When m is 0, l is 1, and Y is S (sulfur atom), the compound represented by the formula (II-b) or its salt can be produced according to the production method as described below: 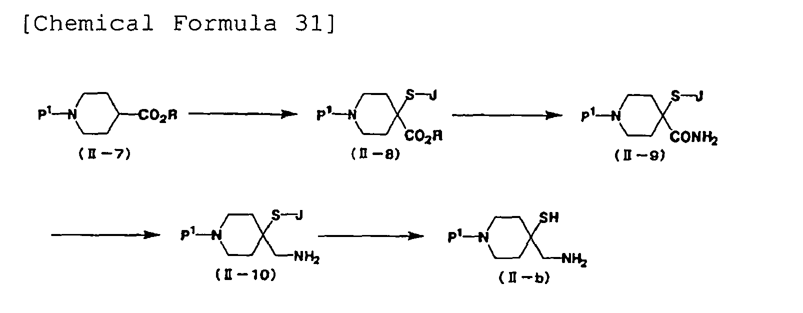 (wherein P¹ and J are as defined above).

### <Production method H>

When m is 0, l is 1, and Y is imino group (-NH-), the compound represented by the formula (II-c) or its salt can be produced according to the production method as described below: 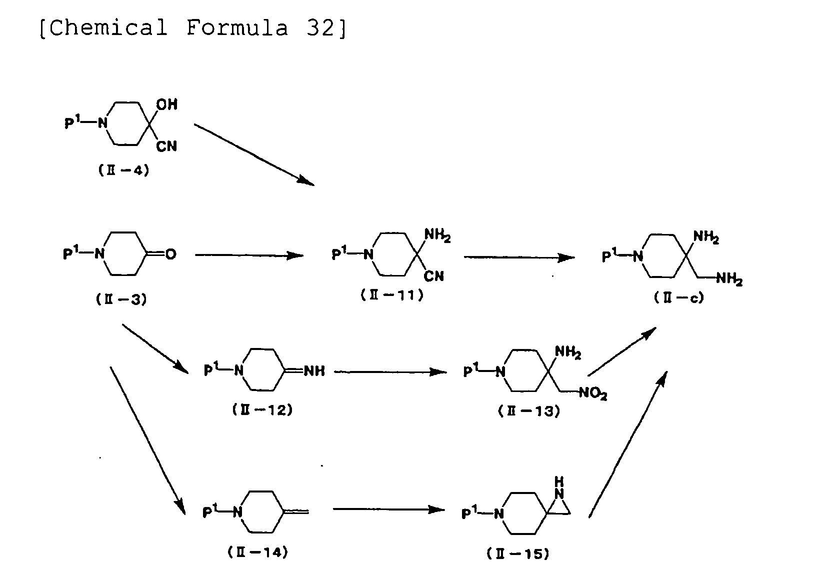 (wherein P¹ and J are as defined above).

### <Production method I>

When m is 1, l is 0, and Y is O (oxygen atom), the compound represented by the formula (II-d) or its salt can be produced according to the production method as described below: 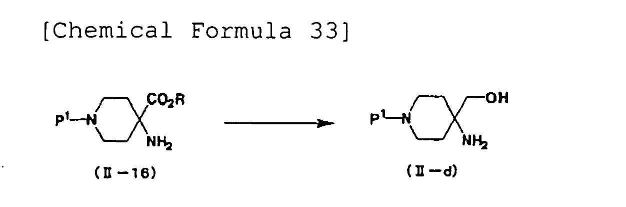 (wherein P¹ is as defined above) .

### <Production method J>

When m is 1 or 2, l is 1, and Y is O (oxygen atom), the compound represented by the formula (II-e) or its salt can be produced according to the production method as described below: 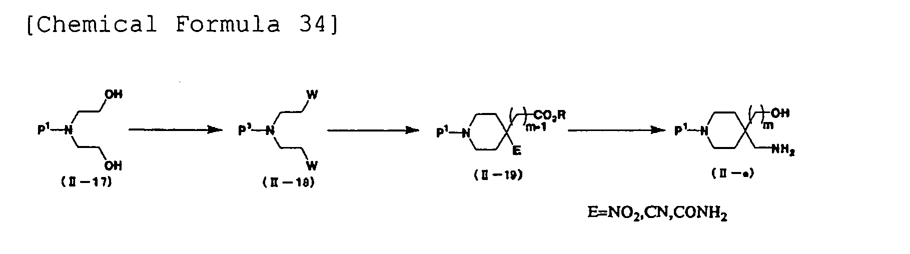 (wherein P¹, W, and E are as defined above).

### <Production method K>

When m is 1 or 2, l is 1, and Y is imino group (-NH-), the compound represented by the formula (II-f) or its salt can be produced according to the production method as described below: 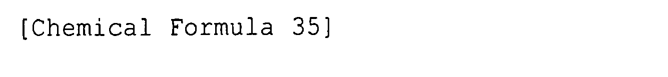 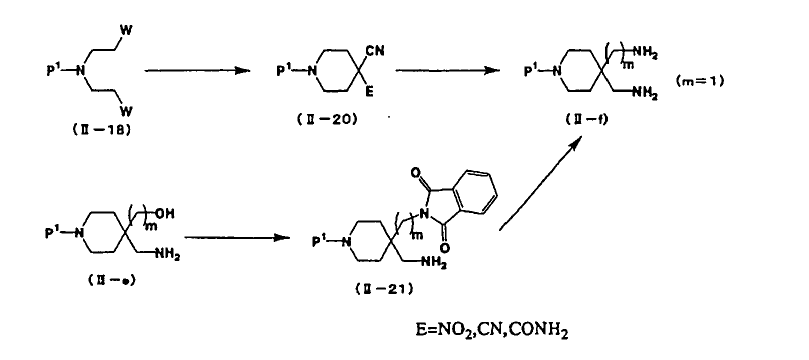 (wherein P¹ and E are as defined above).

### (3) Production method of the compounds represented by the formula (III-1) and the formula (III-2)

The compounds represented by the formula (III-1) and the formula (III-2): 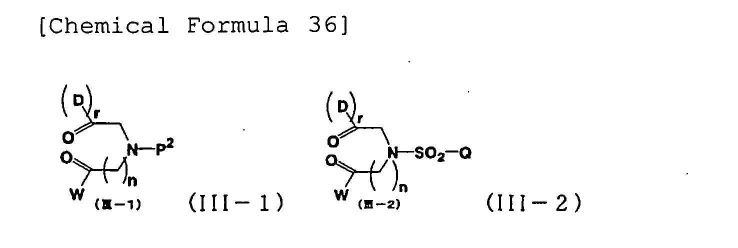 (wherein D, P², Q, W, r, and substitution of the alkylene chain are as defined above) or their salts can be produced by a method known in a publication, for example, by a method described in WO 01/02397 or WO 02/053568.

Next, conversion of the substituent D is described.
The conversion of the substituent may be conducted in any reaction stage in the <Production method A>, <Production method B>, <Production method C>, or <Production method D>, or in the stage of each compound as the starting material or in any reaction stage for producing such starting material.
In synthesizing the compound (I) of the present invention, those skilled in the art may select the most adequate stage for the substituent conversion.
For the ease of understanding, an exemplary conversion of the substituent D from -CH₂OH is described. In this case, the compound in which the substituent D is -CH₂OH is either the one obtained by the procedure as described above from the starting material compound in which the substituent D is -CH₂OH or the one obtained by deprotection of the compound in which the substituent D has been -CH₂OP³ wherein P³ represents the protecting group of the hydroxy (-OH) group. Suitable examples of the protecting group include ester protecting groups such as acetate ester, formate ester, and acetate ester substituted with trifluoro, chloro, or chloromethyl; silyl ether protecting groups such as trimethylsilyl, triethylsilyl, t-butyldimethylsilyl, t-butyldiphenylsilyl, and triisopropylsilyl; alkyl ether protecting groups such as methyl, benzyl, p-methoxybenzyl, 3,4-dimethoxybenzyl, trityl, and t-butyl; and acetal protecting groups such as methoxymethyl, (2-methoxyethoxy)methyl, and benzyloxymethyl. The method used for the deprotection of such protecting group may differ depending on the chemical nature of the protecting group employed. For example, in the case of the ester protecting group, the deprotection may be accomplished by hydrolysis under a basic condition using an inorganic salt such as potassium carbonate, potassium hydrogencarbonate, potassium hydroxide, or sodium hydroxide, or an organic base such as ammonia, pyridine, or triethylamine. In the case of silyl ether protecting group, the deprotection may be accomplished by heating in a large amount of alcohol such as methanol or by using a fluorine anion such as tetrabutylammonium fluoride. In the case of alkyl ether protecting group, the deprotection may be accomplished by using a Lewis acid such as boron trichloride, boron tribromide or aluminum chloride, or a strong acid such as trifluoroacetic acid or bromic acid.
In the case of acetal protecting group, the deprotection may be accomplished by acid hydrolysis.

### 1) Substituent D convertible from -CH₂OH

### 1-1) Production of a compound wherein D is -CH₂-OR' (wherein R' is an optionally substituted C₁₋₆ alkyl group) or its salt

### 1-1-1) Production process using R'-W

The compound wherein D' is -CH₂OH or its salt may be reacted with a compound represented by the formula: R'-W in a solvent which is not involved in the reaction, and preferably, in the mixed solvent of methylene chloride and water in the presence of a base, and preferably, using sodium hydroxide in the presence or absence of a phase transfer catalyst such as quaternary ammonium salt or crown ether, and preferably, in the presence of benzyltriethylammonium chloride at a temperature of -78°C to reflux temperature, and preferably, at 0°C for a time sufficient for the required progress of the reaction, and preferably, for 2 hours for conversion into the compound wherein D is -CH₂-OR' or its salt.

### 1-1-2) Production process using R'-OH

The compound wherein D' is -CH₂OH or its salt is reacted with a compound represented by the formula: R'-OH activated by using a phosphorus compound such as triphenylphosphine or tributylphosphine and an azodicarboxylate as typically represented by diethyl azodicarboxylate (DEAD) in a solvent which is not involved in the reaction for conversion into the compound wherein D is -CH₂-OR' or its salt.

### 1-2) Production of a compound wherein D is -CH₂-O-CO-R" (wherein R" is an optionally substituted C₁₋₆ alkyl group) or its salt

The compound wherein D' is -CH₂OH or its salt may be reacted with R"-CO-W in a solvent which is not involved in the reaction in the presence or absence of a base or in the presence or absence of an acid for conversion into the compound wherein D is -CH₂-O-CO-R" or its salt.

### 1-3) Production of a compound wherein D is -CH₂-NR'R" (wherein -NR' and R" are each an amino group represented, for example, by -NR⁶R⁷ (wherein R⁶ and R⁷ are independently hydrogen atom, a C₁₋₆ alkyl, a C₄₋₇ cycloalkyl, or a C₂₋₆ alkenyl; or R⁶, R⁷ and the nitrogen to which they are binding together represent a five- to seven-membered heterocyclic ring wherein the heterocyclic ring contains 1 to 2 heteroatoms selected from N, S, and O ; the R⁶ and R⁷ being optionally further substituted with an adequate substituent)) or its salt.

The compound wherein D' is -CH₂OH or its salt may be reacted with thionyl chloride, methanesulfonyl chloride, p-toluenesulfonyl chloride, or the like in a solvent which is not involved in the reaction in the presence or absence of a base or in the presence or absence of an acid for conversion into the compound wherein D' is -CH₂-W or its salt. The compound wherein D' is -CH₂-W or its salt may be further reacted with an amine represented by HNR'R" (for example, HNR⁶R⁷ wherein NR⁶R⁷ is as defined above) in a solvent which is not involved in the reaction in the presence or absence of copper powder, copper oxide powder, or iron powder in the presence or absence of a base or in the presence or absence of an acid for conversion into the compound wherein D is -CH₂-NR'R" or its salt. If necessary, a metal such as copper, palladium, chromium, or bismuth may be employed for formation of a complex with the compound wherein D' is represented by -CH₂-W in order to use the compound with a higher activity in the reaction.
Alternatively, the compound wherein D' is -CH₂OH or its salt may be reacted with a phosphorus compound such as triphenylphosphine or tributylphosphine and an azodicarboxylate as typically represented by diethyl azodicarboxylate (DEAD) in a solvent which is not involved in the reaction to activate the hydroxyl group, and the resulting product may be reacted with the compound represented by the formula: NHR'R" for conversion into the compound wherein D is -CH₂-NR'R" or its salt.
When R" is hydrogen in the resulting compound wherein D is -CH₂-NR'R" or its salt, the compound may be reacted with R"'-CO-W (wherein W is as defined above; and R"' is an optionally substituted C₁₋₆ alkyl group) in a solvent which is not involved in the reaction in the presence or absence of a base or in the presence or absence of an acid for conversion into the compound wherein D is -CH₂-NR'-CO-R"' or its salt. When the reaction is conducted by using R"'-S(O)_{z}-W (wherein W, R"', and z are as defined above) instead of the R"'-CO-W, the compound can be converted into the compound wherein D is -CH₂-NR'-S(O)_{z}- R"' or its salt.
When R" is hydrogen in the resulting compound wherein D is -CH₂-NR'R" or its salt, the compound may be also alkylated with R"'-W (wherein R"' is an optionally substituted C₁₋₆ alkyl group) in a solvent which is not involved in the reaction in the presence or absence of a base or in the presence or absence of an acid for conversion into the compound wherein D is -CH₂-NR'R"' or its salt.
When R" is hydrogen in the resulting compound wherein D is -CH₂-NR'R" or its salt, the compound may be also reacted with a ketone or an aldehyde represented by the formula: R^{d1}-CO-R^{d2} (wherein R^{d1} and R^{d2} are independently hydrogen atom, an optionally substituted C₁₋₆ alkyl group, a C₃₋₆cycloalkyl group, or a five- or six-membered heterocyclic group containing at least one heteroatom selected from N, O, and S, or R^{d1}, R^{d2} and carbon atom of the ketone together form a five- or six-membered cyclic group which may contain at least one heteroatom selected from N, O, and S) in a solvent which is not involved in the reaction in the presence of a reducing agent such as sodium borohydride, lithium aluminum hydride, or diisobutylaluminum hydride for reductive amination of the compound to thereby convert the compound into the compound wherein D is -CH₂-NR'-CHR^{d1}R^{d2} or its salt.

### 1-4) Production of a compound wherein D is -CHO or its salt

The compound wherein D' is -CH₂OH or its salt may be reacted in a solvent which is not involved in the reaction for oxidation by manganese dioxide; chromic acid oxidation by chromium (VI) oxide or dichromate; oxidation by lead tetraacetate; oxidation by oxygen; oxidation by activated DMSO; oxidation by halogen compound such as hypohalous acid or its salt to thereby convert the compound into the compound wherein D is -CHO or its salt.

### 1-5) Production of a compound wherein D is -CO₂H or its salt

The compound wherein D' is -CH₂OH or its salt may be reacted in a solvent which is not involved in the reaction for oxidation by manganese dioxide; chromic acid oxidation by chromium (VI) oxide or dichromate; oxidation by lead tetraacetate; oxidation by oxygen; oxidation by activated DMSO; oxidation by halogen compound such as hypohalous acid or its salt to thereby convert the compound into the compound wherein D is -CO₂H or its salt.
The compound wherein D is -CO₂H or its salt can be also produced by reacting the compound wherein D is -CHO or its salt synthesized in 1-4) for oxidation by manganese dioxide; chromic acid oxidation by chromium (VI) oxide or dichromate; oxidation by lead tetraacetate; oxidation by oxygen; oxidation by activated DMSO; oxidation by halogen compound such as hypohalous acid or its salt.

### 2) Substituent D convertible from -CHO

### 2-1) Production of a compound wherein D is -CH(OH)-R^{d3} (wherein R^{d3} is an adequate group selected from the R¹⁵ defined for D) or its salt

The compound wherein D is -CHO or its salt synthesized in 1-4) may be reacted with a nucleophilic reagent such as methyllithium or phenyllithium in a solvent which is not involved in the reaction for conversion into the compound wherein D is -CH(OH)R^{d3} or its salt.
The resulting compound wherein D is -CH(OH)R^{d3} or its salt may be converted into the compound wherein D is -CH(OR')R^{d3} or its salt by the procedure as in 1-1); into the compound wherein D is -CH(O-CO-R')R^{d3} or its salt by the procedure as in 1-2); and into the compound wherein D is -CH(NR'R'')R^{d3} (wherein NR'R" is as defined above) or its salt by the procedure as in 1-3).
The compound wherein D is -CH(OH)R^{d3} or its salt may be also converted into the compound wherein D is -CO-R^{d4} (wherein R^{d4} is an alkyl group adequately selected from R₁₅) by the procedure as in 1-4). The resulting compound wherein D is -CO-R^{d4} or its salt may be reacted with an alkylidenephosphorane represented by the formula: Ph3P=CR^{d5}R^{d6} in a solvent which is not involved in the reaction for conversion into the compound wherein D is -CR^{d4}=CR^{d5}R^{d6} or its salt. The compound wherein D is -CR^{d4}=CR^{d5}R^{d6} or its salt may be hydrogenated by using a catalyst such as activated carbon-palladium to convert the compound into the compound wherein D is -CHR^{d4}-CHR^{d5}R^{d6} (wherein R^{d5} and R^{d6} are each, for example, a C₁₋₆ alkyl group) or its salt.

2-2) Production of a compound wherein D is -CH=CR^{d5}R^{d6} or its salt
The compound wherein D is -CHO or its salt synthesized in 1-4) may be reacted with an alkylidenephosphorane represented by the formula: Ph3P=CR^{d5}R^{d6} in a solvent which is not involved in the reaction for conversion into the compound wherein D is -CH=CR^{d5}R^{d6} or its salt.
The resulting compound wherein D is -CH=CR^{d5}R^{d6} or its salt may be hydrogenated by using a catalyst such as activated carbon-palladium in a solvent which is not involved in the reaction to convert the compound into the compound wherein D is -CH₂-CHR^{d5}R^{d6} or its salt.

### 2-3) Production of a compound wherein D is -CH-NR'R" or its salt

The compound wherein D is -CHO or its salt synthesized in 1-4) may be reacted with the amine represented by the formula: HNR'R" as described above in a solvent which is not involved in the reaction in the presence of a reducing agent such as sodium borohydride, lithium aluminum hydride, or diisobutylaluminum hydride for reductive amination to thereby convert the compound into the compound wherein D is -CH-NR'R" or its salt.

### 3) Substituent D convertible from -CO₂

### 3-1) Production of a compound wherein D is -CO₂R' or its salt

The compound wherein D is -CO₂H or its salt synthesized in 1-5) may be reacted with R'-OH (wherein R' is an optionally substituted C₁₋₆ alkyl group) in a solvent which is not involved in the reaction in the presence or absence of a condensing agent such as carbodiimidazole for conversion into the compound wherein D is -CO₂R' or its salt. The compound wherein D is -CO₂H or its salt may also be reacted with thionyl chloride or the like for conversion into a compound wherein D is -COCl, and the compound may be then reacted with R'-OH for conversion into the compound wherein D is -CO₂R' or its salt.

### 3-2) Production of a compound wherein D is -CO-NR'R" (wherein NR'R" is as defined above) or its salt

The compound wherein D is -CO₂H or its salt synthesized in 1-5) may be reacted with NHR'R"(as defined above) in a solvent which is not involved in the reaction in the presence or absence of a condensing agent such as carbodiimidazole for conversion into the compound wherein D is -CO-NR'R" or its salt. The resulting compound wherein D is -CO-NR'R" or its salt may be reacted with a reducing agent such as lithium aluminum hydride or diisobutylaluminum hydride to convert the compound into the compound wherein D is -CHO or its salt. The resulting compound wherein D is -CO-NR'R" or its salt may be also reacted with a reducing agent such as lithium aluminum hydride or diisobutylaluminum hydride to convert the compound into the compound wherein D is -CH₂-NR'R" or its salt.

### 3-3) Production of a compound wherein D is -CO-R or its salt

The compound wherein D is -CO₂H or its salt synthesized in 1-5) may be reacted with a nucleophilic reagent such as methyllithium or phenyllithium in a solvent which is not involved in the reaction for conversion into the compound wherein D is -CO-R or its salt. The reaction with the nucleophilic reagent may be accomplished by using the compound wherein D is -CO₂R' or its salt obtained in 3-1) or the compound wherein D is -CO-NR'R" or its salt obtained in 3-2).
Furthermore, geometric isomer, tautomer, optical isomer, and other stereoisomers may be present for the compound of the present invention. These stereoisomers and mixtures thereof are within the scope of the present invention. Isolation or purification of such stereoisomer may be accomplished by any of the separation/purification techniques commonly used in the art, for example, recrystalization and various chromatographic processes. It is also possible to separately produce such optiocal isomer, for example, by asymmetric synthesis.

Next, the present invention is further described by referring to Experimental Examples and Examples which by no means limit the scope of the present invention.

### [Experimental Examples]

Excellent FXa inhibitory activity of the compounds of the present invention is confirmed by the test as described below.

### 1) Measurement of the enzyme inhibitory action

### a) Measurement of the human FXa inhibitory action

In vitro FXa inhibitory activity may be measured according to the method of Kettner et al. (Journal of Biological Chemistry, vol. 265, pages 18289 to 18297, 1990). To be more specific, human FXa (product of Enzyme Research Laboratories, Inc., 0.019 U/ml) is mixed with the specimens (of the compound of the present invention) prepared by diluting the compound of the invention with dimethylsulfoxide (DMSO) to different concentrations and synthetic substrate S-2222 (Chromogenix AB, 0.4 mM), and the mixtures are incubated at 37°C in Tris-hydrochloric acid buffer (pH 7.5). The FXa inhibitory activity of the specimen is calculated by continuously observing the absorbance at 405 nm, and comparing the initial speed with the initial speed in the absence of the specimen.
It should be noted that the FXa inhibitory activity of the specimen is generally indicated as IC₅₀.
When the compound of the present invention is evaluated for its FXa inhibitory activity by the procedure as describe above, the strength is in the range of 0.1 nM to 0.5 µM in terms of IC₅₀. Table 1 shows typical measurements.

[Table 1]

**Table 1**

| Example No. of the compound | IC₅₀ (µM) |
|---|---|
| Example 1 | 0.0057 |
| Example 2 | 0.0018 |
| Example 3 | 0.0013 |
| Example 13 | 0.0026 |
| Example 17 | 0.0065 |
| Example 21 | 0.0056 |
| Example 26 | 0.0057 |
| Example 34 | 0.0082 |
| Example 60 | 0.0045 |
| Example 66 | 0.0016 |

### 2) Measurement of Anticoagulant Activity (in Vitro) Measurement of Intrinsic Coagulation Time

Activated partial thromboplastin time (APTT) is measured in the presence of the test compounds of the present invention diluted at various concentrations. To be more specific, each of the test compounds diluted with DMSO at various concentrations is mixed with human plasma and APTT reagent. The mixture is incubated at 37°C for 2 minutes; calcium chloride (25 mM) is added to the mixture; and the coagulation time is thereafter measured. It should be noted that the anticoagulant activity of the test compound is described in terms of the concentration required to double the coagulation time for the case where no test compound is added. In this test, the compounds of the present invention were found to be effective in extending the APTT. The effects of the compounds of the present invention are shown in Table 2.

[Table 2]

**Table 2**

| Example No. of the compound | Concentration when APTT was doubled (µM) |
|---|---|
| Example 2 | 0.59 |
| Example 4 | 0.5 |
| Example 14 | 0.64 |
| Example 17 | 0.87 |
| Example 21 | 0.36 |
| Example 23 | 0.19 |
| Example 26 | 0.44 |
| Example 36 | 0.83 |
| Example 50 | 0.36 |
| Example 52 | 0.57 |
| Example 54 | 0.4 |
| Example 56 | 0.5 |
| Example 64 | 0.89 |

### 3) Characteristics of Anticoagulant Activity (ex vivo)

### a) Ex Vivo Measurement of Coagulation Time in Rats (i.v.)

Male Wistar rats (200 g to 300 g; Japan SLC Inc.) that have been fasting for more than 12 hours are administered through a femoral vein with a single dose (3 to 30 mg/kg) of a drug (compound of the present invention) dissolved in physiological saline (or 10% DMSO solution), and blood is collected at certain time intervals (3.8% sodium citrate (1/10 volume)), and plasma is then separated by centrifugation at 3000 rpm for 10 minutes. Prothrombin time (PT) is measured by the procedure as described below by using the separated plasma.
50 µl of the plasma is incubated at 37°C for 3 minutes and 100 µl of thromboplastin solution is added to start coagulation. The coagulation time is measured. In the actual test, the intravenously administered compounds of the present invention are found to be effective in extending the PT on account of enzyme inhibition.

### b) Ex Vivo Measurement of Coagulation Time in Rats (p.o.)

The test compound is compulsorily administered by oral administration using a feeding needle instead of the administration from the femoral vein at a single dose in the test a), and a certain volume of blood is collected at certain time intervals so as to contain 3.8% sodium citrate (1/10 volume). The blood is evaluated by the procedure as described in a) for extrinsic coagulation time and intrinsic coagulation time.
In this test b), the compounds of the present invention are found to be effective in extending the coagulation time upon oral administration of 10 to 100 mg/kg.
It should be noted that no abnormality in the aspect of safety is observed in the ex vivo test of the rats.

### 4) Evaluation of hERG inhibition by Rb efflux

HEK cells expressing hERG (human ether-a-go-go) is inoculated in a 96 well plate, and the cells are incubated for about 24 hours. After removing the culture medium by using a washing buffer, K⁺ channel open buffer containing the test substance (the compound of the present invention) is added. After incubating at 37°C for 3 hours, the K+ channel open buffer is replaced with Rb+ Load buffer containing the test substance, and the incubation at 37°C is continued for another 3 hours for incorporation of the Rb+ in the cell. After washing with the washing buffer containing the test substance, K+ channel open buffer containing the test substance is added, and the incubation at 37°C is continued for 5 minutes for release of the Rb+ in the cell to the exterior of the cell. After transferring the supernatant to a different plate, the cells are lysed, and the cell lysate is transfered to a different plate. The supernatant and the cell lysate are evaluated for ther Rb+ content in order to calculate hERG inhibition rate. Inhibition rate (%) of the test substance (25 mg/mL) is shown in Table 3. In this test, the compound of the present invention showed an inhibition rate of not more than 50% at 50 µM.

[Table 3]

**Table 3**

| Example No. of the compound | hREG inhibition rate (%) |
|---|---|
| Example 1 | 0 |
| Example 14 | 5 |
| Example 17 | 0 |
| Example 21 | 7 |
| Example 26 | 15 |
| Example 34 | 7 |
| Example 36 | 4 |
| Example 50 | 5 |
| Example 52 | 18 |
| Example 54 | 11 |
| Example 56 | 12 |
| Example 60 | 16 |
| Example 66 | 7 |

As described above, the compound of the present invention has strong inhibition of the FXa activity, while it has no inhibitory activity to trypsin, chymotrypsin, and thrombin, and accordingly, the compound of the present invention is highly specific. In addition, the compound of the present invention exhibits antithrombotic action when it is orally administered to rats at 0.1 to 10 mg/kg, or intravenously administered at 0.01 to 1 mg/kg.
The compound of the present invention has an FXa inhibitory activity (IC₅₀) of 0. 1nM to 0.5µM, and it is highly adapted for oral administration with adequate sustainability of the action. In the meanwhile, the compound of the present invention does not exhibit extension of bleeding time when it is orally administered to rats at a dose of 10 mg/kg or intravenously administered at a dose of 1 mg/kg. Accordingly, the compound of the present invention exhibits anticoagulation activity with no risk of bleeding tendency, and this is the difference from the known anticoagulants such as heparin and warfarin. In addition, since the IC₅₀ of hERG inhibition is higher than 50µM, a discrepancy of at least 100 folds is expected for the effective volume required for QT prolongation, and therefore, the compound of the present invention is highly safe.

The compounds of the present invention may be administered for the disease as described above which is to be prevented or treated by the present invention either alone or by combined application with other pharmacologically active component. Exemplary such pharmacologically active components include known fibrinolytic agents (for example, tissue plasminogen activators (tPA) and their derivatives (including modified agents or so-called "second generation" agents), urokinase, and streptokinase); known anticoagulants (for example, warfarin, heparin, and thrombomodulin); known inhibitors of platelet aggregation (for example, aspirin, thromboxane antagonist, inhibitor of thromboxane synthesis, and GPIIb/IIIa inhibitor); known therapeutic agents for hyperlipidemia (for example, clofibrate and related drugs, HMG-CoA inhibitor, and EPA-E); and known hypotensive agents (for example, nifedipine and diltiazem).
The term "combined application" as used herein covers not only the administration of a combination drug containing both the compound of the present invention and another pharmacologically active component but also the case where the two are in separate dosage forms and administered either at a time or at different times. The mode of administration is in no way limited as long as the compound of the present invention and another pharmacologically active component exist simultaneously in the patient's blood.

The pharmaceutical composition containing one or more of the compounds of the present invention and their pharmaceutically acceptable salts as its effective component may be prepared with a commonly used pharmaceutical vehicle, excipient, or other additives in the form of capsules, pills, tablets, granules, fine granules, or powder; oral solution such as suspension, emulsion, limonade, elixir, or syrup; injection; transnasal formulation; suppository; ointment; and epithem, and are orally or parenterally administered to human and other animals.
The clinical dose of the compound of the present invention to humans may be adequately determined in consideration of the symptom, body weight, age, sex, and the like of the patient to which the compound is to be administered. The adult daily dose in oral administration is generally in the range of 0.1 mg to 1000 mg, and preferably 1 mg to 300 mg, and the dose in parenteral administration is 0.01 to 300 mg, and preferably 0.1 mg to 100 mg. Such dose may be administered as a single dose or divided into several doses. The dose may vary depending on various conditions, and the dose below the above described range may be sufficient in some cases.

In order to accomplish oral administration according to the present invention, capsules, pills, tablets, powder, granules, and the like may be employed for the solid composition. Such solid composition is produced by combining at least one active substance with at least one inactive carrier. To be more specific, the composition may contain an excipient (for example, lactose, saccharose, mannitol, glucose, hydroxypropylcellulose, microcrystalline cellulose, or metasilicic acid), a binder (for example, crystalline cellulose, saccharide, dextrin, hydroxypropylcellulose, hydroxypropylmethylcellulose, polyvinyl pyrrolidone, or Macrogol), a lubricant (for example, magnesium stearate, calcium stearate, or talc), a disintegrant (for example, corn starch, carboxymethylcellulose, or carboxymethylcellulose calcium), a stabilizer (for example, lactose and other sugar alcohols or sugar), a solubilizer or a solubilizing aid (for example, cholesterol, triethanolamine, glutamic acid, or aspartic acid), a colorant, a flavoring agent, an antiseptic, an isotonic agent, a dispersant, an antioxidant (for example, ascorbic acid, or butylhydroxyanisole), a buffer, or a preservative (for example, paraben or benzyl alcohol).
It should be noted that the tablet, the pill and the granules may be coated with sucrose, gelatin, hydroxypropyl methylcellulosse phthalate or other gastric or enteric film coating.
Exemplary injections used for parenteral administration include aseptic aqueous or nonaqueous solution, suspension, and emulsion. Exemplary carriers for the aqueous solution and suspension include distilled water for injection and physiological saline, and exemplary carriers for the nonaqueous solution and suspension include propylene glycol, polyethylene glycol, vegetable oils such as olive oil, and alcohols such as ethyl alcohol, and polysorbate 80 (TM).
Such composition may further comprise an isotonic agent, antiseptic, humectant, emulsifier, dispersant, stabilizer, solubilizer, solubilizing aid, or other additives as described above, and these additives may be sterilized, for example, by filtration with a membrane filter, inclusion of an antimicrobial agent, or UV irradiation.

The composition may be also produced in the form of sterilized solid composition which can be dissolved, emulsified, or suspended before its use as an injection. When the compound of the present invention has low solubility, the compound may be solubilized as desired. Such solubilization may be accomplished by any of known processes applicable for the production of drugs, for example, addition of a surfactant (a polyoxyethylene hydrogenated castor oil, a higher fatty acid ester of polyoxyethylene sorbitan, a sucrose fatty acid ester, and the like); and formation of a solid dispersion of the drug and a solubilizer, for example, a polymer (a water-soluble polymer such as polyethylene glycol (PEG), hydroxypropyl methylcellulose (HPMC), or polyvinyl pyrrolidone (PVP); or an enteric polymer such as hydroxypropyl methylcellulose phthalate (HPMCP), or methyl methacrylate-methacrylic acid copolymer (Eudragit L,S (TM) manufactured by Rohm and Haas Company)). If desired, an inclusion compound may be formed by using α-, β-, or γ-cyclodextrin, hydroxypropyl cyclodextrin, or the like. The procedure employed for the solubilization may also be modified as desired depending on the drug to be prepared by referring to Nagai, T., et al., "Monograph in Pharmacology No.1, Biochemical Availability", Soft Science Inc., 78-82(1988) or Utsumi, I., et al., "Current Pharmaceutical Technology and Its Application", Iyaku Journal, 157-159(1983). Among these, the preferred is formation of a solid dispersion comprising the drug and the solubilizer which exhibits an improved solubility (JP 56-49314 A, FR 2460667 A).

### [Formulation Examples]

Next, examples of the pharmaceutical composition of the present invention are described. The "Compound M" is the compound of the present invention represented by the formula (I) or its pharmaceutically acceptable salt, and to be more specific, a compound selected from the compounds described in Examples.

### (a) Tablet (1 mg)

| | |
|---|---|
| Compound M | 1.0 g |
| Lactose | 90.0 g |
| Sodium carboxymethyl cellulose | 7.0 g |
| Corn starch paste (5% W/V paste) | 1.0 g |
| Magnesium stearate | 1.0 g |

The ingredients as described above were weighed and compressed in the usual manner to prepare tablets each weighing 100 mg.

### (b) Tablet (10 mg)

| | |
|---|---|
| Compound M | 10 g |
| Lactose | 150 g |
| Crosscarmellose sodium | 6.0 g |
| Corn starch | 28.5 g |
| Polyvinyl pyrrolidone | 2.5 g |
| Magnesium stearate | 3 g |

The ingredients as described above were weighed and
compressed in the usual manner to prepare tablets each weighing 200 mg, and the tablets were coated with cellulose acetate phthalate to produce enteric-coated tablets.

### (c) Tablet (100 mg)

| | |
|---|---|
| Compound M | 100 g |
| Lactose | 180 g |
| Crosscarmellose sodium | 13 g |
| Corn starch (5% W/V paste) | 4 g |
| Magnesium stearate | 3 g |

The ingredients as described above were weighed and
compressed in the usual manner to obtain tablets each weighing 300 mg.

### (d) Capsule (50 mg)

| | |
|---|---|
| Compound M | 100 g |
| Lactose | 395.5 g |
| Magnesium stearate | 4.5 g |

The ingredients as described above were weighed and uniformly mixed. The uniform powder was filled into hard capsules (Pharmacopeia No.1) in an amount of 250 mg/capsule.

### (e) Injection (0.1 mg/ml)

| | |
|---|---|
| Compound M | 0.1% W/V |
| Sodium phosphate buffer | 2.3% W/V |
| Citric acid | 0.4% |
| Macrogol 400 | 3.5% |
| Distilled water for injection | adequate amount |
| | to make up 100%. |

The ingredients as described above were mixed, and the resulting solution was put in 1 ml portions into injection amples, which were sealed to prepare injections.

### (f) Injection (1.0 mg/ml)

| | |
|---|---|
| Compound M | 1.0% W/V |
| Sodium phosphate buffer | 3.6% W/V |
| 1M Aqueous solution of | |
| sodium hydroxide | 15% W/V |
| Distilled water for injection | adequate amount |
| | to make up 100%. |

The ingredients as described above were mixed, and the resulting solution was put in 1 ml portions into injection amples, which were sealed to prepare injections.

### EXAMPLES

Next, the present invention is described in further detail by referring to Examples which by no means limit the scope of the present invention.
Nuclear magnetic resonance (NMR) spectrum was measured by using JEOL JNM-EX270 FT-NMR (manufactured by JEOL Ltd.) or JEOL JNM-LA300 FT-NMR (the data taken with this model are preceded by an asterisk; manufactured by JEOL Ltd.).
High performance liquid chromatography (HPLC) was conducted by using Shimadzu LC-10A (manufactured by Shimadzu Corporation).
LC-MS was conducted by using Waters Fraction Lynx MS system (manufactured by Waters) with the column of SunFire column (4.6 mm x 5 cm, 5 µm) manufactured by Waters and mobile phase of acetonitrile and 0.5% aqueous acetic acid solution under the gradient condition of acetonitrile : 0.5% aqueous acetic acid solution = 1 : 9 (0 minutes) to 1 : 1 (6 minutes) to 9 : 1 (9 minutes) to 9 : 1 (10 minutes).

### Example 1

### Synthesis of (-)-1'-acetyl-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one

### <Step 1>

### Synthesis of phenylmethyl (-)-tetrahydro-8a-(methoxymethyl)-5-oxo-1'-(phenylmethyl)spiro[imidazo[1,2-a]pyrazine-2(3H),4'-piperidine]-7-carboxylate

Phenylmethyl (±)-tetrahydro-8a-(methoxymethyl)-5-oxo-1'-(phenylmethyl)spiro[imidazo[1,2-a]pyradine-2(3H),4'-piperidine]-7-carboxylate (600 g) of International publication No. 02/053568 (WO 2002/053568), Example 59, step 1 was separated by an optically active column (DAICEL CHIRALPAK AD; elution solvent, MeOH) to produce phenylmethyl (+)-tetrahydro-8a-(methoxymethyl)-5-oxo-1'-(phenylmethyl)spiro[imidazo[1,2-a]pyrazine-2(3H),4'-piperidine]-7-carboxylate (first peak, 296 g, [α]_{D}²⁵+38.8° (c1.00, CHCl₃), >98%ee) and phenylmethyl (-)-tetrahydro-8a-(methoxymethyl)-5-oxo-1'-(phenylmethyl)spiro[imidazo[1,2-a]pyrazine-2(3H),4'-piperidine]-7-carboxylate (second peak, 290 g, [α]_{D}²⁸-34.8°(cl.00, CHCl₃), >98%ee).

NMR data: DMSO-d₆, 100°C: 7.37-7.17(10H, m), 5.14 (1H,d,J = 13Hz), 5.08 (1H,d,J = 13Hz), 4.23-4.14 (1H,m), 4.22 (1H,d,J = 13Hz), 4.02-3.96 (1H, m), 3.77-3.68 (1H, m), 3.46 (2H,s), 3.32 (1H, d,J = 10Hz), 3.25 (1H,d,J= 10Hz), 3.22 (3H,s), 2.88-2.82 (1H,m), 2.80 (1H,d,J = 13Hz), 2.58-2.45 (2H,m), 2.29-2.17 (2H,m), 1.75-1.59 (2H,m), 1.99-1.38 (2H,m)

### <Step 2>

### Synthesis of phenylmethyl (-)-tetrahydro-8a-(methoxymethy)-1-methyl-5-oxo-1'-(phenylmethyl)spiro[imidazo[1,2-a]pyrazine-2(3H),4'-piperidine]-7-carboxylate

The compound (1.0 g) produced in <Step 1> was dissolved in 1,2-dichloroethane (10 ml), and paraformaldehyde (0.19 g) and triacetoxy sodium borohydride (1.33 g) were added to this solution. After refluxing with heating for one hour and allowing the mixture to cool to room temperature, 1M aqueous solution of sodium hydroxide and methylene chloride were added for extraction with methylene chloride. The thus obtained methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride : methanol = 97 : 3) to produce the title compound (0.95 g) as a white solid.

NMR data: DMSO-d₆, 100°C: 7.39-7.18(10H,m), 5.13 (2H,s), 4.37 (1H,d,J = 13Hz), 4.20 (1H,d,J = 18Hz), 4.11 (1H,d,J = 11Hz), 3.76 (1H,d,J = 18Hz), 3.47 (2H,s), 3.41 (1H,d,J = 10Hz), 3.30 (1H,d,J = 10Hz), 3.19 (3H,s), 3.05-2.75 (4H,m), 2.37 (3H,s), 2.07-1.84 (3H,m), 1.78-1.65 (1H, m), 1.49-1.40 (1H, m), 1.12-1.03 (1H,m)

### <Step 3>

### Synthesis of (-)-tetrahydro-8a-(methoxymethyl)-1-methyl-1'-(phenylmethyl)spiro[imidazo[1,2-a]pyrazine-2(3H),4'-piperidin]-5(1H)-one

The compound (1.2 g) produced in <Step 2> was dissolved in trifluoroacetic acid (5.7 ml), and after adding anisole (0.57 ml), trifluoromethanesulfonic acid (2.84 ml) was slowly added dropwise in an ice bath. The stirring was continued for 30 minutes in an ice bath, and water (20 ml) and ether (20 ml) were slowly added. The organic layer was extracted with water, and the aqueous layer was extracted with ether, and the aqueous layer was adjusted to pH 10 with 1M aqueous solution of sodium hydroxide, and extracted with methylene chloride. The resulting methylene chloride layer was washed with saturated aqueous solution of sodium chloride, and dried with anhydrous sodium sulfate. The solvent was removed under reduced pressure to obtain the title compound (0.85 g) as a pale brown oil.

NMR data: CDCl₃: 7 . 37-7 . 21 (5H, m), 4.41 (1H,d,J = 12Hz), 3.60-3.30(7H,m), 3.33 (3H,s), 2.97-2.83 (3H,m), 2.65 (1H,d,J = 13Hz), 2.37 (3H,s), 2.20-1.75 (5H,m), 1.50-1.37 (1H,m), 1.19-1.06 (1H,m)

### <Step 4>

### Synthesis of (-)-7-[(1-benzenesulfonyl-5-chloroindol-2-yl)]sulfonyl-tetrahydro-8a-(methoxymethyl)-1-methyl-1'-(phenylmethyl)spiro[imidazo[1,2-a]pyrazine-2(3H),4'-piperidin]-5(1H)-one

The compound (0.85 g) produced in <Step 3> was dissolved in methylene chloride (15 ml), and triethylamine (0.36 ml) and 1-benzenesulfonyl-5-chloroindole-2-sulfonyl chloride (0.97 g) were slowly added in an ice bath. After allowing the mixture to warm to room temperature, the mixture was stirred for 30 minutes. Methylene chloride and saturated aqueous solution of sodium hydrogencarbonate were added for extraction with methylene chloride. The resulting methylene chloride layer was washed with saturated aqueous solution of sodium chloride, and dried with anhydrous sodium sulfate. The solvent was removed under reduced pressure, and the resulting residue was purified by silica gel column chromatography [Chromatorex NHTM] (elution solvent; methylene chloride : hexane = 4 : 1 to methylene chloride) to produce the title compound (1.09 g) as a white solid.

NMR data: CDCl₃* : 8.22 (1H,d,J = 9Hz), 8.08-7.99 (2H,m), 7.63-7.21(11H,m), 4.44-4.28 (2H,m), 4.18 (1H,d,J = 11Hz), 3.76-3.63 (2H,m), 3.55-3.40 (3H,m), 3.17 (3H,s), 3.07 (1H,d,J = 11Hz), 2.95-2.81 (3H,m), 2.41 (3H,s), 2.10-1.75 (4H,m), 1.64-1.55 (1H,m), 1.17-1.07 (1H,m)

### <Step 5>

### Synthesis of (-)-7-[(1-benzenesulfonyl-5-chloroindol-2-yl)]sulfonyl-tetrahydro-8a-(methoxymethyl)-1-methyl-spiro[imidazo[1,2-a]pyrazine-2(3H),4'-piperidin]-5(1H)-one

The compound (1.09 g) produced in <Step 4> was dissolved in methylene chloride (10 ml), and 1,8-bis(N,N-dimethylamino)naphthalene (0.49 g), and then, 1-chloroethyl chloroformate (0.42 ml) were added in an ice bath. The mixture was stirred at room temperature for 0.5 hours, and the reaction mixture was concentrated under reduced pressure. Methanol (15 ml) was added to this residue, and the mixture was refluxed under heating for 2 hours. After allowing the mixture to cool, the reaction mixture was concentrated under reduced pressure, and diethylether was added for solidification. After pulvirization, the supernatant was removed by decantation. The procedure as described above was repeated three times, and 1M aqueous solution of sodium hydroxide was added to the residue to adjust the pH to 10. The aqueous layer was extracted with methylene chloride, and the methylene chloride layer was washed with saturated aqueous solution of sodium chloride, and dried with anhydrous sodium sulfate. The solvent was removed under reduced pressure, and the resulting residue was purified by silica gel column chromatography [Chromatorex NHTM] (elution solvent; n-hexane : methylene chloride = 2 : 3 to methylene chloride to methylene chloride : methanol = 100 : 1) to produce the title compound (0.84 g) as a pale brown solid.

NMR data: CDCl₃: 8.22 (1H,d,J = 9Hz), 8.09-8.00 (2H,m), 7.63-7.41(6H,m), 4.45-4.30 (2H,m), 4.24 (1H,d,J = 12Hz), 3.78-3.65 (2H,m), 3.48 (1H,d,J = 10Hz), 3.19 (3H,s), 3.15-3.01 (3H,m), 2.91 (1H,d,J = 13Hz), 2.72-2.60 (1H,m), 2.58-2.46 (1H, m), 2.43 (3H,s), 1.92-1.79 (1H, m), 1.76-1.53 (3H,m), 1.21-1.10 (1H,m)

### <Step 6>

### Synthesis of (-)-1'-acetyl-7-[(1-benzenesulfonyl-5-chloroindol-2-yl)]sulfonyl-tetrahydro-8a-(methoxymethyl)-1-methyl-spiro[imidazo[1,2-a]pyrazine-2(3H),4'-piperidin]-5(1H)-one]

The compound (0.15 g) produced in <Step 5> was dissolved in pyridine (3 ml), and acetic anhydride (27 µl) was added to this solution at room temperature. After stirring for 2 hours and adding water, the solid precipitate was collected by filtration, and this solid was washed with water and dried under reduced pressure to produce the title compound as a pale yellow solid.

NMR data: CDCl₃: 8.22 (1H,d,J = 9Hz), 8.07-7.99 (2H,m), 7.63-7.54 (2H,m), 7.52-7.42 (4H,m), 4.77-4.62 (1H,m), 4.45-4.17 (4H,m), 3.93-3.65 (3H,m), 3.57-3.50 (1H,m), 3.25-3.17 (1H,m), 3.23 (3H,s), 3.16-3.04 (1H,m), 2.91 (1H,d,J = 12Hz), 2.38 (3H,s), 2.10 (3H,s), 1.86-1.52 (3H,m), 1.37-1.17 (1H,m)

### <Step 7>

### Synthesis of (-)-1'-acetyl-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(lH)-one

The compound produced in <Step 6> was dissolved in methanol (4 ml), and 0.24M solution of potassium hydroxide in methanol (1 ml) was added to the solution. After stirring the mixture at room temperature for 4 hours, water, and then, methylene chloride were added for extraction with methylene chloride. The methylene chloride layer was washed with saturated aqueous solution of sodium chloride, and dried with anhydrous sodium sulfate. The solvent was removed under reduced pressure and the resulting residue was purified by silica gel column chromatography [Chromatorex NHTM] (elution solvent; n-hexane : methylene chloride = 1: 4 to methylene chloride to methylene chloride : methanol = 100: 1), and then, by silica gel column chromatography (elution solvent; methylene chloride : methanol = 1 : 9) to produce the title compound (52 mg) as a white solid.

### Example 2

### Synthesis of (-)-7-[(5-chloro-1H-indol-2-yl)sulfonyl]tetrahydro-8a-(methoxymethyl)-1-methyl-1'-propanoyl-spiro[imidazo[1,2-a]pyrazine-2(3H),4'-piperidin]-5(1H)-one

### <Step 1>

### Synthesis of (-)-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one

The procedure of Example 1, <Step 7> was repeated by using the compound (0.5 g) of Example 1, <Step 5> to produce the title compound (95 mg).
LC-MS (ESI⁺) : 482 (M⁺H, Rt = 3.45 min)

### <Step 2>

### Synthesis of (-)-7-[(5-chloro-1H-indol-2-yl)sulfonyl]tetrahydro-8a-(methoxymethyl)-1-methyl-1'-propanoyl-spiro[imidazo[1,2-a]pyrazine-2(3H),4'-piperidin]-5(1H)-one

The compound (20 mg) produced in <Step 1> was dissolved in methylene chloride (1 ml), and triethylamine (6.9 (l) and propionyl chloride (9 (l) were added in an ice bath. After allowing the mixture to warm to room temperature, the mixture was stirred for one hour. Methylene chloride and water were added for extraction with methylene chloride. The methylene chloride layer was washed with saturated aqueous solution of sodium chloride and dried with anhydrous sodium sulfate. The solvent was removed under reduced pressure, and the resulting residue was pufified with silica gel column chromatography [Chromatorex NHTM] (elution solvent; methylene chloride to methylene chloride : methanol = 98 : 2) to produce the title compound (14 mg) as a white solid.

### Example 3

### Synthesis of (-)-7-[(5-chloro-1H-indol-2-yl)sulfonyl]-1'-cyclobutanecarbonyl-tetrahydro-8a-(methoxymethyl)-1-methyl-spiro[imidazo[1,2-a]pyrazine-2(3H),4'-piperidin]-5(1H)-one

The procedure of Example 2, <Step 2> was repeated by using the compound (150 mg) of Example 1, <Step 5>. After the reaction, the procedure of Example 1, <Step 7> was repeated to produce the title compound (89 mg).

### Example 4

### Synthesis of (-)-7-[(5-chloro-1H-indol-2-yl)sulfonyl]tetrahydro-8a-(methoxymethyl)-1-methyl-1'-propenoy1-spiro[imidazo[1,2-a]pyrazine-2(3H),4'-piperidin]-5(1H)-one

The procedure of Example 3 was repeated by using the compound (150 mg) of Example 1, <Step 5> to produce the title compound (68 mg).

### Example 5

### Synthesis of (-)-7-[(5-chloro-1H-indol-2-yl)sulfonyl]-1'-(2-furoylcarbonyl)-tetrahydro-8a-(methoxymethyl)-1-methyl-spiro[imidazo[1,2-a]pyrazine-2(3H),4'-piperidin]-5(1H)-one

The procedure of Example 3 was repeated by using the compound (150 mg) of Example 1, <Step 5> to produce the title compound (92 mg).

### Example 6

### Synthesis of (-)-7-[(5-chloro-1H-indol-2-yl)sulfonyl]-1'-(trifluoroacetyl)-tetrahydro-8a-(methoxymethyl)-1-methyl-spiro[imidazo[1,2-a]pyrazine-2(3H),4'-piperidin]-5(1H)-one

The procedure of Example 1, <Step 6> was repeated by using the compound (45 mg) of Example 2, <Step 1> to produce the title compound (25 mg).

### Example 7

### Synthesis of (-)-7-[(5-chloro-1H-indol-2-yl)sulfonyl]tetrahydro-1'-(methoxyacetyl)-8a-(methoxymethyl)-1-methyl-spiro[imidazo[1,2-a]pyrazine-2(3H),4'-piperidin]-5(1H)-one

The procedure of Example 3 was repeated by using the compound (150 mg) of Example 1, <Step 5> to produce the title compound (84 mg).

### Example 8

### Synthesis of (-)-7-[(5-chloro-1H-indol-2-yl)sulfonyl]tetrahydro-8a-(methoxymethyl)-1-methyl-1'-(dimethylaminoacetyl)-spiro[imidazo[1,2-a]pyrazine-2(3H),4'-piperidin]-5(1H)-one

The compound (0.15 g) of Example 1, <Step 5> was dissolved in methylene chloride (3 ml), and N,N-dimethylglycine (30 mg) and WSC.HCl (56 mg) were added to the solution. After stirring overnight at room temperature, the reaction mixture was concentrated, and the resulting residue was reacted by repeating the procedure of Example 1, <Step 7> to produce the title compound (85 mg) as a white solid.

### Example 9

### Synthesis of (-)-1'-(aminoacetyl)-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one

The procedure of Example 8 was repeated by using the compound (150 mg) of Example 1, <Step 5>. After the reaction, the mixture was dissolved in 4M solution of hydrogen chloride in ethyl acetate (3 ml), and the mixture was stirred at room temperature for one hour. Water was added and 1M aqueous solution of sodium hydroxide was added to adjust the pH to at least 10. After adding methylene chloride for extraction with methylene chloride, the methylene chloride layer was washed with saturated aqueous solution of sodium chloride, and the solution was dried with anhydrous sodium sulfate. The solvent was removed under reduced pressure, and the resulting residue was purified by silica gel column chromatography [Chromatorex NHTM] (elution solvent; methylene chloride → methylene chloride : methanol = 98: 2) to produce the title compound (69 mg).

### Example 10

### Synthesis of (-)-7-[(5-chloro-1H-indol-2-yl)sulfonyl]tetrahydro-1'-(hydroxyacetyl)-8a-(methoxymethyl)-1-methyl-spiro[imidazo[1,2-a]pyrazine-2(3H),4'-piperidin]-5(1H)-one

The procedure of Example 3 was repeated by using the compound (150 mg) of Example 1, <Step 5> to produce the title compound (54 mg).

### Example 11

### Synthesis of (-)-7-[(5-chloro-1H-indol-2-yl)sulfonyl]tetrahydro-8a-(methoxymethyl)-1'-(2-methoxypropenoyl)-1-methyl-spiro[imidazo[1,2-a]pyrazine-2(3H),4'-piperidin]-5(1H)-one

The procedure of Example 8 was repeated by using the compound (150 mg) of Example 1, <Step 5> to produce the title compound (17 mg).

### Example 12

### Synthesis of (-)-7-[(5-chloro-1H-indol-2-yl)sulfonyl]tetrahydro-8a-(methoxymethyl)-1'-[(1Z)-(2-methoxypropenoyl)]-1-methyl-spiro[imidazo[1,2-a]pyrazine-2(3H),4'-piperidin]-5(1H)-one

The compound produced in Example 11 was purified by silica gel column chromatography [Chromatorex NHTM] (elution solvent; methylene chloride : methanol = 95 : 5) to produce the title compound (12 mg) as a white solid.

### Example 13

### Synthesis of (-)-7-[(5-chloro-1H-indol-2-yl)sulfonyl]tetrahydro-8a-(methoxymethyl)-1-methyl-1'-propinoyl-spiro[imidazo[1,2-a]pyrazine-2(3H),4'-piperidin]-5(1H)-one

The compound (70 mg) of Example 2, <Step 1> was dissolved in methylene chloride (2 ml), and propiolic acid (11 µl) and WSC.HCl (33 mg) were added to the solution. After stirring overnight at room temperature, methylene chloride and water were added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography [Chromatorex NHTM] (elution solvent; methylene chloride → methylene chloride : methanol = 98 : 2) to produce the title compound (33 mg) as a white solid.

### Example 14

### Synthesis of (-)-1'-acetyl-7-[[(1E)-2-(5-chloro-2-thienyl)ethenyl]sulfonyl]tetrahydro-8a-(methoxymethyl)-1-methyl-spiro[imidazo[1,2-a]pyrazine-2(3H),4'-piperidin]-5(1H)-one

### <Step 1>

### Synthesis of (-)-tetrahydro-8a-(methoxymethyl)-1'-(phenylmethyl)spiro[imidazo[1,2-a]pyrazine-2(3H),4'-piperidin]-5(1H)-one

The procedure of Example 1, <Step 3> was repeated by using the compound (5 g) of Example 1, <Step 1> to produce the title compound (3.2 g).
LC-MS (ESI⁺) : 345 (M⁺H, Rt = 0.23 min)

### <Step 2>

### Synthesis of (-)-7-[[(1E)-2-(5-chloro-2-thienyl)ethenyl]sulfonyl]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (3.1 g) of <Step 1> to produce the title compound (5 g).

NMR data: DMSO-d₆: 7.46 (1H,d,J = 15Hz), 7.36-7.21 (5H,m), 7.09 (1H,d,J = 4Hz), 6.92 (1H,d,J = 4Hz), 6.29 (1H,d,J = 15Hz), 4.25-4.12 (2H,m), 4.02 (1H,d,J = 12Hz), 3.65 (1H,d,J = 9Hz), 3.53 (1H,d,J = 17Hz), 3.49 (2H,s), 3.43-3.35 (1H,m), 3.41 (3H,s), 2.91 (1H,d,J = 12Hz), 2.67-2.47 (2H, m), 2.51 (1H,d,J = 12Hz), 2.40 (1H,s), 2.38-2.18 (2H,m), 1.84-1.66 (2H,m), 1.53-1.40 (2H,m)

### <Step 3>

### Synthesis of (-)-7-[[(1E)-2-(5-chloro-2-thienyl)ethenyl]sulfonyl]tetrahydro-8a-(methoxymethyl)-1-methyl-1'-(phenylmethyl)-spiro[imidazo[1,2-a]pyrazine-2(3H),4'-piperidin]-5(1H)-one

The procedure of Example 1, <Step 2> was repeated by using the compound (0.35 g) of <Step 2> to produce the title compound (0.35 g).
LC-MS (ESI⁺): 565 (M⁺H, Rt = 4.28 min)

### <Step 4>

### Synthesis of (-)-7-[[(lE)-2-(5-chloro-2-thienyl)ethenyl]sulfonyl]tetrahydro-8a-(methoxymethyl)-1-methyl-spiro[imidazo[1,2-a]pyrazine-2(3H),4'-piperidin]-5(1H)-one

The procedure of Example 1, <Step 5> was repeated by using the compound (0.33 g) of <Step 3> to produce the title compound (0.13 g).
LC-MS (ESI⁺) : 475 (M⁺H, Rt = 3.41 min)

### <Step 5>

### Synthesis of (-)-1'-acetyl-7-[[(1E)-2-(5-chloro-2-thienyl)ethenyl]sulfonyl]tetrahydro-8a-(methoxymethyl)-1-methyl-spiro[imidazo[1,2-a]pyrazine-2(3H),4'-piperidin]-5(1H) -one

The procedure of Example 1, <Step 6> was repeated by using the compound (50 mg) of <Step 4> to produce the title compound (33 mg).

### Example 15

### Synthesis of (-)-1'-acetyl-7-[(6-chloro-benzo[b]thiophen-2-yl)sulfonyl]tetrahydro-8a-(methoxymethyl)-1-methyl-spiro[imidazo[1,2-a]pyrazine-2(3H),4'-piperidin]-5(1H)-one

### <Step 1>

### Synthesis of (-)-7-[(6-chloro-benzo[b]thiophen-2-yl)sulfonyl]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (2 g) of Example 14, <Step 1> to produce the title compound (3.5 g).

NMR data: CDCl₃*: 7.94-7.76 (3H,m), 7.46 (1H,dd, J = 2,8Hz), 7.39-7.18 (5H,m), 4.29 (1H,d,J = 17Hz), 4.20-4.09 (2H,m), 3.71-3.30 (3H,m), 3.68 (1H,d,J = 10Hz), 3.42 (3H,s), 3.21-3.08 (2H,m), 2.91 (1H,d,J = 12Hz), 2.72-2.51 (2H,m), 2.50-2.20 (2H,m), 2.33 (1H,d,J = 12Hz), 1.94-1.34 (4H,m)

### <Step 2>

### Synthesis of (-)-7-[(6-chloro-benzo[b]thiophen-2-yl)sulfonyl]tetrahydro-8a-(methoxymethyl)-1-methyl-1'-(phenylmethyl)-spiro[imidazo[1,2-a]pyrazine-2(3H),4'-piperidin]-5(1H)-one

The procedure of Example 1, <Step 2> was repeated by using the compound (2 g) of <Step 1> to produce the title compound (1.5 g).
LC-MS (ESI⁺) : 589 (M⁺H, Rt = 4.93 min)

### <Step 3>

### Synthesis of (-)-7-[(6-chloro-benzo[b]thiophen-2-yl)sulfonyl]tetrahydro-8a-(methoxymethyl)-1-methyl-spiro[imidazo[1,2-a]pyrazine-2(3H),4'-piperidin)-5(1H)-one

The procedure of Example 1, <Step 5> was repeated by using the compound (1.5 g) of <Step 2> to produce the title compound (1.1 g).
LC-MS (ESI⁺) : 499 (M⁺H, Rt = 3.98 min)

### <Step 4>

### Synthesis of (-)-1'-acetyl-7-[(6-chloro-benzo[b]thiophen-2-yl)sulfonyl]tetrahydro-8a-(methoxymethyl)-1-methyl-spiro[imidazo[1,2-a]pyrazine-2(3H),4'-piperidin]-5(1H)-one

The procedure of Example 1, <Step 6> was repeated by using the compound (50 mg) of <Step 3> to produce the title compound (33 mg).

### Example 16

### Synthesis of (-)-1'-acetyl-7-[(6-chloronaphthalen-2-yl)sulfonyl]tetrahydro-8a-(methoxymethyl)-1-methyl-spiro[imidazo[1,2-a]pyrazine-2(3H),9'-piperidin]-5(1H)-one

### <Step 1>

### Synthesis of (-)-7-[(6-chloronaphthalen-2-yl)sulfonyl]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (0.72 g) of Example 1, <Step 3> to produce the title compound (1.23 g).
LC-MS (ESI⁺): 583 (M⁺H, Rt = 4.73 min)

### <Step 2>

### Synthesis of (-)-7-[(6-chloronaphthalen-2-yl)sulfonyl]tetrahydro-8a-(methoxymethyl)-spiro[imidazo[1,2-a]pyrazine-2(3H),4'-piperidin]-5(1H)-one

The procedure of Example 1, <Step 5> was repeated by using the compound (1.23 g) of <Step 1> to produce the title compound (0.91 g).
LC-MS (ESI⁺): 493 (M⁺H, Rt = 4.00 min)

### <Step 3>

### Synthesis of (-)-1'-acetyl-7-[(6-chloronaphthalen-2-yl)sulfonyl]tetrahydro-8a-(methoxymethyl)-1-methyl-spiro[imidazo[1,2-a]pyrazine-2(3H),4'-piperidin]-5(1H)-one

The procedure of Example 1, <Step 6> was repeated by using the compound (49 mg) of <Step 2> to produce the title compound (47 mg).

### Example 17

### Synthesis of (-)-7-[(6-chloro-benzo[b]thiophen-2-yl)sulfonyl]tetrahydro-8a-(methoxymethyl)-1-methyl-1'-propinoyl-spiro[imidazo[1,2-a]pyrazine-2(3H),4'-piperidin]-5(1H)-one

The procedure of Example 1, <Step 6> was repeated by using the compound (50 mg) of Example 15, <Step 3> to produce the title compound (50 mg).

### Example 18

### Synthesis of (±)-1'-acetyl-7-[(5-chloro-1H-indol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-5(1H)-one

### <Step 1>

### Synthesis of tert-butyl (±)-tetrahydro-5-oxo-1'-(phenylmethyl)-8a-(phthalimid-1-ylmethyl)-spiro[imidazo[1,2-a]pyrazine-2(3H),4'-piperidine]-7-carboxylate

N-[3-(phthalimid-1-yl)-2-oxopropyl]-N-[(tert-butoxy) carbonyl]-glycine ethyl ester (0.140 g) and 4-amino-1-(phenylmethyl)-4-piperidinemethaneamine (0.21 g) synthesized by the standard procedure described in International publication No. 02/053568 (WO 2002/053568), Example 45, steps 1 and 2 were dissolved in toluene (15 ml), and acetic acid (0.04 ml) was added. The mixture was stirred at room temperature for 30 minutes, and the stirring was continued at 50 to 60°C for 3 hours, and at 70 to 80°C for another 3 hours. After adding saturated aqueous solution of sodium hydrogencarbonate, the solution was extracted with methylene chloride, and the methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride : methanol = 4 : 1 to 1 : 1) to produce the title compound (0.18 g) as a pale yellow solid.
LC-MS (ESI⁺): 560 (M⁺H, Rt = 3.72 min)

### <Step 2>

### Synthesis of tert-butyl (±)-tetrahydro-1-methyl-5-oxo-1'-(phenylmethyl)-8a-(phthalimid-1-ylmethyl)-spiro[imidazo[1,2-a]pyrazine-2(3H),4'-piperidine]-7-carboxylate

The procedure of Example 1, <Step 3> was repeated by using the compound (22 g) of <Step 1> to produce the title compound (16 g).
LC-MS (ESI⁺): 574 (M⁺H, Rt = 3.74 min)

### <Step 3>

### Synthesis of tert-butyl (±)-8a-(aminomethyl)-tetrahydro-1-methyl-5-oxo-1'-(phenylmethyl)-spiro[imidazo[1,2-a]pyrazine-2(3H),4'-piperidine]-7-carboxylate

The compound (9 g) produced in <Step 2> was dissolved in ethanol (90 ml), and hydrazine monohydrate (3.8 ml) was added to the solution. After refluxing with heating for one hour, the precipitate was removed by filtration, and the solution was concentrated under reduced pressure to produce the title compound (7.3 g).

NMR data: DMSO-d₆, 100°C: 7.34-7.18 (5H,m), 4.23 (1H,d,J = 11Hz), 4.16 (1H,d,J = 12Hz), 4.12 (1H,d,J = 18Hz), 4.00-3.85 (2H,m), 3.68 (1H,d,J = 18Hz), 3.53-3.39 (2H,m), 3.47 (2H,s), 3.04-2.72 (4H,m), 2.65 (1H,d,J = 13Hz), 2.60 (1H,d,J = 13Hz), 2.38 (3H,s), 2.08-1.87 (3H,m), 1.80-1.67 (1H, m), 1.43(9H,s)

### <Step 4>

### Synthesis of tert-butyl (±)-tetrahydro-l-methyl-8a-(N-methylaminomethyl)-5-oxo-1'-(phenylmethyl)-spiro[imidazo[1,2-a]pyrazine-2(3H),4'-piperidine]-7-carboxylate

The compound (3 g) produced in <Step 3> was dissolved in methanol (50 ml), and paraformaldehyde (0.33 g) and sodium methoxide (0.55 g) were added to the solution. After stirring the mixture overnight at room temperature, sodium borohydride (0.38 g) was added to the reaction mixture, and the mixture was stirred at room temperature for 4 hours. Water and methylene chloride were added for extraction with methylene chloride, and the methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride : 4M ammonia - methanol = 99 : 1 → 97 : 3) to produce the title compound (1.7 g) as a white solid.
LC-MS (ESI⁺) : 458 (M⁺H, Rt = 0.25 min)

### <Step 5>

### Synthesis of tert-butyl (±)-tetrahydro-8a-(N-methoxycarbonyl-N-methylaminomethyl)-1-methyl-5-oxo-1'-(phenylmethyl)-spiro[imidazo[1,2-a]pyrazine-2(3H),4'-piperidine]-7-carboxylate

The compound (0.35 g) produced in <Step 4> was dissolved in methylene chloride (5 ml), and in an ice bath, triethylamine (0.16 ml) and methoxycarbonyl chloride (88 µl) were added and the mixture was stirred for 1.5 hours. Saturated aqueous solution of sodium hydrogencarbonate and methylene chloride were added for extraction by methylene chloride, and the methylene chloride layer was washed with saturated aqueous solution of sodium chloride, and dried with anhydrous sodium sulfate. The solvent was removed under reduced pressure to produce the title compound (0.4 g) as a white solid.

NMR data: DMSO-d₆, 100°C: 7.33-7.17 (5H, m), 4.26 (1H,d,J = 11Hz), 4.10 (1H,d,J = 18Hz), 4.04(H,d,J = 14Hz), 3.70 (1H,d,J = 18Hz), 3.56-3.49 (1H,m), 3.52 (3H,s), 3.46 (2H,s), 3.24 (1H,d,J = 15Hz), 2.87 (3H,s), 2.84-2.73 (3H,m), 2.57 (1H,d,J = 11Hz), 2.35 (3H,s), 2.07-1.88 (3H,m), 1.79-1.62 (1H,m), 1.43(9H,s), 1.27-1.19 (1H,m), 1.08-0.97 (1H,m)

### <Step 6>

### Synthesis of (±)-tetrahydro-8a-(N-methoxycarbonyl-N-methylaminomethyl)-1-methyl-1'-(phenylmethyl)-spiro[imidazo[1,2-a]pyrazine-2(3H),4'-piperidin]-5(1H)-one

The compound (0.4 g) produced in <Step 5> was dissolved in 10% solution of hydrogen chloride in methanol (7.7 ml), and the solution was stirred at room temperature for 4 hours. The solvent was removed under reduced pressure, and saturated aqueous solution of sodium hydrogencarbonate and 1M aqueous solution of sodium hydroxide were added to the residue to adjust the pH to 10 or higher. Methylene chloride was added for extraction with methylene chloride, and the methylene chloride layer was washed with saturated aqueous solution of sodium chloride and dried with anhydrous sodium sulfate. The solvent was removed under reduced pressure to produce the title compound (0.23 g) as a white solid.
LC-MS (ESI⁺): 416 (M⁺H, Rt = 0.23 min)

### <Step 7>

### Synthesis of (±)-7-[(1-benzenesulfonyl-5-chloroindol-2-yl)sulfonyl]tetrahydro-8a-(N-methoxycarbonyl-N-methylaminomethyl)-1-methyl-1'-(phenylmethyl)-spiro[imidazo[1,2-a]pyrazine-2(3H),4'-piperidin]-5(1H)-one

The procedure of Example 1, <Step 4> was repeated by using the compound (0.22 g) of <Step 6> to produce the title compound (0.26 g).
LC-MS (ESI⁺) : 769 (M⁺H, Rt = 5.25 min)

### <Step 8>

### Synthesis of (±)-7-((1-benzenesulfonyl-5-chloroindol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-one

The procedure of Example 1, <Step 5> was repeated by using the compound (0.26) of <Step 7> to produce the title compound (0.17 g).
LC-MS (ESI⁺) : 680 (M⁺H, Rt = 4.44 min)

### <Step 9>

### Synthesis of (±)-1'-acetyl-7-[(1-benzenesulfonyl-5-chloroindol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-one

The procedure of Example 1, <Step 6> was repeated by using the compound (0.17 g) of <Step 8> to produce the title compound (0.15 g).
LC-MS (ESI⁺) : 721 (M⁺H, Rt = 7.49 min)

### <Step 10>

### Synthesis of (±)-1'-acetyl-7-[(5-chloro-1H-indol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-5(1H)-one

The procedure of Example 1, <Step 7> was repeated by using the compound (0.15 g) of <Step 9> to produce the title compound (0.1 g).

### Example 19

### Synthesis of (±)-1'-acetyl-7-[(5-chloro-1H-indol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-5(1H)-one

### <Step 1>

### Synthesis of tert-butyl (±)-tetrahydro-8a-(N-methanesulfonyl-N-methylaminomethyl)-1-methyl-1'-5-oxo-(phenylmethyl)-spiro[imidazo[1,2-a]pyrazine-2(3H),4'-piperidine]-7-carboxylate

The procedure of Example 1, <Step 4> was repeated by using the compound (0.25 g) of Example 18, <Step 4> to produce the title compound (0.29 g).
LC-MS (ESI⁺) : 536 (M⁺H, Rt = 2.63 min)

### <Step 2>

### Synthesis of (±)-tetrahydro-8a-(N-methanesulfonyl-N-methylaminomethyl)-1-methyl-1'-(phenylmethyl)-spiro[imidazo[1,2-a]pyrazine-2(3H),4'-piperidin]-5(1H)-one

The procedure of Example 18, <Step 6> was repeated by using the compound (0.29 g) of <Step 1> to produce the title compound (0.17 g).
LC-MS (ESI⁺): 436 (M⁺H, Rt = 0.23 min)

### <Step 3>

### Synthesis of (±)-7-[(1-benzenesulfonyl-5-chloroindol-2-yl)sulfonyl]tetrahydro-8a-(N-methanesulfonyl-N-methylaminomethyl)-1-methyl-1'-(phenylmethyl)-spiro[imidazo[1,2-a]pyrazine-2(3H),4'-piperidin]-5(1H)-one

The procedure of Example 1, <Step 4> was repeated by using the compound (0.25 g) of <Step 2> to produce the title compound (0.26 g).
LC-MS (ESI⁺): 789 (M⁺H, Rt = 5.07 min)

### <Step 4>

### Synthesis of (±)-7-[(1-benzenesulfonyl-5-chloroindol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-one

The procedure of Example 1, <Step 5> was repeated by using the compound (0.26 g) of <Step 3> to produce the title compound (0.17 g).
LC-MS (ESI⁺) : 699 (M⁺H, Rt = 4.39 min)

### <Step 5>

### Synthesis of (±)-1'-acetyl-7-[(1-benzenesulfonyl-5-chloroindol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-one

The procedure of Example 1, <Step 6> was repeated by using the compound (0.17 g) of <Step 4> to produce the title compound (0.15 g).
LC-MS (ESI⁺) : 741 (M⁺H, Rt = 7.36 min)

### <Step 6>

### Synthesis of (±)-1'-acetyl-7-[(5-chloro-1H-indol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-one

The procedure of Example 1, <Step 7> was repeated by using the compound (0.15 g) of <Step 5> to produce the title compound (90 mg).

### Example 20

### Synthesis of (±)-1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one

### <Step 1>

### Synthesis of tert-butyl (±)-8a-(acetylaminomethyl)-tetrahydro-1-methyl-5-oxo-1'-(phenylmethyl)-spiro[imidazo[l,2-a]pyrazine-2(3H),4'-piperidine]-7-carboxylate

The procedure of Example 2, <Step 2> was repeated by using the compound (0.50 g) of Example 18, <Step 3> to produce the title compound (0.52 g).
LC-MS (ESI⁺): 486 (M⁺H, Rt = 2.17 min)

### <Step 2>

### Synthesis of (±)-8a-(acetylaminomethyl)-tetrahydro-1-methyl-1'-(phenylmethyl)-spiro[imidazo[1,2-a]pyrazine-2(3H),4'-piperidin]-5(1H)-one

The procedure of Example 18, <Step 6> was repeated by using the compound (0.50 g) of <Step 2> to produce the title compound (0.22 g).
LC-MS (ESI⁺): 386 (M⁺H, Rt = 0.23 min)

### <Step 3>

### Synthesis of (±)-8a-(acetylaminomethyl)-7-[(1-benzenesulfonyl-5-chloroindol-2-yl)sulfonyl]tetrahydro-1-methyl-1'-(phenylmethyl)-spiro[imidazo[1,2-a]pyrazine-2(3H),4'-piperidin]-5(1H)-one

The procedure of Example 1, <Step 4> was repeated by using the compound (0.22 g) of <Step 2> to produce the title compound (0.39 g).
LC-MS (ESI⁺) : 739 (M⁺H, Rt = 4.79 min)

### <Step 4>

### Synthesis of (±)-8a-(acetylaminomethyl)-7-[(1-benzenesulfonyl-5-chloroindol-2-yl)sulfonyl]tetrahydro-1-methyl-spiro[imidazo[1,2-a]pyrazine-2(3H),4'-piperidin]-5(1H)-one

The procedure of Example 1, <Step 5> was repeated by using the compound (0.42 g) of <Step 3> to produce the title compound (0.32 g).
LC-MS (ESI⁺) : 649 (M⁺H, Rt = 4.16 min)

### <Step 5>

### Synthesis of (±)-1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one

The procedure of Example 1, <Step 6> was repeated by using the compound (0.2 g) of <Step 4>, and after the reaction, the procedure of Example 1, <Step 7> was repeated to produce the title compound (0.11 g).

### Example 21

### Synthesis of optically active 1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one

The compound (61 mg) of Example 20 was separated by an optically active column (DAICEL CHIRALPAK AD-H, elution solvent, hexane : ethanol : diethylamine = 60 : 40 : 0.1) to obtain optically active 1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one (first peak, 20 mg) and optically active 1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one (second peak, 23 mg).

### Example 22

### Synthesis of (-)-1'-acetyl-7-[(5-chloro-1H-indol-2-yl)sulfonyl]tetrahydro-8a-(hydroxymethyl)-1-methyl-spiro[imidazo[1,2-a]pyrazine-2(3H),9'-piperidin]-5(1H)-one

### <Step 1>

### Synthesis of (-)-1'-acetyl-7-[(1-benzenesulfonyl-5-chloroindol-2-yl)sulfonyl]tetrahydro-8a-(hydroxymethyl)-1-methyl-spiro[imidazo[1,2-a]pyrazine-2(3H),4'-piperidin]-5(1H)-one

The compound (1.4 g) of Example 1, <Step 6> was dissolved in methylene chloride (30 ml), and the solution was cooled to -78°C. A solution of boron tribromide (2 ml) in methylene chloride (5 ml) was added dropwise, and after allowing the mixture to warm to room temperature, the mixture was stirred overnight at room temperature.
Ice was guradually added to the reaction mixture, and 1M aqueous solution of sodium hydroxide was added to adjust the pH to the range of about 11 to 12. Methylene chloride was added for extraction with methylene chloride, and the methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride : 4M ammonia - methanol = 100 : 1) to produce the title compound (98 mg) as a white solid.
LC-MS (ESI⁺): 650 (M⁺H, Rt = 6.90 min)

### <Step 2>

### Synthesis of (-)-1'-acetyl-7-[(5-chloro-1H-indol-2-yl)sulfonyl]tetrahydro-8a-(hydroxymethyl)-1-methyl-spiro[imidazo[1,2-a]pyrazine-2(3H),4'-piperidin]-5(1H)-one

The procedure of Example 1, <Step 7> was repeated by using the compound (50 mg) of <Step 1> to produce the title compound (32 mg).

### Example 23

### Synthesis of (-)-1'-acetyl-7-[(5-chloro-1H-indol-2-yl)sulfonyl]tetrahydro-1-ethyl-8a-(methoxymethyl)-spiro[imidazo[1,2-a]pyrazine-2(3H),4'-piperidin]-5(1H)-one

### <Step 1>

### Synthesis of phenylmethyl (-)-1-ethyl-tetrahydro-8a-(methoxymethyl)-5-oxo-1'-(phenylmethyl)-spiro[imidazo[1,2-a]pyrazine-2(3H),4'-piperidine]-7-carboxylate

The procedure of Example 1, <Step 2> was repeated by using the compound (0.5 g) of Example 1, <Step 1> to produce the title compound (0.1 g).
LC-MS (ESI⁺) : 507 (M⁺H, Rt = 3.96 min)

### <Step 2>

### Synthesis of (-)-1-ethyl-tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 3> was repeated by using the compound (0.1 g) of <Step 1> to produce the title compound (70 mg).
LC-MS (ESI⁺): 373 (M⁺H, Rt = 0.23 min)

### <Step 3>

### Synthesis of (-)-1-ethyl-7-[(1-benzenesulfonyl-5-chloroindol-2-yl)]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (70 mg) of <Step 2> to produce the title compound (0.14 g).
LC-MS (ESI⁺) : 726 (M⁺H, Rt = 6.16 min)

### <Step 4>

### Synthesis of (-)-7-[(1-benzenesulfonyl-5-chloroindol-2-yl)]-1-ethyl-tetrahydro-8a-(methoxymethyl)-spiro[imidazo[1,2-a]pyrazine-2(3H),4'-piperidin]-5(1H)-one

The procedure of Example 1, <Step 5> was repeated by using the compound (0.14 g) of <Step 3> to produce the title compound (75 mg).
LC-MS (ESI⁺): 636 (M⁺H, Rt = 4.99 min)

### <Step 5>

### Synthesis of (-)-1'-acetyl-7-[(5-chloro-1H-indol-2-yl)sulfonyl]tetrahydro-1-ethyl-8a-(methoxymethyl)-spiro[imidazo[1,2-a]pyrazine-2(3H),9'-piperidin)-5(1H)-one

The procedure of Example 20, <Step 5> was repeated by using the compound (35 mg) of <Step 4> to produce the title compound (20 mg).

### Example 24

### Synthesis of (±)-1'-acetyl-7-[(5-chloro-1H-indol-2-yl)sulfonyl]tetrahydro-1-(hydroxyethyl)-8a-(methoxymethyl)-spiro[imidazo[1,2-a]pyrazine-2(3H),4'-piperidin]-5(1H)-one <Step 1> Synthesis of phenylmethyl (±)-tetrahydro-1-(hydroxyethyl)-8a-(methoxymethyl)-5-oxo-1'-(phenylmethyl)-spiro[imidazo[1,2-a]pyrazine-2(3H),4'-piperidine]-7-carboxylate

N-[3-methoxy-2-oxopropyl]-N-[(phenylmethoxy)-carbonyl]-glycine ethyl ester (1 g) and 4-(2-hydroxyethylamino)-1-(phenylmethyl)piperidinemethaneamine (0.90 g) synthesized by the standard procedure described in International publication No. 02/053568 (WO 2002/053568), Example 45, step 2 were dissolved in toluene (20 ml), and acetic acid (0.04 ml) was added. The mixture was stirred at room temperature for 2 hours, and then refluxed with heating for 2 hours. The reaction mixture was concentrated, and the resulting residue was purified by silica gel column chromatography (elution solvent; methylene chloride : methanol = 99.5 : 0.5 → 90 : 10) to produce the title compound (1.18 g) as a pale yellow solid.
LC-MS (ESI⁺) : 523 (M⁺H, Rt = 3.21 min)

### <Step 2>

### Synthesis of (±)-tetrahydro-1-(hydroxyethyl)-8a-(methoxymethyl)-1'-(phenylmethyl)spiro[imidazo[1,2-a]pyrazine-2(3H),4'-piperidin]-5(1H)-one

The procedure of Example 1, <Step 3> was repeated by using the compound (0.14 g) of <Step 1> to produce the title compound (0.11 g).
LC-MS (ESI⁺) : 389 (M⁺H, Rt = 0.23 min)

### <Step 3>

### Synthesis of (±)-7-[(1-benzenesulfonyl-5-chloroindol-2-yl)sulfonyl]tetrahydro-1-(hydroxyethyl)-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (0.10 g) of <Step 2> to produce the title compound (0.11 g).
LC-MS (ESI⁺) : 742 (M⁺H, Rt = 5.07 min)

### <Step 4>

### Synthesis of (±)7-[(1-benzenesulfonyl-5-chloroindol-2-yl)sulfonyl]tetrahydro-1-(hydroxyethyl)-8a-(methoxymethyl)-spiro[imidazo[1,2-a]pyrazine-2(3H),4'-piperidin]-5(1H)-one

The procedure of Example 1, <Step 5> was repeated by using the compound (0.11 g) of <Step 3> to produce the title compound (60 mg).
LC-MS (ESI⁺) : 652 (M⁺H, Rt = 4.44 min)

### <Step 5>

### Synthesis of (±)-1'-acetyl-7-[(5-chloro-1H-indol-2-yl)sulfonyl]tetrahydro-1-(hydroxyethyl)-8a-(methoxymethyl)-spiro[imidazo[1,2-a]pyrazine-2(3H),4'-piperidin]-5(1H)-one

The procedure of Example 20, <Step 5> was repeated by using the compound (60 mg) of <Step 4> to produce the title compound (23 mg).

### Example 25

### Synthesis of (-)-1'-acetyl-7-[(5-chloro-1H-indol-2-yl)sulfonyl]tetrahydro-8a-(methoxymethyl)-spiro[imidazo[1,2-a]pyrazine-2(3H),4'-piperidin]-5(1H)-one

### <Step 1>

### Synthesis of (-)-7-[(1-benzenesulfonyl-5-chloroindol-2-yl)sulfonyl]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (0.2 g) of Example 14, <Step 1> to produce the title compound (0.36 g).

NMR data: CDCl₃: 8.23 (1H,d,J = 9Hz), 8.17-7.98 (1H,m), 8.04 (1H,d,J = 9Hz), 7.63-7.53 (2H,m), 7.52-7.40 (4H,m), 7.35-7.20 (5H,m), 4.43 (1H,d,J = 17Hz), 4.20 (1H,d,J = 12Hz), 4.12 (1H,d,J = 13Hz), 3.80 (1H,d,J = 17Hz), 3.61 (1H,d,J = 10Hz), 3.48 (2H,s), 3.32 (1H,d,J = 10Hz), 3.19 (3H,s), 2.92 (1H,d,J = 12Hz), 2.88 (1H,d,J = 13Hz), 2.70-2.15 (4H,m), 1.85-1.40 (4H,m)

### <Step 2>

### Synthesis of (-)-7-[(1-benzenesulfonyl-5-chloroindol-2-yl)sulfonyl]tetrahydro-8a-(methoxymethyl)-spiro[imidazo[1,2-a]pyrazine-2(3H),4'-piperidin]-5(1H)-one

The procedure of Example 1, <Step 5> was repeated by using the compound (0.33 g) of <Step 2> to produce the title compound (0.25 g).
NMR data: CDCl₃*: 8.23 (1H,d,J = 9Hz), 8.11-7.97 (1H,m), 8.04 (1H,d,J = 9Hz), 7.67-7.40(6H,m), 4.44 (1H, d,J = 17Hz), 4.25 (1H,d,J = 12Hz), 4.13 (1H,d,J = 13Hz), 3.81 (1H,d,J = 17Hz), 3.62 (1H,d,J = 10Hz), 3.34 (1H,d,J = 10Hz), 3.21 (3H,s), 3.06-2.82 (2H,m), 2.92 (1H,d,J = 12Hz), 2.90 (1H,d,J = 13Hz), 2.82-2.60 (2H,m), 2.35 (1H,s), 1.80-1.35 (4H,m)

### <Step 3>

### Synthesis of (-)-1'-acetyl-7-[(5-chloro-1H-indol-2-yl)sulfonyl]tetrahydro-8a-(methoxymethyl)-spiro[imidazo[1,2-a]pyrazine-2(3H),4'-piperidin]-5(1H)-one

The procedure of Example 20, <Step 5> was repeated by using the compound (50 mg) of <Step 2> to produce the title compound (21 mg) .

### Example 26

### Synthesis of (-)-7-[(5-chloro-1H-indol-2-yl)sulfonyl]tetrahydro-8a-(methoxymethyl)-1'-propanoyl-spiro[imidazo[1,2-a]pyrazine-2(3H),4'-piperidin]-5(1H)-one

The procedure of Example 3 was repeated by using the compound (50 mg) of Example 25, <Step 2> to produce the title compound (30 mg).

### Example 27

Synthesis of (-)-1'-acetyl-7-[[(1E)-2-(5-chloro-2-thienyl)ethenyl]sulfonyl]tetrahydro-8a-(methoxymethyl)-spiro[imidazo[1,2-a]pyrazine-2(3H),4'-piperidin]-5(1H)-one

### <Step 1>

### Synthesis of (-)-7-[[(1E)-2-(5-chloro-2-thienyl)ethenyl]sulfonyl]tetrahydro-8a-(methoxymethyl)-spiro[imidazo[1,2-a]pyrazine-2(3H),4'-piperidin]-5(1H)-one

The procedure of Example 1, <Step 5> was repeated by using the compound (4.5 g) of Example 14, <Step 2> to produce the title compound (2.6 g).
LC-MS (ESI⁺) : 461 (M⁺H, Rt = 3.04 min)

### <Step 2>

### Synthesis of (-)-1'-acetyl-7-[[(1E)-2-(5-chloro-2-thienyl)ethenyl]sulfonyl]tetrahydro-8a-(methoxymethyl)-spiro[imidazo[1,2-a]pyrazine-2(3H),4'-piperidin]-5(1H)-one

The procedure of Example 1, <Step 6> was repeated by using the compound (0.1 g) of <Step 1> to produce the title compound (70 mg).

### Example 28

### Synthesis of (-)-1'-acetyl-7-[(6-chloro-benzo[b]thiophen-2-yl)sulfonyl]tetrahydro-8a-(methoxymethyl)-spiro[imidazo[1,2-a]pyrazine-2(3H),9'-piperidin]-5(1H)-one

### <Step 1>

### Synthesis of (-)-7-[(6-chloro-benzo[b]thiophen-2-yl)sulfonyl]tetrahydro-8a-(methoxymethyl)-spiro[imidazo[1,2-a]pyrazine-2(3H),4'-piperidin]-5(1H)-one

The procedure of Example 1, <Step 5> was repeated by using the compound (1.5 g) of Example 15, <Step 1> to produce the title compound (0.33 g).
LC-MS (ESI⁺) : 485 (M⁺H, Rt = 3.70 min)

### <Step 2>

### Synthesis of (-)-1'-acetyl-7-[(6-chloro-benzo[b]thiophen-2-yl)sulfonyl]tetrahydro-8a-(methoxymethyl)-spiro[imidazo[1,2-a]pyrazine-2(3H),4'-piperidin]-5(1H)-one

The procedure of Example 1, <Step 6> was repeated by using the compound (50 mg) of <Step 1> to produce the title compound (50 mg).

### Example 29

### Synthesis of (±)-1'-acetyl-7-[(6-chloronaphthalen-2-yl)sulfonyl]tetrahydro-8a-(methoxymethyl)-spiro[imidazo[1,2-a]pyrazine-2(3H),4'-piperidin]-5(1H)-one

### <Step 1>

### Synthesis of (±)-7-[(6-chloronaphthalen-2-yl)sulfonyl]tetrahydro-8a-(methoxymethyl)-1'-(phenylmethyl)-spiro[imidazo[1,2-a]pyrazine-2(3H),4'-piperidin]-5(1H)-one

The procedure of Example 1, <Step 4> was repeated by using the compound (14.7 g) of Example 14, <Step 1> to produce the title compound (22.0 g).
LC-MS (ESI⁺) : 569 (M⁺H, Rt = 4.50 min)

### <Step 2>

### Synthesis of (±)-7-[(6-chloronaphthalen-2-yl)sulfonyl]tetrahydro-8a-(methoxymethyl)-spiro[imidazo[1,2-a]pyrazine-2(3H),4'-piperidin]-5(1H)-one

The procedure of Example 1, <Step 5> was repeated by using the compound (22.0 g) of <Step 1> to produce the title compound (15.8 g).
LC-MS (ESI⁺) : 478 (M⁺H, Rt = 3.68 min)

### <Step 3>

### Synthesis of (±)-1'-acetyl-7-[(6-chloronaphthalen-2-yl)sulfonyl]tetrahydro-8a-(methoxymethyl)-spiro[imidazo[l,2-a]pyrazine-2(3H),4'-piperidin]-5(1H)-one

The procedure of Example 1, <Step 6> was repeated by using the compound (0.3 g) of <Step 2> to produce the title compound (0.3 g).

### Example 30

### Synthesis of (-)-7-[(5-chloro-1H-indol-2-yl)sulfonyl]tetrahydro-8a-(methoxymethyl)-1'-propanoyl-spiro[5H-oxazolo[3,2-a]pyrazine-2(3H),4'-piperidin]-5-one

### <Step 1>

### Synthesis of phenylmethyl (-)-tetrahydro-8a-(methoxymethyl)-5-oxo-1'-(phenylmethyl)-spiro[5H-oxazolo[3,2-a]pyrazine-2(3H),4'-piperidine]-7-carboxylate

Phenylmethyl (±)-tetrahydro-8a-(methoxymethyl)-5-oxo-1'-(phenylmethyl)-spiro[5H-oxazolo[3,2-a]pyrazine-2(3H),4'-piperidine]-7-carboxylate (23.4 g) of International publication No. 02/053568 (WO 2002/053568), Example 45, step 3 was separated by the procedure of Example 1, <Step 1> to obtain phenylmethyl (+)-tetrahydro-8a-(methoxymethyl)-5-oxo-1'-(phenylmethyl)-spiro[5H-oxazolo[3,2-a]pyrazine-2(3H),4'-piperidine]-7-carboxylate (first peak, 8.8 g, [α]_{D}²⁵+48.8° (c1. 00, CHCl₃), >98%ee) and phenylmethyl (-)-tetrahydro-8a-(methoxymethyl)-5-oxo-1'-(phenylmethyl)-spiro[5H-oxazolo[3,2-a]pyrazine-2(3H),4'-piperidine]-7-carboxylate (second peak, 9.0 g, [α]_{D}²⁸-48.6° (c1. 00, CHCl₃), >98%ee).

NMR data: DMSO-d₆, 100°C: 7.90-7.20(10H,m), 5.12 (2H,s), 4.35 (1H,d,J = 13Hz), 4.22 (1H,d,J = 18Hz), 4.12 (1H,d,J = 12Hz), 3.80 (1H,d,J = 18Hz), 3.47 (2H,s), 3.37 (1H,d,J = 11Hz), 3.31 (1H,d,J = 11Hz), 3.23 (3H, s), 3.10-2.89 (2H,m), 2.58-2.43 (2H,m), 2.38-2.26 (2H,m), 1.83-1.72 (2H,m), 1.58-1.43 (2H,m)

### <Step 2>

### Synthesis of (-)-tetrahydro-8a-(methoxymethyl)-1'-(phenylmethyl)-spiro[5H-oxazolo[3,2-a]pyrazine-2(3H),4'-piperidin]-5-one

The procedure of Example 1, <Step 3> was repeated by using the compound (2 g) of <Step 1> to produce the title compound (1.3 g).
NMR data: CDCl₃: 7.80-7.40 (5H, m), 4.38 (1H,d,J = 12Hz), 3.62-3.37(7H,m), 3.38 (3H,s), 3.04 (1H,d,J = 12Hz), 2.62 (1H,d,J = 13Hz), 2.69-2.23 (4H,m), 1.96-1.48 (4H,m)

### <Step 3>

### Synthesis of (-)-7-[(1-benzenesulfonyl-5-chloroindol-2-yl)sulfonyl]tetrahydro-8a-(methoxymethyl)-1'-(phenylmethyl)-spiro[5H-oxazolo[3,2-a]pyrazine-2(3H),4'-piperidin]-5-one

The procedure of Example 1, <Step 4> was repeated by using the compound (0.8 g) of <Step 2> to produce the title compound (1.6 g).
NMR data: CDCl₃: 8.23 (1H,d,J = 9Hz), 8.08-8.03 (2H,m), 7.63-7.56 (2H,m), 7.52-7.92 (4H,m), 7.34-7.23 (5H,m), 4.97 (1H,d,J = 17Hz), 4.33 (1H,d,J = 13Hz), 4.26 (1H,d,J = 12Hz), 3 . 82 (1H,d,J = 18Hz), 3.60 (1H,d,J = 10Hz), 3.49 (2H,s), 3.44-3.37 (1H,m), 3.16 (3H,s), 3.13 (1H,d,J = 12Hz), 2.91 (1H,d,J = 13Hz), 2.67-2.26 (4H,m), 1.98-1.50 (4H,m)

### <Step 4>

### Synthesis of (-)-7-[(1-benzenesulfonyl-5-chloroindol-2-yl)sulfonyl]tetrahydro-8a-(methoxymethyl)-spiro[5H-oxazolo[3,2-a]pyrazine-2(3H),4'-piperidin]-5-one

The procedure of Example 1, <Step 5> was repeated by using the compound (1.6 g) of <Step 3> to produce the title compound (1.1 g).
NMR data: CDCl₃: 8.23 (1H,d,J = 9Hz), 8.09-8.01 (2H,m), 7.64-7.56 (2H,m), 7.53-7.42 (4H,m), 4.48 (1H,d,J = 17Hz), 4.39-4.26 (2H,m), 3.83 (1H,d,J = 17Hz), 3.62 (1H,d,J = 10Hz), 3.46-3.37 (1H,m), 3.17 (3H,s), 3.13 (1H,d,J = 12Hz), 3.10-2.94 (2H,m), 2.93 (1H,d,J = 13Hz), 2.83-2.67 (2H,m), 1.92-1.46 (4H,m)

### <Step 5>

### Synthesis of (-)-7-[(5-chloro-1H-indol-2-yl)sulfonyl]tetrahydro-8a-(methoxymethyl)-1'-propanoyl-spiro[5H-oxazolo[3,2-a]pyrazine-2(3H),4'-piperidin]-5-one

The procedure of Example 3 was repeated by using the compound (0.1 g) of <Step 4> to produce the title compound (69 mg).

### Example 31

### Synthesis of (-)-7-[(6-chloro-benzo[b]thiophen-2-yl)sulfonyl]tetrahydro-8a-(methoxymethyl)-1'-propanoyl-spiro[5H-oxazolo[3,2-a]pyrazine-2(3H),4'-piperidin]-5-one

### <Step 1>

### Synthesis of (-)-7-[(6-chloro-benzo[b]thiophen-2-yl)sulfonyl]tetrahydro-8a-(methoxymethyl)-1'-(phenylmethyl)-spiro[5H-oxazolo[3,2-a]pyrazine-2(3H),4'-piperidin]-5-one

The procedure of Example 1, <Step 4> was repeated by using the compound (0.93 g) of Example 30, <Step 2> to produce the title compound (1.55 g).
LC-MS (ESI⁺): 576 (M⁺H, Rt = 4.73 min)

### <Step 2>

### Synthesis of (-)-7-[(6-chloro-benzo[b]thiophen-2-yl)sulfonyl]tetrahydro-8a-(methoxymethyl)-spiro[5H-oxazolo[3,2-a]pyrazine-2(3H),4'-piperidin]-5-one

The procedure of Example 1, <Step 5> was repeated by using the compound (1.55 g) of <Step 1> to produce the title compound (1.10 g).
LC-MS (ESI⁺): 486 (M⁺H, Rt = 3.80 min)

### <Step 3>

### Synthesis of (-)-7-[(6-chloro-benzo[b]thiophen-2-yl)sulfonyl]tetrahydro-8a-(methoxymethyl)-1'-propanoyl-spiro[5H-oxazolo[3,2-a]pyrazine-2(3H),4'-piperidin]-5-one

The procedure of Example 2, <Step 2> was repeated by using the compound (1.55 g) of <Step 2> to produce the title compound (1.10 g).

### Example 32

### Synthesis of (-)-7-[(5-chloro-1H-indol-2-yl)sulfonyl]tetrahydro-8a-(methoxymethyl)-1'-propenoyl-spiro[5H-oxazolo[3,2-a]pyrazine-2(3H),4'-piperidin]-5-one

The procedure of Example 20, <Step 5> was repeated by using the compound (0.1 g) of Example 30, <Step 4> to produce the title compound (62 mg).

### Example 33

### Synthesis of (-)-7-[(6-chloro-benzo[b]thiophen-2-yl)sulfonyl]tetrahydro-8a-(methoxymethyl)-1'-propenoyl-spiro[5H-oxazolo[3,2-a]pyrazine-2(3H),4'-piperidin]-5-one

The procedure of Example 2, <Step 2> was repeated by using the compound (50 mg) of Example 31, <Step 2> to produce the title compound (47 mg).

### Example 34

### Synthesis of (-)-1'-acetyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one

### <Step 1>

### Synthesis of (-)-1'-acetyl-7-[(1-benzenesulfonyl-5-chloroindol-2-yl)sulfonyl]tetrahydro-8a-(methoxymethyl)-spiro[5H-oxazolo[3,2-a]pyrazine-2(3H),4'-piperidin]-5-one

The procedure of Example 1, <Step 6> was repeated by using the compound (0.1 g) of Example 30, <Step 4> to produce the title compound (79 mg).
NMR data: CDCl₃: 8.23 (1H,d,J = 9Hz), 8.08-8.00 (2H,m), 7.64-7.42(6H,m), 4.53-4.21 (3H,m), 4.08-3.97 (1H,m), 3.83 (1H,d,J = 17Hz), 3.64-3.14(6H,m), 3.21 (3H,s), 3.02-2.91 (1H,m), 2.13-2.07 (3H,m), 1.96-1.48 (4H,m)

### <Step 2>

### Synthesis of (-)-1'-acetyl-7-[(5-chloro-1H-indol-2-yl)sulfonyl]tetrahydro-8a-(methoxymethyl)-spiro[5H-oxazolo[3,2-a]pyrazine-2(3H),9'-piperidin]-5-one

The procedure of Example 1, <Step 7> was repeated by using the compound (73 mg) of <Step 1> to produce the title compound (25 mg).

### Example 35

### Synthesis of (-)-1'-acetyl-7-[(6-chloro-benzo[b]thiophen-2-yl)sulfonyl]tetrahydro-8a-(methoxymethyl)-spiro[5H-oxazolo[3,2-a]pyrazine-2(3H),4'-piperidin]-5-one

### <Step 1>

### Synthesis of phenylmethyl (-)-tetrahydro-8a-(methoxymethyl)-5-oxo-spiro[5H-oxazolo[3,2-a]pyrazine-2(3H),4'-piperidine]-7-carboxylate

The procedure of Example 1, <Step 5> was repeated by using the compound (1.2 g) of Example 30, <Step 1> to produce the title compound (0.87 g).
LC-MS (ESI⁺) : 390 (M⁺H, Rt = 1.86 min)

### <Step 2>

### Synthesis of phenylmethyl (-)-1'-acetyl-tetrahydro-8a-(methoxymethyl)-5-oxo-spiro[5H-oxazolo[3,2-a]pyrazine-2(3H),4'-piperidine]-7-carboxylate

The procedure of Example 1, <Step 6> was repeated by using the compound (0.85 g) of <Step 1> to produce the title compound (0.93 g).
LC-MS (ESI⁺) : 432 (M⁺H, Rt = 5.01 min)

### <Step 3>

### Synthesis of (-)-1'-acetyltetrahydro-8a-(methoxymethyl)-spiro[5H-oxazolo[3,2-a]pyrazine-2(3H),4'-piperidin]-5-one

The compound (0.90 g) of <Step 2> was dissolved in methanol (9 ml), and 10% palladium-carbon (90 mg) was added to the solution. After stirring the mixture overnight at room temperature in a hydrogen atmosphere, the reaction mixture was filtered through Celite, and washed with methanol. The filtrate was concentrated, and the resulting residue was purified by silica gel column chromatography [Chromatorex NHTM] (elution solvent; methylene chloride : methanol = 30 : 1) to produce the title compound (0.52 g).
NMR data: DMSO-d₆, 100°C: 4.17 (1H,d,J = 11Hz), 3.59 (1H,d,J = 11Hz), 3.44 (1H,d,J = 11Hz), 3.33 (3H,s), 3 . 08 (1H,d,J = 11Hz), 3.64-2.88(8H,m), 1.97 (3H,s), 1.80-1.65 (2H,m), 1.50-1.38 (2H,m)

### <Step 4>

### Synthesis of (-)-1'-acetyl-7-[(6-chloro-benzo[b]thiophen-2-yl)sulfonyl]tetrahydro-8a-(methoxymethyl)-spiro[5H-oxazolo[3,2-a]pyrazine-2(3H),4'-piperidin]-5-one

The procedure of Example 1, <Step 4> was repeated by using the compound (0.85 g) of <Step 3> to produce the title compound (0.93 g).

### Example 36

### Synthesis of (-)-1'-acetyl-7-[[(1E)-2-(5-chloro-2-thienyl)ethenyl]sulfonyl]tetrahydro-8a-(methoxymethyl)-spiro[5H-oxazolo[3,2-a]pyrazine-2(3H),4'-piperidin]-5-one

The procedure of Example 1, <Step 4> was repeated by using the compound (50 mg) of Example 35, <Step 3> to produce the title compound (63 mg).

### Example 37

### Synthesis of (-)-1'-acetyl-7-[(6-chloronaphthalen-2-yl)sulfonyl]tetrahydro-8a-(methoxymethyl)-spiro[5H-oxazolo[3,2-a]pyrazine-2(3H),4'-piperidin]-5-one

The procedure of Example 1, <Step 4> was repeated by using the compound (50 mg) of Example 35, <Step 3> to produce the title compound (74 mg).

### Example 38

### Synthesis of (-)-7-[(6-chloro-benzo[b]thiophen-2-yl)sulfonyl]tetrahydro-8a-(methoxymethyl)-1'-propinoyl-spiro[5H-oxazolo[3,2-a]pyrazine-2(3H),4'-piperidin]-5-one

The procedure of Example 8, <Step 1> was repeated by using the compound (50 mg) of Example 31, <Step 2> to produce the title compound (46 mg).

### Example 39

### Synthesis of (-)-1'-n-butanoyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one

The procedure of Example 3 was repeated by using the compound (50 mg) of Example 30, <Step 4> to produce the title compound (59 mg).

### Example 40

### Synthesis of (-)-7-[(5-chloro-1H-indol-2-yl)sulfonyl]tetrahydro-1'-cyclopropanoyl-8a-(methoxymethyl)-spiro[5H-oxazolo[3,2-a]pyrazine-2(3H),4'-piperidin]-5-one

The procedure of Example 3 was repeated by using the compound (0.1 g) of Example 30, <Step 4> to produce the title compound (59 mg).

### Example 41

### Synthesis of (-)-7-[(5-chloro-1H-indol-2-yl)sulfonyl]tetrahydro-1'-(methoxyacetyl)-8a-(methoxymethyl)-spiro[5H-oxazolo[3,2-a]pyrazine-2(3H),4'-piperidin]-5-one

The procedure of Example 3 was repeated by using the compound (0.1 g) of Example 30, <Step 4> to produce the title compound (69 mg).

### Example 42

### Synthesis of (-)-7-[(5-chloro-1H-indol-2-yl)sulfonyl]tetrahydro-1'-(hydroxyacetyl)-8a-(methoxymethyl)-spiro[5H-oxazolo[3,2-a]pyrazine-2(3H),4'-piperidin]-5-one

The procedure of Example 3 was repeated by using the compound (80 mg) of Example 30, <Step 4> to produce the title compound (45 mg).

### Example 43

### Synthesis of (-)-7-[(5-chloro-1H-indol-2-yl)sulfonyl]tetrahydro-1'-(2-furanoyl)-8a-(methoxymethyl)-spiro[5H-oxazolo[3,2-a]pyrazine-2(3H),4'-piperidin]-5-one

The procedure of Example 3 was repeated by using the compound (0.1 g) of Example 30, <Step 4> to produce the title compound (35 mg).

### Example 44

Synthesis of (-)-7-[(5-chloro-1H-indol-2-yl)sulfonyl]tetrahydro-8a-(methoxymethyl)-1'-(2-methylpropanoyl)-spiro[5H-oxazolo[3,2-a]pyrazine-2(3H),4'-piperidin]-5-one

The procedure of Example 3 was repeated by using the compound (0.15 g) of Example 30, <Step 4> to produce the title compound (48 mg).

### Example 45

### Synthesis of (-)-7-[(5-chloro-1H-indol-2-yl)sulfonyl]tetrahydro-8a-(methoxymethyl)-1'-(3-methoxypropanoyl)-spiro[5H-oxazolo[3,2-a]pyrazine-2(3H),4'-piperidin]-5-one

The procedure of Example 8 was repeated by using the compound (0.1 g) of Example 30, <Step 4> to produce the title compound (7 mg).

### Example 46

### Synthesis of (-)-7-[(5-chloro-1H-indol-2-yl)sulfonyl]tetrahydro-8a-(methoxymethyl)-1'-(2-oxopropanoyl)-spiro[5H-oxazolo[3,2-a]pyrazine-2(3H),4'-piperidin]-5-one

The procedure of Example 8 was repeated by using the compound (0.1 g) of Example 30, <Step 4> to produce the title compound (3 mg).

### Example 47

### Synthesis of (-)-7-[(5-chloro-1H-indol-2-yl)sulfonyl]tetrahydro-8a-(methoxymethyl)-1'-(2,2-dimethylpropanoyl)-spiro[5H-oxazolo[3,2-a]pyrazine-2(3H),4'-piperidin]-5-one

The procedure of Example 3 was repeated by using the compound (0.1 g) of Example 30, <Step 4> to produce the title compound (30 mg).

### Example 48

### Synthesis of (±)-1'-acetyl-7-[(5-chloro-1H-indol-2-yl)sulfonyl]tetrahydro-8a-(hydroxymethyl)-spiro[5H-oxazolo[3,2-a]pyrazine-2(3H),4'-piperidin]-5-one

### <Step 1>

### Synthesis of phenylmethyl (±)-tetrahydro-8a-(1-methylethoxymethyl)-5-oxo-1'-(phenylmethyl)-spiro[imidazo[1,2-a]pyrazine-2(3H),4'-piperidine]-7-carboxylate

The procedure of Example 18, <Step 1> was repeated by using ethyl-2-[(1-methylethoxymethyl-2-oxopropan-1-yl)(phenylmethyl 7-carboxylate)amino]acetate (5.6 g) and 4-amino-4-(hydroxymethyl)-1-(phenylmethyl)piperidine (7.5 g) synthesized by the standard procedure described in International publication No. 02/053568 (WO 2002/053568), Example 45, steps 1 and 2 to thereby produce the title compound (9.0 g).
LC-MS (ESI⁺) : 509 (M⁺H, Rt = 3.98 min)

### <Step 2>

### Synthesis of phenylmethyl (±)-tetrahydro-8a-(1-methylethoxymethyl)-5-oxo-spiro[imidazo[1,2-a]pyrazine-2(3H),4'-piperidine]-7-carboxylate

The procedure of Example 1, <Step 5> was repeated by using the compound (4.5 g) of <Step 1> to produce the title compound (1.1 g).
LC-MS (ESI⁺) : 418 (M⁺H, Rt = 2.83 min)

### <Step 3>

### Synthesis of phenylmethyl (±)-1'-acetyl-tetrahydro-8a-(1-methylethoxymethyl)-5-oxo-spiro[imidazo[1,2-a]pyrazine-2(3H),4'-piperidine]-7-carboxylate

The procedure of Example 1, <Step 6> was repeated by using the compound (4.2 g) of <Step 2> to produce the title compound (5.3 g).
NMR data: DMSO-d₆, 100°C: 7.39-7.27 (5H,m), 5.12 (2H,s), 4.37 (1H, d,J = 13Hz), 4.22 (1H, d,J = 18Hz), 4.14 (1H,d,J = 11Hz), 3.81 (1H,d,J = 18Hz), 3.64-3.27 (5H,m), 3.39 (2H,s), 3.16 (1H,d,J = 11Hz), 3.02-2.92 (1H, m), 1.97 (3H,s), 1.79-1.69 (2H,m), 1.53-1.45 (2H,m), 1.03 (3H,d, J = 6Hz), 1.02 (3H,d, J = 6Hz)

### <Step 4>

### Synthesis of (±)-1'-acetyl-tetrahydro-8a-(hydroxymethyl)-spiro[imidazo[1,2-a]pyrazine-2(3H),4'-piperidin]-5(1H)-one trifluoromethanesulfonate

The procedure of Example 1, <Step 3> was repeated by using the compound (5.3 g) of <Step 3> to produce the title compound (7.3 g).
LC-MS (ESI⁺) : 284 (M⁺H, Rt = 0.21 min)

### <Step 5>

### Synthesis of (±)-1'-acetyl-7-[(5-chloro-1H-indol-2-yl)sulfonyl]tetrahydro-8a-(hydroxymethyl)-spiro[5H-oxazolo[3,2-a]pyrazine-2(3H),4'-piperidin]-5-one

The procedure of Example 3 was repeated by using the compound (50 mg) of <Step 4> to produce the title compound (22 mg).

### Example 49

### Synthesis of (±)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one

### <Step 1>

### Synthesis of tert-butyl (±)-1'-acetyl-tetrahydro-8a-(hydroxymethyl)-5-oxo-spiro[imidazo[1,2-a]pyrazine-2(3H),4'-piperidine]-7-carboxylate

The compound (7.2 g) of Example 48, <Step 4> was dissolved in dioxane (72 ml), and 1M aqueous solution of sodium hydroxide (72 ml) was added. Di-tert-butyl bicarbonate was added to the reaction mixture, and the mixture was stirred at room temperature for 2 hours. Methylene chloride was added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; ethyl acetate to ethyl acetate : methanol = 10 : 1) to produce the title compound (2.5 g).
LC-MS (ESI⁺) : 384 (M⁺H, Rt = 3.60 min)

### <Step 2>

### Synthesis of tert-butyl (±)-1'-acetyl-tetrahydro-S-oxo-8a-(2-pyridylmethoxymethyl)-spiro[imidazo[1,2-a]pyrazine-2(3H),4'-piperidine]-7-carboxylate

The compound (0.3 g) of <Step 1> was dissolved in methylene chloride (2.4 ml), and (phenylmethyl)triethyl ammonium chloride (18 mg) and 50% aqueous sodium hydroxide (2.4 ml) were added. In an ice bath, 2-pyridylmethyl chloride (0.2 g) was added to the reaction mixture, and the mixture was stirred at room temperature for one hour. Water and methylene chloride were added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; ethyl acetate to ethyl acetate : methanol = 10 : 1) to produce the title compound (2.5 g).
LC-MS (ESI⁺): 475 (M⁺H, Rt = 3.34 min)

### <Step 3>

### Synthesis of (±)-1'-acetyl-7-[(1-benzenesulfonyl-5-chloroindol-2-yl)sulfonyl]tetrahydro-8a-(2-pyridylmethoxymethyl)-spiro[imidazo[1,2-a]pyrazine-2(3H),4'-piperidin]-5(1H)-one

The procedure of Example 18, <Step 6> was repeated by using the compound (0.12 g) of <Step 2>, and after the reaction, the procedure of Example 1, <Step 4> was repeated to produce the title compound (0.13 g).
LC-MS (ESI⁺) : 728 (M⁺H, Rt = 7.28 min)

### <Step 4>

### Synthesis of (±)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one

The procedure of Example 1, <Step 7> was repeated by using the compound (0.11 g) of <Step 3> to produce the title compound (78 mg).

### Example 50

### Synthesis of (-)-1'-acetyl-7-[(5-chloro-1H-indol-2-yl)sulfonyl]tetrahydro-8a-(hydroxymethyl)-spiro[5H-oxazolo[3,2-a]pyrazine-2(3H),4'-piperidin]-5-one

### <Step 1>

### Synthesis of (-)-1'-acetyl-7-[(1-benzenesulfonyl-5-chloroindol-2-yl)sulfonyl]tetrahydro-8a-(hydroxymethyl)-spiro[5H-oxazolo[3,2-a]pyrazine-2(3H),4'-piperidin]-5-one

The compound (0.74 g) of Example 34, <Step 1> was dissolved in methylene chloride (37 ml), and boron tribromide (0.54 ml) was added at -78(C. The mixture was stirred at the same temperature for 25 minutes, and for another 2 hours in an ice bath. Ether was added at the same temperature, and after adding ether, the mixture was stirred for 30 minutes. Water and methylene chloride were added for extraction with methylene chloride. The methylene chloride layer was washed with saturated aqueous solution of sodium chloride, and dried with anhydrous sodium sulfate. The solvent was removed under reduced pressure to produce the title compound (0.69 g).
LC-MS (ESI⁺) : 637 (M⁺H, Rt = 6.88 min)

### <Step 2>

### Synthesis of (-)-1'-acetyl-7-[(5-chloro-1H-indol-2-yl)sulfonyl]tetrahydro-8a-(hydroxymethyl)-spiro[5H-oxazolo[3,2-a]pyrazine-2(3H),4'-piperidin]-5-one

The procedure of Example 1, <Step 7> was repeated by using the compound (98 mg) of <Step 1> to produce the title compound (30 mg).

### Example 51

### Synthesis of (-)-7-[(5-chloro-1H-indol-2-yl)sulfonyl]tetrahydro-8a-(hydroxymethyl)-1'-propanoyl-spiro[5H-oxazolo[3,2-a]pyrazine-2(3H),4'-piperidin]-5-one

### <Step 1>

### Synthesis of (-)-7-[(1-benzenesulfonyl-5-chloroindol-2-yl)sulfonyl]tetrahydro-8a-(hydroxymethyl)-spiro[5H-oxazolo[3,2-a]pyrazine-2(3H),4'-piperidin]-5-one

The procedure of Example 50, <Step 1> was repeated by using the compound (0.1 g) of Example 30, <Step 4> to produce the title compound (89 mg).
LC-MS (ESI⁺): 595(M-HBr, Rt = 4.04 min)

### <Step 2>

### Synthesis of (-)-7-[(5-chloro-1H-indol-2-yl)sulfonyl]tetrahydro-8a-(hydroxymethyl)-1'-propanoyl-spiro[5H-oxazolo[3,2-a]pyrazine-2(3H),4'-piperidin]-5-one

The procedure of Example 3 was repeated by using the compound (30 mg) of <Step 1> to produce the title compound (13 mg).

### Example 52

### Synthesis of (-)-7-[(5-chloro-1H-indol-2-yl)sulfonyl]tetrahydro-1'-cyclopropanoyl-8a-(hydroxymethyl)-spiro[5H-oxazolo[3,2-a]pyrazine-2(3H),4'-piperidin]-5-one

The procedure of Example 3 was repeated by using the compound (0.2 g) of Example 51, <Step 1> to produce the title compound (52 mg).

### Example 53

### Synthesis of (-)-7-[(5-chloro-1H-indol-2-yl)sulfonyl]tetrahydro-1'-(2-furanoyl)-8a-(hydroxymethyl)-spiro[5H-oxazolo[3,2-a]pyrazine-2(3H),4'-piperidin]-5-one

The procedure of Example 3 was repeated by using the compound (0.2 g) of Example 51, <Step 1> to produce the title compound (45 mg).

### Example 54

### Synthesis of (-)-1'-acetyl-7-[(5-chloro-1H-indol-2-yl)sulfonyl]tetrahydro-8a-(methoxycarbonylmethoxymethyl)-spiro[5H-oxazolo[3,2-a]pyrazine-2(3H),4'-piperidin]-5-one

The procedure of Example 49, <Step 2> was repeated by using the compound (0.1 g) of Example 50, <Step 2> to produce the title compound (10 mg).

### Example 55

### Synthesis of (-)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one

The procedure of Example 49, <Step 2> was repeated by using the compound (50 mg) of Example 50, <Step 2> to produce the title compound (12 mg).

### Example 56

### Synthesis of (-)-1'-acetyl-7-[(5-chloro-1H-indol-2-yl)sulfonyl]tetrahydro-8a-(2-pyrimidyloxymethyl)-spiro[5H-oxazolo[3,2-a]pyrazine-2(3H),9'-piperidin]-5-one

The procedure of Example 49, <Step 2> was repeated by using the compound (40 mg) of Example 50, <Step 2> to produce the title compound (25 mg).

### Example 57

### Synthesis of (±)-1'-acetyl-8a-(aminomethyl)-7-[(5-chloro-1H-indol-2-yl)sulfonyl]tetrahydro-spiro[5H-oxazolo[3,2-a]pyrazine-2(3H),4'-piperidin]-5-one

### <Step 1>

### Synthesis of tert-butyl (±)-tetrahydro-5-oxo-1'-(phenylmethyl)-8a-(phthalimid-1-ylmethyl)-spiro[5H-oxazolo[3,2-a]pyrazine-2(3H),4'-piperidine]-7-carboxylate

The procedure of Example 18, <Step 1> was repeated by using N-[3-(phthalimid-1-yl)-2-oxopropyl]-N-[(tert-butoxy) carbonyl]-glycine ethyl ester (20 g) and 4-(aminomethyl)-1-(phenylmethyl)-4-piperidinol (19 g) synthesized by the standard procedure described in International publication No. 02/053568 (WO 2002/053568), Example 45, steps 1 and 2 to produce the title compound (21 g).
LC-MS (ESI⁺) : 561 (M⁺H, Rt = 3.90 min)

### <Step 2>

### Synthesis of tert-butyl (±)-tetrahydro-5-oxo-8a-(phthalimid-1-ylmethyl)-spiro[5H-oxazolo[3,2-a]pyrazine-2(3H),9'-piperidine]-7-carboxylate

The procedure of Example 1, <Step 5> was repeated by using the compound (1 g) of <Step 1> to produce the title compound (0.4 g).
LC-MS (ESI⁺) : 431 (M⁺H, Rt = 0.23 min)

### <Step 3>

### Synthesis of tert-butyl (±)-1'-acetyl-tetrahydro-7-oxo-8a-(phthalimid-1-ylmethyl)-spiro[5H-oxazolo[3,2-a]pyrazine-2(3H),4'-piperidine]-7-carboxylate

The procedure of Example 1, <Step 6> was repeated by using the compound (0.4 g) of <Step 2> to produce the title compound (0.4 g).
LC-MS (ESI⁺): 513 (M⁺H, Rt = 5.63 min)

### <Step 4>

### Synthesis of (±)-1'-acetyl-7-[(1-benzenesulfonyl-5-chloroindol-2-yl)sulfonyl]tetrahydro-8a-(phthalimid-1-ylmethyl)-spiro[5H-oxazolo[3,2-a]pyrazine-2(3H),4'-piperidin]-5-one

The procedure of Example 18, <Step 6> was repeated by using the compound (0.4 g) of <Step 3>, and after the reaction, the procedure of Example 1, <Step 4> was repeated to produce the title compound (37 mg).
LC-MS (ESI⁺) : 766 (M⁺H, Rt = 8.23 min)

### <Step 5>

### Synthesis of (±)-1'-acetyl-8a-(aminomethyl)-7-[(5-chloro-1H-indol-2-yl)sulfonyl]tetrahydro-spiro[5H-oxazolo[3,2-a]pyrazine-2(3H),9'-piperidin]-5-one

The procedure of Example 18, <Step 3> was repeated by using the compound (32 mg) of <Step 5> to produce the title compound (11 mg).

### Example 58

### Synthesis of (-)-1'-acetyl-8a-(aminomethyl)-7-[(5-chloro-1H-indol-2-yl)sulfonyl]tetrahydro-spiro[5H-oxazolo[3,2-a]pyrazine-2(3H),9'-piperidin]-5-one

### <Step 1>

### Synthesis of (-)-1'-acetyl-7-[(1-benzenesulfonyl-5-chloroindol-2-yl)sulfonyl]tetrahydro-8a-(methanesulfonyloxymethyl)-spiro[5H-oxazolo[3,2-a]pyrazine-2(3H),4'-piperidin]-5-one

The compound (0.5 g) of Example 50, <Step 1> was dissolved in methylene chloride (2.4 ml), and triethylamine (0.22 ml) was added to the solution. Methanesulfonyl chloride (76 µl) was added in an ice bath, and after allowing the mixture to warm to room temperature, the mixture was stirred for one hour. Saturated aqueous solution of sodium hydrogencarbonate and methylene chloride were added for extraction with methylene chloride, and the methylene chloride layer was washed with saturated aqueous solution of sodium chloride, and dried with anhydrous sodium sulfate. The solvent was removed under reduced pressure to produce the title compound (0.57 g).
LC-MS (ESI⁺) : 715 (M⁺H, Rt = 7.70 min)

### <Step 2>

### Synthesis of (-)-1'-acetyl-7-[(5-chloro-1H-indol-2-yl)sulfonyl]tetrahydro-8a-(methanesulfonyloxymethyl)-spiro[5H-oxazolo[3,2-a]pyrazine-2(3H),4'-piperidin]-5-one

The procedure of Example 1, <Step 7> was repeated by using the compound (1.2 g) of <Step 1> to produce the title compound (0.76 g).
LC-MS (ESI⁺): 577 (M⁺H, Rt = 6.45 min)

### <Step 3>

### Synthesis of (-)-1'-acetyl-8a-(azidomethyl)-7-[(5-chloro-1H-indol-2-yl)sulfonyl]tetrahydro-spiro[5H-oxazolo[3,2-a]pyrazine-2(3H),4'-piperidin]-5-one

The compound (0.58 g) of <Step 2> was suspended in dimethylformamide (4 ml), and sodium azide (0.66 g) was added to the suspension. The suspension was stirred with heating at 110 to 120°C for 10 hours, and after allowing the suspension to cool, water and methylene chloride were added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride → methylene chloride : methanol = 4 : 1) to produce the title compound (0.11 g).
LC-MS (ESI⁺): 522 (M⁺H, Rt = 6.88 min)

### <Step 4>

### Synthesis of (-)-1'-acetyl-8a-(aminomethyl)-7-[(5-chloro-1H-indol-2-yl)sulfonyl]tetrahydro-spiro[5H-oxazolo[3,2-a]pyrazine-2(3H),9'-piperidin]-5-one

The compound (0.11 g) of <Step 3> was dissolved in tetrahydrofuran (2.8 ml) - water (0.7 ml), and triphenylphosphine (83 mg) was added to the solution. The mixture was refluxed with heating for 30 minutes, and after cooling in an ice bath, 1M hydrochloric acid was added and the mixture was extracted with ethyl acetate. The ethyl acetate layer was extracted with 1M hydrochloric acid, and after combining with the aqueous layer, the mixture was extracted with ethyl acetate. 1M aqueous solution of sodium hydroxide was added to the aqueous layer to adjust the pH to about 10, and the mixture was extracted with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride to methylene chloride : methanol = 9 : 1) to produce the title compound (0.09 g).

### Example 59

### Synthesis of (±)-1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),4'-piperidin]-5-one

### <Step 1>

### Synthesis of tert-butyl (±)-8a-(aminomethyl)-tetrahydro-5-oxo-1'-(phenylmethyl)-spiro[5H-oxazolo[3,2-a]pyrazine-2(3H),4'-piperidine]-7-carboxylate

The procedure of Example 18, <Step 3> was repeated by using the compound (1.0 g) of Example 57, <Step 1> to produce the title compound (0.75 g).
LC-MS (ESI⁺) : 431 (M⁺H, Rt = 0.23 min)

### <Step 2>

### Synthesis of tert-butyl (±)-8a-(N,N-dimethylaminomethyl)-tetrahydro-5-oxo 1'-(phenylmethyl)-spiro[5H-oxazolo[3,2-a]pyrazine-2(3H),4'-piperidine]-7-carboxylate

The procedure of Example 1, <Step 2> was repeated by using the compound (2.0 g) of <Step 1> to produce the title compound (2.4 g).
LC-MS (ESI⁺) : 459 (M⁺H, Rt = 0.53 min)

### <Step 3>

### Synthesis of (±)-tetrahydro-8a-(N,N-dimethylaminomethyl)-1'-(phenylmethyl) spiro[5H-oxazolo[3,2-a]pyrazine-2(3H),4'-piperidin]-5-one

The procedure of Example 18, <Step 6> was repeated by using the compound (2.4 g) of <Step 2> to produce the title compound (1.7 g).
LC-MS (ESI⁺) : 359 (M⁺H, Rt = 0.23 min)

### <Step 4>

### Synthesis of (±)-7-[(1-benzenesulfonyl-5-chloroindol-2-yl)sulfonyl]tetrahydro-8a-(N,N-dimethylaminomethyl)-1'-(phenylmethyl)-spiro[5H-oxazolo[3,2-a]pyrazine-2(3H),4'-piperidin]-5-one

The procedure of Example 1, <Step 4> was repeated by using the compound (1 g) of <Step 3> to produce the title compound (2.1 g).
LC-MS (ESI⁺) : 712 (M⁺H, Rt = 5.83 min)

### <Step 5>

### Synthesis of (±)-7-[(1-benzenesulfonyl-5-chloroindol-2-yl)sulfonyl]tetrahydro-8a-(N,N-dimethylaminomethyl)-spiro[5H-oxazolo[3,2-a]pyrazine-2(3H),4'-piperidin]-5-one

The procedure of Example 1, <Step 5> was repeated by using the compound (2.1 g) of <Step 4> to produce the title compound (1.4 g).
LC-MS (ESI⁺) : 622 (M⁺H, Rt = 3.40 min)

### <Step 6>

### Synthesis of (±)-1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),9'-piperidin]-5-one

The procedure of Example 20, <Step 5> was repeated by using the compound (0.2 g) of <Step 5> to produce the title compound (0.11 g).

### Example 60

### Synthesis of optically active 1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),4'-piperidin]-5-one

The procedure of Example 21 was repeated by using the compound (70 mg) of Example 59, <Step 6> to produce the title compound (first peak, 29 mg; second peak, 27 mg).

### Example 61

### Synthesis of (±)-8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one

### <Step 1>

### Synthesis of (±)-7-[(1-benzenesulfonyl-5-chloroindol-2-yl)sulfonyl]tetrahydro-1'-(phenylmethyl)-8a-(phthalimid-1-ylmethyl)-spiro[5H-oxazolo[3,2-a]pyrazine-2(3H),4'-piperidin]-5-one

The procedure of Example 18, <Step 6> was repeated by using the compound (0.96 g) of Example 57, <Step 1>. After the reaction, the procedure of Example 1, <Step 4> was repeated to produce the title compound (0.98 g).
LC-MS (ESI⁺) : 814 (M⁺H, Rt = 5.85 min)

### <Step 2>

### Synthesis of (±)-7-[(1-benzenesulfonyl-5-chloroindol-2-yl)sulfonyl]tetrahydro-8a-(phthalimid-1-ylmethyl)-spiro[5H-oxazolo[3,2-a]pyrazine-2(3H),4'-piperidin]-5-one

The procedure of Example 1, <Step 5> was repeated by using the compound (0.98 g) of <Step 2> to produce the title compound (0.81 g).
LC-MS (ESI⁺) : 724 (M⁺H, Rt = 4.91 min)

### <Step 3>

### Synthesis of (±)-7-[(1-benzenesulfonyl-5-chloroindol-2-yl)sulfonyl]tetrahydro-8a-(phthalimid-1-ylmethyl)-1'-propionyl-spiro[5H-oxazolo[3,2-a]pyrazine-2(3H),4'-piperidin]-5-one

The procedure of Example 2, <Step 2> was repeated by using the compound (0.4 g) of <Step 3> to produce the title compound (0.38 g).
LC-MS (ESI⁺): 766 (M⁺H, Rt = 8.21 min)

### <Step 4>

### Synthesis of (±)-8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one

The procedure of Example 18, <Step 3> was repeated by using the compound (0.27 g) of <Step 4> to produce the title compound (0.14 g).

### Example 62

### Synthesis of optically active 8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one

The procedure of Example 21 was repeated by using the compound (0.11 g) of Example 61, <Step 5> to produce the title compound (first peak, 42 mg; second peak, 46 mg)).

### Example 63

### Synthesis of (±)-7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one

### <Step 1>

### Synthesis of tert-butyl (±)-tetrahydro-8a-[(N-2-nitrobenzenesulfonyl)-aminomethyl]-5-oxo-1'-(phenylmethyl)-spiro[5H-oxazolo[3,2-a]pyrazine-2(3H),4'-piperidine]-7-carboxylate

The procedure of Example 1, <Step 4> was repeated by using the compound (10.8 g) of Example 59, <Step 1> to produce the title compound (10.8 g).
LC-MS (ESI⁺): 615 (M⁺H, Rt = 3.88 min)

### <Step 2>

### Synthesis of tert-butyl (±)-tetrahydro-8a-((N-2-nitrobenzenesulfonyl)-N-methylaminomethyl]-5-oxo-1'-(phenylmethyl)-spiro[5H-oxazolo[3,2-a]pyrazine-2(3H),4'-piperidine]-7-carboxylate

The procedure of Example 18, <Step 4> was repeated by using the compound (10.7 g) of <Step 1> to produce the title compound (9.4 g).
LC-MS (ESI⁺) : 630 (M⁺H, Rt = 4.26 min)

### <Step 3>

### Synthesis of tert-butyl (±)-tetrahydro-8a-(N-methylaminomethyl)-5-oxo-1'-(phenylmethyl)-spiro[5H-oxazolo[3,2-a]pyrazine-2(3H),4'-piperidine]-7-carboxylate

The compound (8 g) of <Step 2> was dissolved in acetonitrile (120 ml), and cesium carbonate (8.3 g) and thiophenol (1.3 ml) were added in an ice bath. After stirring at room temperature for 3 hours, 0.5M aqueous solution of sodium hydroxide and methylene chloride were added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride → methylene chloride : methanol = 10 : 1) to produce the title compound (4.1 g).
LC-MS (ESI⁺) : 445 (M⁺H, Rt = 0.25 min)

### <Step 4>

### Synthesis of tert-butyl (±)-8a-[(N-ethoxycarboxymethyl)-N-methylaminomethyl]-tetrahydro-5-oxo-1'-(phenylmethyl)-spiro[5H-oxazolo[3,2-a]pyrazine-2(3H),4'-piperidine]-7-carboxylate

The compound (2.5 g) of <Step 3> was dissolved in acetonitrile (25 ml), and potassium carbonate (0.93 g), sodium iodide (42 mg), and ethyl bromoacetate (0.68 ml) were added in an ice bath. After stirring overnight at room temperature, water and methylene chloride were added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride → methylene chloride : methanol = 30: 1) to produce the title compound (2.6 g).
LC-MS (ESI⁺): 531 (M⁺H, Rt = 3.90 min)

### <Step 5>

### Synthesis of (±)-tetrahydro-8a-[(N-ethoxycarboxymethyl)-N-methylaminomethyl]-1'-(phenylmethyl)-spiro[5H-oxazolo[3,2-a]pyrazine-2(3H),4'-piperidin]-5-one

The procedure of Example 18, <Step 6> was repeated by using the compound (2.5 g) of <Step 4> to produce the title compound (2.1 g).
LC-MS (ESI⁺) : 431 (M⁺H, Rt = 0.25 min)

### <Step 6>

### Synthesis of (±)-7-[(1-benzenesulfonyl-5-chloroindol-2-yl)sulfonyl]tetrahydro-8a-[(N-ethoxycarboxymethyl)-N-methylaminomethyl]-1'-(phenylmethyl)-spiro[5H-oxazolo[3,2-a]pyrazine-2(3H),4'-piperidin]-5-one

The procedure of Example 1, <Step 4> was repeated by using the compound (1.1 g) of <Step 5> to produce the title compound (1.8 g).
LC-MS (ESI⁺) : 785 (M⁺H, Rt = 5.79 min)

### <Step 7>

### Synthesis of (±)-7-[(1-benzenesulfonyl-5-chloroindol-2-yl)sulfonyl]tetrahydro-8a-[(N-ethoxycarboxymethyl)-N-methylaminomethyl]-spiro[5H-oxazolo[3,2-a]pyrazine-2(3H),4'-piperidin]-5-one

The procedure of Example 1, <Step 5> was repeated by using the compound (1.2 g) of <Step 6> to produce the title compound (0.69 g).
LC-MS (ESI⁺) : 694 (M⁺H, Rt = 4.99 min)

### <Step 8>

### Synthesis of (±)-7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one

The procedure of Example 3 was repeated by using the compound (1 g) of <Step 7> to produce the title compound (0.73 g).

### Example 64

### Synthesis of optically active 7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one

The procedure of Example 21 was repeated by using the compound (0.15 g) of Example 61, <Step 5> to produce the title compound (first peak, 58 mg; second peak, 57 mg).

### Example 65

### Synthesis of (±)-N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine

The compound (0.35 g) of Example 63, <Step 8> was dissolved in methanol (5 ml), and 1M aqueous solution of sodium hydroxide (2.3 ml) was added. The mixture was stirred at room temperature for 2 hours, and 1M hydrochloric acid was added to adjust the pH to 4. Methylene chloride was added for extraction with methylene chloride, and the methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride to methylene chloride : methanol = 9 : 1) to produce the title compound (0.24 g).

### Example 66

### Synthesis of optically active N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine

The procedure of Example 21 was repeated by using the compound (0.15 g) of Example 65 to produce the title compound (first peak, 62 mg; second peak, 62 mg).

### Example 67

### Synthesis of (±)-N-[[(1'-acetyl)-7-[(5-chloro-1H-indol-2-yl)sulfonyl]tetrahydro-5-oxo-spiro[5H-oxazolo[3,2-a]pyrazine-2(3H),4'-piperidin]-8a-yl]methyl]-N-methylglycine

The procedure of Example 65 was repeated by using the compound (0.38 g) of Example 63, <Step 7> to produce the title compound (0.19.g).

### Example 68

### Synthesis of (±)-1-acetyl-8'-[(5-chloro-1H-indol-2-yl)sulfonyl]tetrahydro-9'a-(methoxymethyl)-spiro[piperidine-4,3'(4'H)-[2H]pyrazino[1,2-a]pyrimidin]-6' (7'H)-one

### <Step 1>

### Synthesis of phenylmethyl (±)-tetrahydro-9'a-(methoxymethyl)-6'(7'H)-oxo-1-(phenylmethyl)-spiro[piperidine-4,3'(4'H)-[2H]pyrazino[1,2-a]pyrimidin]-8'-carboxylate

The procedure of Example 18, <Step 1> was repeated by using 4,4-di(aminomethyl)-1-(phenylmethyl) piperidine (4.3 g) and N-(3-methoxy-2-oxopropyl)-N-[(phenylmethoxy) carbonyl]-glycine ethyl ester (5 g) to produce the title compound (6.3 g).
LC-MS (ESI⁺): 493 (M⁺H, Rt = 3.09 min)

### <Step 2>

### Synthesis of phenylmethyl (±)-tetrahydro-9'a-(methoxymethyl)-6'(7'H)-oxo-spiro[piperidine-4,3'(4'H)-[2H]pyrazino[1,2-a] pyrimidine]-8'-carboxylate

The procedure of Example 1, <Step 5> was repeated by using the compound (5 g) of <Step 1> to produce the title compound (3.2 g).
LC-MS (ESI⁺) : 403 (M⁺H, Rt = 1.40 min)

### <Step 3>

### Synthesis of phenylmethyl (±)-1-acetyl-tetrahydro-9'a-(methoxymethyl)-6'(7'H)-oxo-spiro[piperidine-4,3'(4'H)-[2H]pyrazino[1,2-a] pyrimidine]-8' carboxylate

The procedure of Example 2, <Step 2> was repeated by using the compound (0.4 g) of <Step 2> to produce the title compound (0.41 g).
LC-MS (ESI⁺) : 495 (M⁺H, Rt = 4.30 min)

### <Step 4>

### Synthesis of (±)-1-acetyl-tetrahydro-9'a-(methoxymethyl)-spiro[piperidine-4,3'(4'H)-[2H]pyrazino[1,2-a] pyrimidin]-6' (7' H) -one

The procedure of Example 35, <Step 3> was repeated by using the compound (0.41 g) of <Step 3> to produce the title compound (0.33 g).
LC-MS (ESI⁺): 311 (M⁺H, Rt = 0.22 min)

### <Step 5>

### Synthesis of (±)-1-acetyl-8'-[(5-chloro-1H-indol-2-yl)sulfonyl]tetrahydro-9'a-(methoxymethyl)-spiro[piperidine-4,3'(4'H)-[2H]pyrazino[1,2-a] pyrimidin]-6'(7'H)-one

The procedure of Example 1, <Step 4> was repeated by using the compound (0.13 g) of <Step 4>. After the reaction, the procedure of Example 1, <Step 7> was repeated to produce the title compound (0.15 g).

### Example 69

### Synthesis of (±)-1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one

### <Step 1>

### Synthesis of phenylmethyl (±)-tetrahydro-8a-(methoxymethyl)-5-oxo-l'-(phenylmethyl)-spiro[5H-thiazolo[3,2-a]pyrazine-2(3H),4]-7-carboxylate

4-Aminomethyl-1-(phenylmethyl)-4-piperidinethiol (2.5 g), N-(3-methoxy-2-oxopropyl)-N-[(phenylmethoxy) carbonyl]-glycine ethyl ester (1.5 g) which had been synthesized by a known synthesis method from 4-carboxy-4-(4-methoxy (phenylmethyl)thio)-1-(tert-butyl 7-carboxylate)piperidine which is a known compound (JP 8-500591 A) was dissolved in ethanol (30 ml), and after adding sodium acetate (0.76 g), the mixture was refluxed with heating for 2 hours. The solvent was removed under reduced pressure, and toluene (75 ml) was added to the residue. After adding p-toluenesulfonic acid monohydrate (catalytic amount), the mixure was refluxed with heating for 2 hours. After removing the solvent under reduced pressure, saturated aqueous solution of sodium hydrogencarbonate and methylene chloride were added to the residue for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography (elution solvent; methylene chloride : methanol = 50 : 1 to 25 : 1) to produce the title compound (2.4 g).

NMR data: DMSO-d₆, 100°C: 7.38-7.17(10H,m), 5.11 (2H,s), 4.60 (1H,d,J = 12Hz), 4.39 (1H,d,J = 13Hz), 4.22 (1H,d,J = 18Hz), 3.85 (1H,d,J = 18Hz), 3.57 (1H,d,J = 10Hz), 3.47 (3H,s), 3.42 (1H,d,J = 10Hz), 3.27 (1H,d,J = 13Hz), 3.22 (2H,s), 3.18 (1H,d,J = 12Hz), 2.65-2.40 (2H,m), 2.35-2.23 (1H, m), 2.18-2.07 (1H, m), 1.96-1.74 (2H,m), 1.70-1.61 (2H,m)

### <Step 2>

### Synthesis of phenylmethyl (±)-tetrahydro-8a-(methoxymethyl)-5-oxo-spiro[5H-thiazolo[3,2-a]pyrazine-2(3H),4'-piperidine]-7-carboxylate

The procedure of Example 1, <Step 5> was repeated by using the compound (1.5 g) of <Step 1> to produce the title compound (1.0 g).
LC-MS (ESI⁺) : 406 (M⁺H, Rt = 2.45 min)

### <Step 3>

### Synthesis of phenylmethyl (±)-1'-acetyl-tetrahydro-8a-(methoxymethyl)-5-oxo-spiro[5H-thiazolo[3,2-a]pyrazine-2(3H),4'-piperidine]-7-carboxylate

The procedure of Example 2, <Step 2> was repeated by using the compound (0.95 g) of <Step 2> to produce the title compound (1.0 g).
LC-MS (ESI⁺) : 448 (M⁺H, Rt = 5.61 min)

### <Step 4>

### Synthesis of (±)-1'-acetyl-tetrahydro-8a-(methoxymethyl)-spiro[5H-thiazolo[3,2-a]pyrazine-2(3H),4'-piperidin]-5-one

The procedure of Example 1, <Step 3> was repeated by using the compound (0.5 g) of <Step 3> to produce the title compound (0.29 g).
LC-MS (ESI⁺) : 314 (M⁺H, Rt = 0.33 min)

### <Step 5>

### Synthesis of (±)-1'-acetyl-7-[(1-benzenesulfonyl-5-chloroindol-2-yl)sulfonyl]tetrahydro-8a-(methoxymethyl)-spiro[5H-thiazolo[3,2-a]pyrazine-2(3H),4'-piperidin]-5-one

The procedure of Example 1, <Step 4> was repeated by using the compound (0.2 g) of <Step 4> to produce the title compound (0.43 g).
LC-MS (ESI⁺) : 667 (M⁺H, Rt = 8.23 min)

### <Step 6>

### Synthesis of (±)-1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one

The procedure of Example 1, <Step 7> was repeated by using the compound (0.15 g) of <Step 5> to produce the title compound (95 mg).

### Example 70

### Synthesis of optically active 1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one

The procedure of Example 21 was repeated by using the compound (0.80 mg) of Example 69 to produce the title compound (first peak, 34 mg; second peak, 33 mg).

### Example 71

### Synthesis of (±)-1'-acetyl-7-[(5-chloro-1H-indol-2-yl)sulfonyl]tetrahydro-8a-(methoxymethyl)-1,1-dioxide-spiro[5H-thiazolo[3,2-a]pyrazine-2(3H),4'-piperidin]-5-one

### <Step 1>

### Synthesis of (±)-1'-acetyl-7-[(1-benzenesulfonyl-5-chloroindol-2-yl)sulfonyl]tetrahydro-8a-(methoxymethyl)-1,1-dioxide-spiro[5H-thiazolo[3,2-a]pyrazine-2(3H),4'-piperidin]-5-one

The compound (0.2 g) of Example 69, <Step 5> was dissolved in methylene chloride (4 ml), and m-chloroperbenzoic acid (0.24 g) was added to the solution in an ice bath. After stirring the mixture at the same temperature for 15 minutes, the mixture was stirred overnight at room temperature. Saturated aqueous solution of sodium hydrogencarbonate was added to the reaction mixture in an ice bath. Then, methylene chloride was added for extraction with methylene chloride. The methylene chloride layer was washed with saturated aqueous solution of sodium chloride, and dried with anhydrous sodium sulfate. The solvent was removed under reduced pressure, and the resulting residue was purified by silica gel column chromatography [Chromatorex NHTM] (elution solvent; methylene chloride to methylene chloride : methanol = 4 : 1) to produce the title compound (0.2 g).
LC-MS (ESI⁺) : 699 (M⁺H, Rt = 8.17 min)

### <Step 2>

### Synthesis of (±)-1'-acetyl-7-[(5-chloro-1H-indol-2-yl)sulfonyl]tetrahydro-8a-(methoxymethyl)-1,1-dioxide-spiro[5H-thiazolo[3,2-a]pyrazine-2(3H),9'-piperidin]-5-one

The procedure of Example 1, <Step 7> was repeated by using the compound (0.15 g) of <Step 1> to produce the title compound (73 mg).

Table 4 shows the structures of the compounds of the present invention produced in the Examples as described above. Table 5 shows the physical data for the Examples.

[Table 4]

**Table 4-1**

| Example | 1 | (-)-form | | Example | 2 | (-)-form |
|---|---|---|---|---|---|---|
| 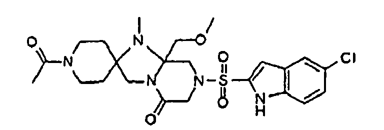 | | | | 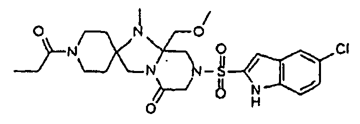 | | |
| | | | | | | |

| Example | 3 | (-)-form | | Example | 4 | (-)-form |
|---|---|---|---|---|---|---|
| 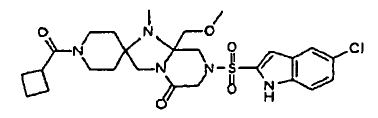 | | | | 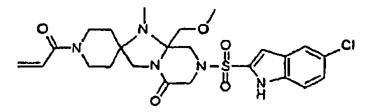 | | |
| | | | | | | |

| Example | 5 | (-)-form | | Example | 6 | (-)-torm |
|---|---|---|---|---|---|---|
| 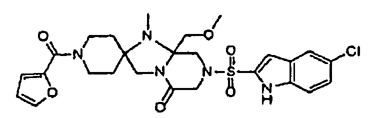 | | | | 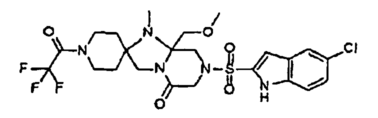 | | |
| | | | | | | |

| Example | 7 | (-)-form | | Example | 8 | (-)-form |
|---|---|---|---|---|---|---|
| 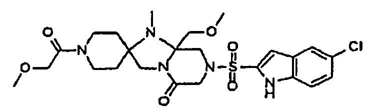 | | | | 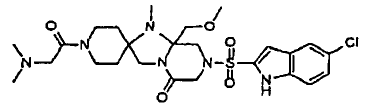 | | |
| | | | | | | |

| Example | 9 | (-)-form | | Example | 10 | (-)-form |
|---|---|---|---|---|---|---|
| 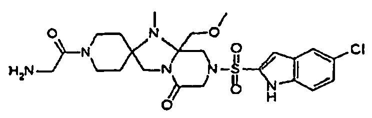 | | | | 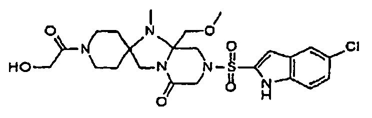 | | |
| | | | | | | |

| Example | 11 | (-)-form | | Example | 12 | (-)-form. |
|---|---|---|---|---|---|---|
| 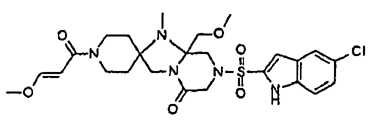 | | | | 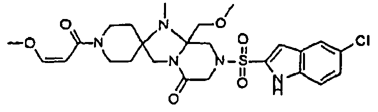 | | |

[Table 5]

**Table 4-2**

| Example | 13 | (-)-form | | Example | 14 | (-)-form |
|---|---|---|---|---|---|---|
| 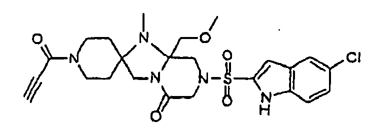 | | | | 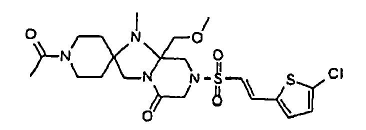 | | |
| | | | | | | |

| Example | 15 | (-)-form | | Example | 16 | (-)-form |
|---|---|---|---|---|---|---|
| 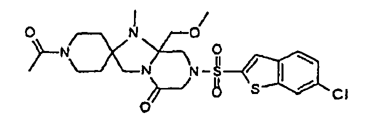 | | | | 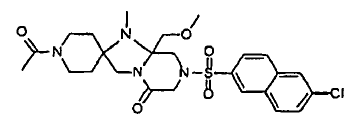 | | |
| | | | | | | |

| Example | 17 | (-)-form | | Example | 18 | racemate |
|---|---|---|---|---|---|---|
| 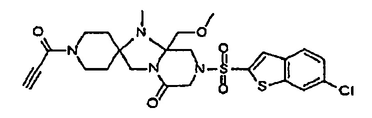 | | | | 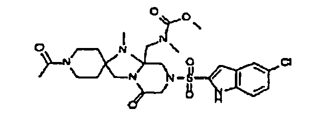 | | |
| | | | | | | |

| Example | 19 | racemate | | Example | 20 | racemate |
|---|---|---|---|---|---|---|
| 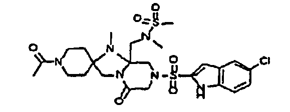 | | | | 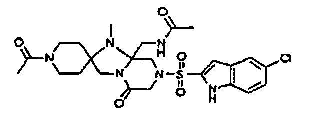 | | |
| | | | | | | |

| Example | 21 | (-)-form | | Example | 22 | (-)-form |
|---|---|---|---|---|---|---|
| 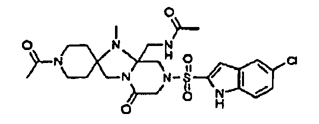 | | | | 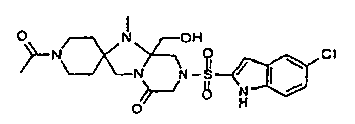 | | |
| | | | | | | |

| Example | 23 | (-)-form | | Example | 24 | racemate |
|---|---|---|---|---|---|---|
| 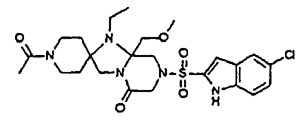 | | | | 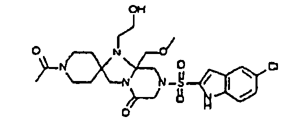 | | |

[Table 6]

**Table 4-3**

| Example | 25 | (-)-form | | Example | 26 | (-)-form |
|---|---|---|---|---|---|---|
| 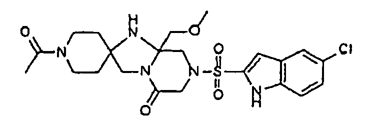 | | | | 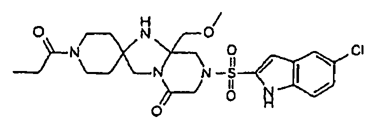 | | |
| | | | | | | |

| Example | 27 | (-)-form | | Example | 28 | (-)-form |
|---|---|---|---|---|---|---|
| 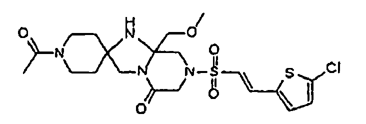 | | | | 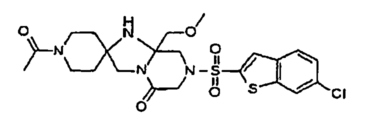 | | |
| | | | | | | |

| Example | 29 | racemate | | Example | 30 | (-)-form |
|---|---|---|---|---|---|---|
| 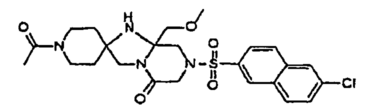 | | | | 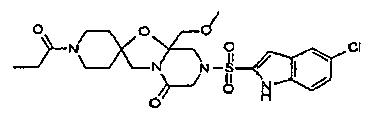 | | |
| | | | | | | |

| Example | 31 | (-)-form | | Example | 32 | (-)-form |
|---|---|---|---|---|---|---|
| 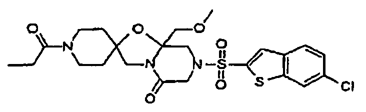 | | | | 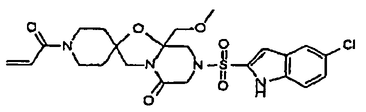 | | |
| | | | | | | |

| Example | 33 | (-)-form | | Example | 34 | (-)-form |
|---|---|---|---|---|---|---|
| 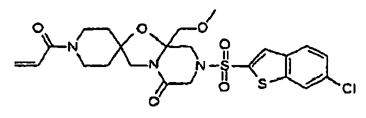 | | | | 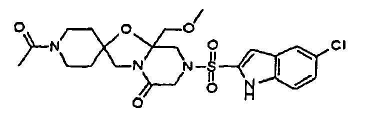 | | |
| | | | | | | |

| Example | 35 | (-)-form | | Example | 36 | (-)-form |
|---|---|---|---|---|---|---|
| 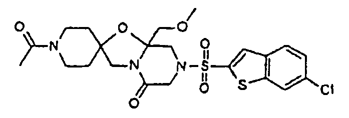 | | | | 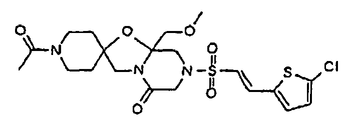 | | |

[Table 7]

**Table 4-4**

| Example | 37 | (-)-form | | Example | 38 | (-)-form |
|---|---|---|---|---|---|---|
| 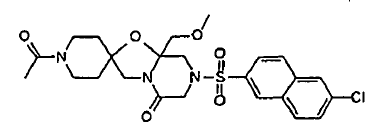 | | | | 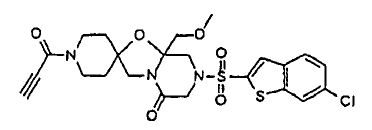 | | |
| | | | | | | |

| Example | 39 | (-)-form | | Example | 40 | (-)-form |
|---|---|---|---|---|---|---|
| 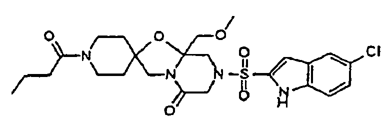 | | | | 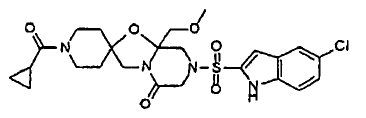 | | |
| | | | | | | |

| Example , | 41 | (-)-form | | Example | 42 | (-)-form |
|---|---|---|---|---|---|---|
| 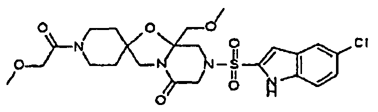 | | | | 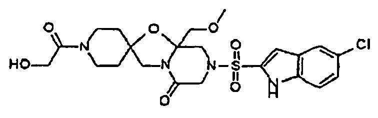 | | |
| | | | | | | |

| Example | 43 | (-)-form | | Example | 44 | (-)-form |
|---|---|---|---|---|---|---|
| 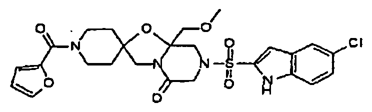 | | | | 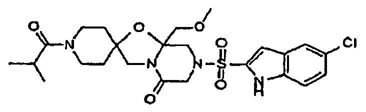 | | |
| | | | | | | |

| Example | 45 | (-)-form | | Example | 46 | (-)-form |
|---|---|---|---|---|---|---|
| 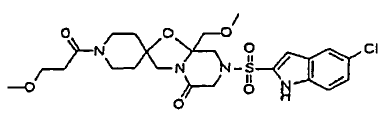 | | | | 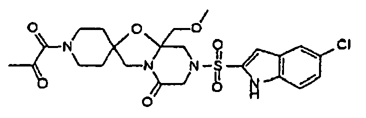 | | |
| | | | | | | |

| Example | 47 | (-)-form | | Example | 48 | racemate. |
|---|---|---|---|---|---|---|
| 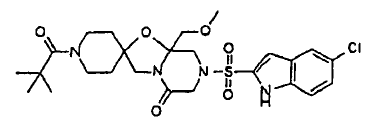 | | | | 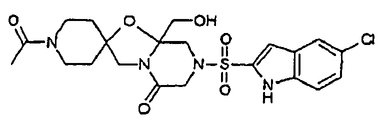 | | |

[Table 8]

**Table 4-5**

| Example | 49 | racemate | | Example | 50 | (-)-form |
|---|---|---|---|---|---|---|
| 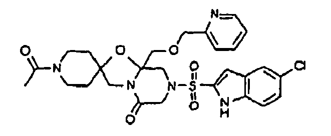 | | | | 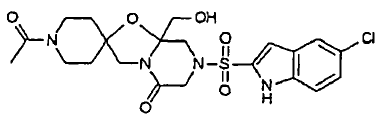 | | |
| | | | | | | |

| Example | 51 | (-)-form | | Example | 52 | (-)-form |
|---|---|---|---|---|---|---|
| 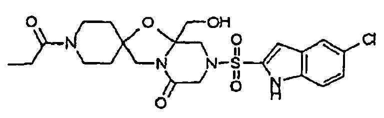 | | | | 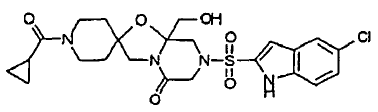 | | |
| | | | | | | |

| Example | 53 | (-)-form | | Example | 54 | (-)-form |
|---|---|---|---|---|---|---|
| 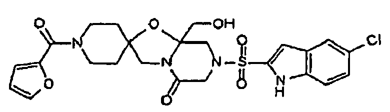 | | | | 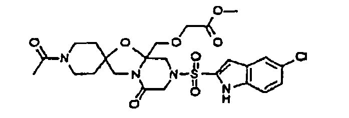 | | |
| | | | | | | |

| Example | 55 | (-)-form | | Example | 56 | (-)-form |
|---|---|---|---|---|---|---|
| 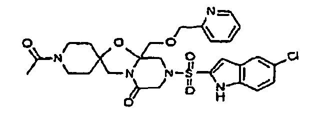 | | | | 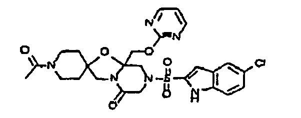 | | |
| | | | | | | |

| Example | 57 | racemate | | Example | 58 | (-)-form |
|---|---|---|---|---|---|---|
| 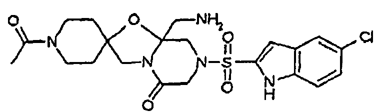 | | | | 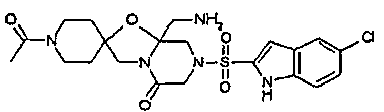 | | |
| | | | | | | |

| Example | 59 | racemate | | Example | 60 | (-)-form |
|---|---|---|---|---|---|---|
| 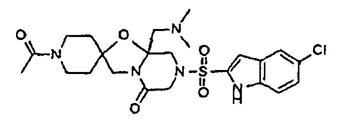 | | | | 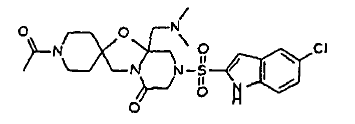 | | |

[Table 9]

**Table 4-6**

| Example | 61 | racemate | | Example | 62 | (-)-form |
|---|---|---|---|---|---|---|
| 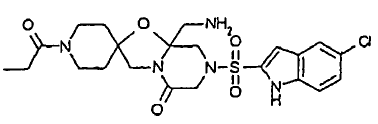 | | | | 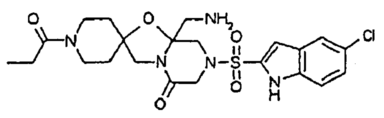 | | |
| | | | | | | |

| Example | 63 | racemate | | Example | 64 | (-)-form |
|---|---|---|---|---|---|---|
| 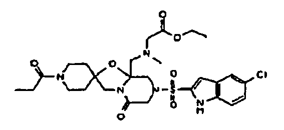 | | | | 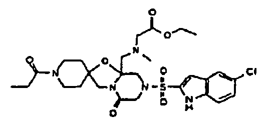 | | |
| | | | | | | |

| Example | 65 | racemate | | Example | 66 | (-)-form |
|---|---|---|---|---|---|---|
| 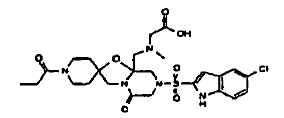 | | | | 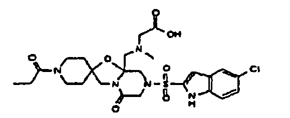 | | |
| | | | | | | |

| Example | 67 | racemate | | Example | 68 | racemate |
|---|---|---|---|---|---|---|
| 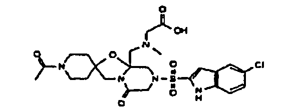 | | | | 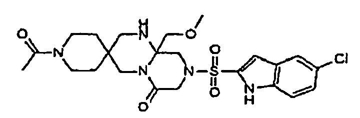 | | |
| | | | | | | |

| Example | 69 | racemate | | Example | 70 | (-)-form |
|---|---|---|---|---|---|---|
| 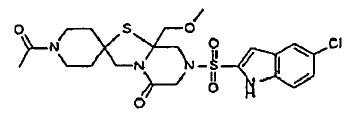 | | | | 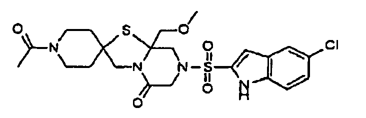 | | |
| | | | | | | |

| Example | 71 | racemate | | | | |
|---|---|---|---|---|---|---|
| 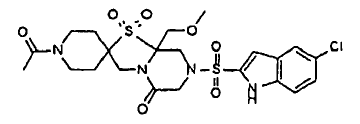 | | | | | | |

[Table 10]

**Table 5-1**

| Ex. No. | NMR(ppm) (300MHz. *:270MHz) |
|---|---|
| 1 | CDCl₃:9.30-9.15 (1H, brs), 7.69 (1H, s), 7.40 (1H, d, J=9Hz), 7.34 (1H, d, J=9Hz), 7.02 (1H. s). 4.76-4.56 (1H, m), 4.31 (1H, d, J=17Hz), 4.22-4.12 (2H, m), 3.91-3.56 (3H, m), 3.53-3.43 (1H, m), 3.36 (3H, s), 3.19 (1H, d, J=12Hz), 3.09-2.86 (1H, m), 2.54-2.27 (5H, m), 2.13-2.04 (3H, m), 1.89-1.50 (3H, m). 1.13-1.04 (1H, m) |
| 2 | DMSO-d₆:12.5 (1H, brs), 7.82-7.77 (1H. m), 7.50 (1H, d, J=9Hz), 7.33 (1H, dd, J=2, 9Hz), 7.11 (1H. s), 4.47-4.27 (1H. m), 4.17-4.02 (3H, m). 3.91-3.66 (1H, m), 3.59 (1H, d, J=10Hz). 3.50 (1H, d, J=16Hz), 3.44-3.28 (2H, m). 3.22 (3H, s), 3.07-2.96 (1H, m), 2.95-2.83 (1H, m), 2.57-2.36 (1H, m), 2.33-2.19 (3H, m), 2.29 (3H, s), 1.79-1.27 (3H, m), 1.13-1.12 (1H, m), 1.00-0.87 (3H, m) |
| 3 | DMSO-d₆:12.5 (1H, brs), 7.79 (1H, d, J=2Hz). 7.50 (1H, d, J=9Hz), 7.33 (1H, dd, J=2, 9Hz), 7.11 (1H, s), 4.43-4.23 (1H, m), 4.15-3.99 (3H, m), 3.72-3.48 (1 H, m), 3.58 (1H, d, J=10Hz), 3.49 (1H, d, J=17Hz), 3.38 (1H, d, J=10Hz), 3.43-3.16 (2H, m), 3.22 (3H, s), 3.04-2.94 (1H, m), 2.91-2.76 (1H, m), 2.61-1.78 (7H, m), 2.29 (3H, s), 1.77-1.48 (2H, m), 1.43-1.27 (1H, m), 1.13-0.97 (1H, m) |
| 4 | DMSO-d₆, 100°C*:12.1 (1H, brs), 7.74 (1H, d, J=2Hz), 7.49 (1H, d, J=9Hz), 7.29 (1H, dd, J=2, 9Hz), 7.08 (1H, s), 6.66 (1H, dd, J=11. 17Hz), 6.01 (1H, dd, J=2, 17Hz), 5.59 (1H, dd, J=2, 11Hz), 4.30-3.98 (5H, m), 3.57 (1H, d, J=10Hz), 3.55 (1H, d, J=17Hz), 3.38 (1H, d, J=10Hz), 3.22 (3H, s), 3.05 (1H, d, J=12Hz), 2.98-2.56 (2H, m), 2.56 (1H, d, J=12Hz), 2.33 (3H, s). 1.80-1.38 (3H, m), 1.14-1.01 (1H, m) |
| 5 | DMSO-d₆, 100°C:12.1 (1H, brs), 7.76-7.67 (2H, m), 7.49 (1H, d, J=9Hz), 7.29 (1H, dd, J=2, 9Hz), 7.07 (1H, d, J=1Hz), 6.88 (1H, d, J=1, 3Hz), 6.54 (1H, dd, J=2, 3Hz), 4.38-4.17 (2H, m), 4.16 (1H, d, J=11Hz), 4.10 (1 H, d, J=12Hz), 4.07 (1 H, d, J=16Hz), 3.57 (1H, d, J=10Hz), 3.55 (1H, d, J=16Hz), 3.39 (1H, d, J=10Hz), 3.22 (3H, s), 3.08 (1H, d. J=11Hz), 3.00-2.82 (2H, m), 2.56 (1H, d, J=12Hz), 2.35 (3H, s). 1.87-1.74 (1H, m), 1.69-1.50 (2H, m), 1.17-1.08 (1H, m) |
| 6 | DMSO-d₆*:12.5 (1H, s). 7.79 (1H, d, J=2Hz). 7.50 (1H, d, J=9Hz), 7.33 (1H, dd. J=2. 9Hz), 7.12 (1H, s), 4.36-4.01 (4H, m), 3.88-3.63 (1H, m). 3.60 (1H, d, J=10Hz), 3.55-3.43 (1H, m), 3.40 (1H, d, J=10Hz), 3.36-3.29 (1H, m), 3.29-3.13 (4H, m), 3.06 (1H, d, J=12Hz), 2.90-2.73 (1H, m), 2.31 (3H, s). 1.84-1.40 (3H, m), 1.27-1.15 (1H. m) |
| 7 | DMSO-d₆, 100°C*:12.1 (1H, brs), 7.77-7.72 (1H, m), 7.49 (1H, d, J=9Hz), 7.32-7.25 (1H, m), 7.07 (1H, s). 4.18-3.92 (5H, m), 4.01 (2H, s), 3.56 (1H, d, J=9Hz), 3.55 (1H, d, J=17Hz), 3.37 (1H, d, J=10Hz), 3.21 (3H. s). 3.18 (3H, s), 3.04 (1 H, d, J=12Hz), 2.83-2.60 (2H, m), 2.55 (1H, d, J=12Hz). 2.33 (3H, s), 1.82-1.39 (3H, m), 1.13-1.00 (1H, m) |
| 8 | DMSO-d₆:12.5 (1H, brs), 7.82-7.76 (1H, m). 7.50 (1H, d, J=9Hz), 7.36-7.28 (1H. m), 7.11 (1H, s), 4.42-4.21 (1H, m), 4.17-3.85 (4H, m), 3.59 (1H, d, J=10Hz), 3.55-3.45 (1H, m), 3.43-3.28 (1H, m). 3.23 (3H, s). 3.13-2.80 (4H, m), 2.60-2.36 (1H, m), 2.29 (3H, s), 2.21-2.06 (7H, m), 1.82-1.37 (3H, m), .1.15-1.02 (1H, m) |

[Table 11]

**Table 5-2**

| | |
|---|---|
| 9 | DMSO-d₆*:12.6 (1H, brs), 8.06 (2H, brs), 7.83-7.77 (1H, m), 7.51 (1H, d, J=9Hz), 7.33 (1H, dd. J=2, 9Hz), 7.14-7.08 (1H, m), 4.44-4.22 (1H, m), 4.18-4.02 (3H, m). 3:93-3.75 (2H, m), 3.73-3.57 (1H, m), 3.60 (1H, d, J=10Hz), 3.51 (1H, d, J=17Hz), 3.45-3.35 (1H, m), 3.23 (3H. s). 3.10-2.88 (2H. m). 2.67-2.42 (2H, m). 2.30 (3H, s), 1.86-1.47 (2H, m), 1.42-1.07 (2H, m) |
| 10 | DMSO-d₆*:12.5 (1H. brs), 7.79 (1H, d, J=2Hz). 7.50 (1H, d, J=9Hz), 7.33 (1H, dd. J=2, 9Hz). 7.1 (1H, s), 4.51-4.20 (2H, m), 4.17-3.91 (5H, m), 3.72-3.44 (1H, m). 3.59 (1H, d, J=10Hz), 3.50 (1H, d, J=16Hz), 3.43-3.17 (1H, m), 3.39 (1H, d, J=10Hz), 3.22 (3H, s). 3.01 (1H. d, J=12Hz), 2.95-2.77 (1H. m), 2.58-2.39 (1H. m). 2.30 (3H, s). 1.83-1.27 (3H, m), 1.15-1.01 (1H, m) |
| 11 | DMSO-d₆: 100°C*:12.1 (1H, brs), 7.77-7.70 (1H, m), 7.49 (1H, d, J=9Hz), 7.35 (0.5H, d, J=12Hz, trans form), 7.34-7.26 (1H, m), 7.07 (1H, s), 6.24 (0.5H, d, J=7Hz, cis form), 5.76 (0.5H, d, J=12Hz, trans form). 4.86 (0.5H, d, J=7Hz, cis form). 4.30-3.93 (5H, m), 3.70-3.48 (5H, m), 3.38 (1H. d, J=10Hz), 3.27-3.16 (1H, m). 3.21 (3H, s), 3.09-2.92 (1H, m), 2.78-2.60 (1H, m), 2.59-2.44 (1H, m), 2.33 (3H, s), 1.78-1.37 (3H, m), 1.13-0.97 (1H, m) |
| 12 | DMSO-d₆: 100°C*:12.0 (1H, brs), 7.76-7.70 (1H, m), 7.49 (1H, d, J=9Hz), 7.34-7.24 (1H, m), 7.07 (1H, s). 6.24 (1H, d, J=7Hz), 4.86 (1H, d, J=7Hz), 4.20-3.93 (5H, m), 3.63 (3H, s), 3.56 (1H, d. J=9Hz), 3.55 (1H, d, J=17Hz), 3.37 (1H, d, J=10Hz), 3.21 (3H. s), 3.03 (1H. d, J=12Hz). 2.76-2.58 (2H. m), 2.55 (1H, d, J=12Hz), 2.33 (3H, s). 1.77-1.37 (3H. m), 1.11-0.98 (1H. m) |
| 13 | DMSO-d₆:12.5 (1H, s), 7.79 (1H, s). 7.50 (1H, d, J=9Hz), 7.36-7.27 (1H, m), 7.10 (1H, s). 4.56-4.47 (1H, m). 4.38-3.99 (5H, m). 3.59 (1H. d, J=10Hz), 3.55-3.45 (1H, m), 3.43-3.27 (1H, m), 3.23 (3H, s), 3.16-2.95 (2H. m), 2.66-2.40 (2H, m), 2.30 (3H, s), 1.77-1.06 (4H, m) |
| 14 | CDCl₃*:7,48 (1H, dd, J=1, 15Hz). 7.10 (1H. d. J=4Hz), 6.93 (1H, d, J=4Hz), 6.29 (1H, d, J=15Hz), 4.78-4.59 (1H, m), 4.29-4.04 (3H, m), 3.92-3.53 (5H, m), 3.37 (3H, s), 3.27-2.88 (2H, m), 2.53 (1H, d, J=11Hz), 2.36 (3H, s), 2.13-2.07 (3H, m), 1.90-1.49 (3H, m), 1.27-1.14 (1H, m) |
| 15 | DMSO-d₆*:8.35 (1H, s), 8.19 (1H, s). 8.08 (1H, d, J=9Hz), 7.64-7.54 (1H, m), 4.43-3.98 (4H, m). 3.88-3.20 (4H, m), 3.28 (3H, s), 3.10-2.84 (2H, m). 2.71-2.21 (2H, m), 2.32 (3H, s), 2.02-1.88 (3H, m), 1.82-1.00 (4H, m) |
| 16 | DMSO-d₆*:8.61 (1H, s), 8.29 (1H, d, J=9Hz), 8.25 (1H, s), 8.17 (1H, d, J=9Hz), 7.92 (1H, d, J=9Hz), 7.80-7.67 (1H, m), 4.44-4.00 (4H, m), 3.87-3.18 (3H, m), 3.25 (3H, s), 3.08-2.82 (2H, m), 2.60-2.22 (3H, m), 2.31 (3H, s), 2.02-1.86 (3H, m), 1.82-1.19 (3H, m), 1.15-0.93 (1H, m) |
| 17 | DMSO-d₆, 100°C:8.24 (1 H. d, J=2Hz), 8.12 (1 H, s). 8.06 (1 H, d, J=9Hz), 7.54 (1H, dd, J=2, 9Hz), 4.40-4.01 (3H, m), 4.21 (1H, s), 4.16 (1H, d, J=12Hz), 4.08 (1H, d. J=17Hz), 3.69-3.56 (2H, m), 3.47 (1H, d, J=10Hz), 3.27 (3H, s), 3.22-2.87 (1H. m), 3.09 (1H, d, J=11Hz), 2.75-2.30 (1H, m), 2.68 (1H, d, J=12Hz), 2.36 (3H, s), 1.88-1.38 (3H, m), 1.24-1.09 (1H, m) |

[Table 12]

**Table 5-3**

| | |
|---|---|
| 18 | DMSO-d₆:7.78 (1 H, s), 7.50 (1H, d, J=9Hz), 7.31 (1H, d, J=9Hz), 7.09 (1H, s), 4.41-4.18 (3H, m), 4.11-3.99 (1H, m), 3.90-3.70 (2H, m). 3.66-2.22 (12H, m), 2.30 (3H, s), 2.00-1.56 (4H, m), 1.52-0.98 (3H, m) |
| 19 | DMSO-d₆:7.78 (1H, s), 7.49 (1H, d, J=9Hz), 7.31 (1H, dd, J=2, 9Hz), 7.09 (1H, s), 4.43-4.20 (2H, m), 3.98-3.23 (5H, m), 3.18-2.63 (7H, m), 2.86 (3H, s), 2.58-2.22 (1H, m), 2.30 (3H, s), 2.00-1.57 (4H, m), 1.54-1.02 (3H, m) |
| 20 | DMSO-d₆:12.5 (1H, s), 7.79 (2H, s), 7.50 (1H, d, J=9Hz), 7.37-7.28 (1H, m), 7.10 (1H, s). 4.45-4.12 (2H, m), 4.02 (1H, d, J=16Hz), 3.96-3.50 (2H, m), 3.88 (1H, d, J=12Hz), 3.56 (1H, d, J=16Hz), 3.26-3.10 (1H, m), 3.00-2.62 (3H, m), 2.44-2.25 (1H, m), 2.29 (3H, s), 2.00-1.91 (3H, m), 1.84-1.21 (3H, m). 1.81 (3H, s), 1.11-1.00 (1H, m) |
| 21 | DMSO-d6:12.5 (1H. s), 7.79 (2H, s), 7.50 (1H. d, J=9Hz), 7.37-7.28 (1H, m), 7.10 (1H, s), 4.45-4.12 (2H, m), 4.02 (1H, d, J=16Hz), 3.96-3.50 (2H, m), 3.88 (1H, d, J=12Hz), 3.56 (1H, d, J=16Hz), 3.26-3.10 (1H, m), 3.00-2.62 (3H, m), 2.44-2.25 (1H, m), 2.29 (3H, s), 2.00-1.91 (3H, m) 1.84-1.21 (3H, m) 1.81 (3H, s), 1.11-1.00 (1H, m) |
| 22 | DMSO-d₆, 100°C:7.75-7.71 (1H, m), 7.49 (1H, d, J=9Hz), 7.29 (1H, dd, J=2, 9Hz), 7.07-7.03 (1H, m), 4.11 (1H, d, J=11Hz), 4.06-3.96 (2H, m), 3.60 (1 H, d, J=11Hz), 3.54 (1H, d, J=17Hz), 3.52 (1 H, d, J=11Hz), 3.06 (1H, d, J=11Hz), 3.12-2.85 (3H, m), 2.58 (1H, d, J=11Hz), 2.54-2.40 (1H, m), 2.34 (3H, s), 1.94 (3H, s), 1.77-1.57 (2H, m), 1.56-1.40 (1H, m), 1.08-0.97 (1H, m) |
| 23 | DMSO-d₆*:12.5 (1H, brs), 7.82-7.75 (1H, m), 7.49 (1H, d, J=9Hz), 7.32 (1H, dd, J=2, 9Hz), 7.10 (1H, s). 4.43-4.21 (1H, m). 4.15-3.97 (3H, m), 3.84-3.57 (1H, m), 3.52 (1H, d, J=11Hz), 3.51 (1 H, d, J=16Hz), 3.43-3.33 (1H, m), 3.22-3.13 (1H, m), 3.19 (3H, s), 3.03-2.87 (2H, m), 2.80-2.65 (1H, m), 2.52-2.28 (2H, m), 1.98-1.88 (3H, m). 1.85-1.20 (3H, m), 1.16-1.04 (1H, m), 0.99 (3H, t. J=7Hz) |
| 24 | CDCl₃:10.55 (1 H. s), 7.70-7.65 (1H, m), 7.40 (1H, dd, J=4, 9Hz), 7.34-7.27 (1H, m), 7.02 (1H, s), 4.73-4.50 (1H, m), 4.37-4.18 (3H, m), 3.88-3.44 (5H, m), 3.38-3.20 (4H, m), 3.18-2.80 (4H, m), 2.52-2.28 (2H, m), 2.12-2.04 (3H, m), 1.89-1.63 (2H, m), 1.50-1.41 (1H, m), 1.17-1.02 (1H, m) |
| 25 | DMSO-d₆, 100°C:12.1 (1H, brs), 7.74 (1H, d, J=2Hz), 7.49 (1H, d, J=8Hz), 7.29 (1H, dd, J=2, 8Hz), 7.06 (1H, s), 4.05 (1H, d, J=17Hz), 3.97-3.90 (1H, m), 3.95 (1H, d, J=11 Hz), 3.56-3.17 (5H, m), 3.55 (1 H, d, J=17Hz), 3.47 (1H, d, J=10Hz), 3.25 (3H, s), 3.02-2.87 (1H, m), 2.74 (1H, brs). 2.56 (1H. d, J=12Hz), 1.93 (3H. s), 1.75-1.50 (2H, m), 1.33-1.20 (2H, m) |
| 26 | CDCl₃:9.64-9.32 (1H, m). 7.70 (1H, s). 7.40-7.28 (2H, m), 7.05-6.98 (1H, m), 4.32 (1H. d, J=17Hz), 4.17 (1H, d, J=11Hz), 4.15-4.06 (1H, m), 3.73-3.25 (10H, m). 2.97 (1H, d, J=12Hz), 2.47 (1H, brs), 2.41-2.22 (3H, m). 1.84-1.55 (2H, m), 1.38-1.23 (2H, m), 1.19-1.05 (3H, m) |

[Table 13]

**Table 5-4**

| | |
|---|---|
| 27 | DMSO-d₆, 100°C:7.51 (1H, dd, J=1, 15Hz), 7.44 (1H, d, J=4Hz), 7.13 (1H, d, J=4Hz), 6.92 (1H, d, J=15Hz), 4.01 (1H, d, J=12Hz), 3.92 (1H, d, J=17Hz), 3.80 (1H, d, J=12Hz), 3.69 (1H, d, J=17Hz), 3.54 (1H, d, J=10Hz), 3.50-3.30 (6H, m), 3.32 (3H, s), 2.78-2.68 (2H, m), 1.95 (3H, s), 1.75-1.55 (2H, m), 1.40-1.33 (2H, m) |
| 28 | CDCI₃:7.92-7.80 (3H, m), 7.51-7.44 (1H, m), 4.32 (1H, d, J=17Hz), 4.22-4.11 (2H, m), 3.75-3.23 (7H, m), 3.44 (3H, s), 2.98 (1H, d, J=12Hz), 2.50-2.30 (2H, m), 2.10-2.02 (3H, m), 1.86-1.50 (2H, m), 1.38-1.20 (2H, m) |
| 29 | DMSO-d₆. 100°C:8.51 (1H, s), 8.23 (1H, d, J=9Hz), 8.16 (1H, s), 8.13 (1H, d. J=9Hz), 7.84 (1H, d, J=9Hz), 7.67 (1H, d, J=9Hz), 4.06 (1H, d, J=17Hz), 3.95 (1H, d, J=12Hz), 3.94 (1H, d, J=12Hz), 3.55-3.15 (7H, m). 3.25 (3H, s), 3.03-2.85 (1H, m), 2.77-2.65 (1H, brs), 2.62-2.40 (1H, m), 1.92 (3H, s), 1.72-1.48 (2H, m), 1.33-1.16 (2H, m) |
| 30 | DMSO-d₆:12.5 (1H. s), 7.83-7.78 (1H, m), 7.51 (1H, d, J=9Hz), 7.37-7.32 (1H, m), 7.12 (1H, s), 4.17-3.98 (3H, m), 3.71-3.07 (4H, m), 3.58 (1H, d, J=16Hz), 3.54 (1H, d, J=10Hz), 3.43 (1H, d, J=10Hz), 3.29 (3H, s), 3.18-3.07 (1H, m), 2.68 (1H, d, J=11Hz), 2.36-2.19 (2H, m), 1.80-1.61 (2H, m). 1.52-1.29 (2H, m), 1.00-0.87 (3H, m) |
| 31 | DMSO-d₆, 100°C*:8.25 (1H, d. J=2Hz), 8.13 (1H, s), 8.06 (1H, d. J=9Hz), 7.55 (1H. dd. J=2, 9Hz), 4.11 (1H, d, J=12Hz), 4.15-4.02 (1H, m), 4.08 (1H, d, J=16Hz), 3.69 (1H. d, J=16Hz), 3.68-3.21 (4H, m), 3.57 (1H, d, J=11Hz), 3.46 (1 H, d, J=11Hz), 3.32 (3H, s), 3.16 (1H, d, J=12Hz), 2.84 (1H, d, J=12Hz), 2.27 (2H, q. J=7Hz), 1.81-1.68 (2H, m). 1.53-1.35 (2H, m), 0.99 (3H. t, J=7Hz) |
| 32 | DMSO-d₆:12.5 (1H, s), 7.83-7.77 (1H. m), 7.51 (1H, d, J=9Hz), 7.34 (1H, dd, J=2, 9Hz), 7.12 (1H, s), 6.87-6.69 (1H, m), 6.12-6.02 (1H, m), 5.70-5.59 (1H, m), 4.17-3.98 (3H, m). 3.79-3.07 (8H, m), 3.29 (3H, s), 2.75-2.63 (1H, m), 1.83-1.63 (2H, m), 1.54-1.31 (2H, m) |
| 33 | DMSO-d₆, 100°C*:8.24 (1H, d, J=2Hz), 8.12 (1H, s), 8.05 (1H, d, J=9Hz), 7.54 (1H, dd. J=2, 9Hz), 6.66 (1H, dd, J=11, 17Hz). 6.00 (1H, dd, J=2, 17Hz), 5.59 (1H, dd, J=2, 11Hz), 4.15-4.00 (3H, m), 3.73-3.25 (4H, m), 3.68 (1H, d, J=16Hz), 3.56 (1H, d, J=11 Hz), 3.46 (1 H. d, J=11Hz). 3.31 (3H, s), 3.16 (1H. d. J=12Hz), 2.84 (1H, d, J=12Hz), 1.83-1.70 (2H, m), 1.55-1.37 (2H, m) |
| 34 | DMSO-d₆, 100°C:12.1 (1H, brs), 7.74 (1H, d, J=2Hz), 7.50 (1H. d, J=9Hz), 7.30 (1H, dd, J=2. 9Hz). 7.08 (1H, s), 4.14-4.02 (3H, m), 3.62 (1H. d, J=17Hz), 3.59-3.20 (4H, m), 3.51 (1H, d, J=17Hz), 3.40 (1H, d, J=10Hz), 3.27 (3H, s), 3.14 (1H, d, J=12Hz), 2.73 (1H, d, J=12Hz), 1.95 (3H, s), 1,79-1.68 (2H, m), 1.50-1.35 (2H, m) |
| 35 | DMSO-d₆, 100°C*:8.24 (1H, d, J=2Hz), 8.12 (1H, s), 8.05 (1H, d, J=9Hz), 7.54 (1H, dd, J=2, 9Hz), 4.12-4.05 (1H, m), 4.10 (1H, d, J=12Hz), 4.07 (1H, d, J=16Hz), 3.68 (1H, d, J=16Hz), 3.63-3,20 (4H, m), 3.56 (1H, d, J=11Hz), 3.45 (1H, d. J=11 Hz), 3.31 (3H, s), 3.15 (1H, d, J=12Hz), 2.83 (1H, d, J=12Hz), 1.95 (3H, s), 1.80-1.68 (2H, m), 1.54-1.35 (2H, m) |

[Table 14]

**Table 5-5**

| | |
|---|---|
| 36 | DMSO-d₆, 100°C*:7.53 (1H, d, J=16Hz), 7.44 (1H, d, J=4Hz). 7.14 (1H, d, J=4Hz), 6.92 (1H, d, J=16Hz), 4.14 (1H, d. J=12Hz), 4.00-3.89 (2H, m), 3.75 (1H, d, J =17Hz), 3.64-3.24 (4H, m), 3.56 (1H, d, J=11Hz), 3.46 (1H, d, J=11Hz), 3.33 (3H, s), 3.16 (1H, d, J =12 Hz), 2.85 (1H, d, J=12Hz), 1.97 (3H, s), 1.80-1.68 (2H, m), 1.56-1.42 (2H, m) |
| 37 | DMSO-d₆, 100°C*:8.57-8.47 (1H, m), 8.23 (1H, d, J=9Hz), 8.20-8.13 (1H, m), 8.13 (1H, d, J=9Hz), 7.86 (1H, dd, J=2. 9Hz), 7.67 (1H, dd, J=2, 9Hz), 4.12 (1H, d, J=12Hz), 4.08 (1H, d, J=12Hz), 4.07 (1H, d, J=16Hz), 3.68-3.14 (4H, m), 3.58 (1H, d, J=16Hz), 3.51 (1H, d, J=1 1Hz), 3.42 (1 H, d, J=11 Hz), 3.27 (3H, s), 3.13 (1 H, d, J=12Hz), 2.72 (1H, d, J=12Hz), 1.94 (3H, s), 1.80-1.63 (2H, m), 1.50-1.30 (2H, m) |
| 38 | DMSO-d₆, 100°C*:8.25 (1H, d, J=2Hz). 8.13 (1H, s). 8.06 (1H, d, J=9Hz), 7.55 (1H, dd. J=2, 9Hz). 4.22 (1H, s), 4.17-4.03 (1H, m), 4.13 (1H, d, J=12Hz). 4.10 (1H, d, J=11Hz), 3.96-3.10 (4H, m), 3.68 (1H, d, J=16Hz), 3.57 (1H, d, J=11Hz), 3.47 (1H, d, J=11Hz), 3.32 (3H, s), 3.18 (1H, d, J=11 Hz), 2.85 (1H, d, J=12Hz). 1.92-1.68 (2H, m), 1.63-1.36 (2H, m) |
| 39 | CDCl₃:9.39-9.18 (1H, m), 7.71 (1H, s), 7.44-7.32 (2H, m), 7.06-7.01 (1H, m), 4.37-4.25 (2H, m), 4.23-4.15 (1H, m), 4.02-3.86 (1H, m). 3.68-3.50 (4H, m), 3.48-3.35 (4H, m). 3.31-3.15 (2H, m), 2.43-2.20 (3H, m), 1.99-1.55 (4H, m), 1.51-1.36 (2H, m), 1.00-0.91 (3H, m) |
| 40 | CDCl₃:9.61-9.29 (1H. m), 7.70 (1H, s), 7.44-7.30 (2H, m), 7.06-7.01 (1H, m), 4.36-4.16 (3H. m), 4.01-3.79 (2H, m), 3.77-3.37 (4H, m), 3.43 (3H, s), 3.33-3.15 (2H, m), 2.45-2.28 (1H, m), 2.05-1.36 (5H, m), 1.08-0.88 (2H, m), 0.85-0.66 (2H, m) |
| 41 | CDCl₃:9.60-9.32 (1H, m), 7.73-7.67 (1H, m). 7.41 (1H, d, J=9Hz), 7.38-7.30 (1H, m), 7.06-7.01 (1H, m), 4.37-4.24 (2H, m), 4.23-4.04 (3H, m), 4.03-3.87 (1H, m), 3.67-3.34 (7H, m), 3.42 (3H, s), 3.39 (1H, d, J=12Hz), 3.30-3.17 (2H, m), 2.37 (1H, d, J=72Hz), 2.00-1.68 (2H. m), 1.53-1.37 (2H, m) |
| 42 | CDCl₃:9.32-9.20 (1H, m). 7.71 (1H, s), 7,41 (1H, d, J=9Hz), 7.39-7.33 (1H, m), 7.04 (1H, s). 4.38-4.24 (2H, m), 4.23-3.99 (4H, m), 3.67-3.56 (3H, m), 3.54-3.35 (5H, m), 3.34-3.14 (2H, m), 3.24 (1H, d, J=12Hz). 2.37 (1H, d, J=12Hz), 2.02-1.87 (1H, m), 1.85-1.70 (1H, m), 1.55-1.40 (2H, m) |
| 43 | DMSO-d₆, 100°C*:12.1 (1H, brs), 7.77-7.68 (2H, m), 7.51 (1H, d, J=9Hz), 7.30 (1H, dd, J=2, 9Hz), 7.09 (1H, s), 6.89 (1H, d, J=3Hz), 6.57-6.52 (1H, m), 4.17-4.03 (3H, m), 3.89-3.36 (4H, m), 3.63 (1H, d, J=16Hz), 3.53 (1H, d, J=11Hz). 3.42 (1H, d, J=11Hz), 3.28 (3H, s), 3.18 (1H, d. J=12Hz), 2.76 (1H, d, J=12Hz), 1.88-1.77 (2H, m), 1.58-1.47 (2H, m) |
| 44 | DMSO-d₆, 1 D0°C*:12.1 (1H, brs), 7.75 (1H, d, J=2Hz), 7.51 (1H. d, J=9Hz), 7.30 (1H, dd. J=2, 9Hz), 7.09 (1H, s), 4.14-4.02 (3H, m), 3.67-3.20 (4H, m). 3.63 (1H, d, J=17Hz), 3.52 (1H, d, J=11Hz), 3.41 (1H, d, J=11 Hz), 3.28 (3H, s). 3.15 (1H, d, J=12Hz), 2.90-2.70 (1H, m), 2.74 (1H, d, J=12Hz). 1.78-1.70 (2H, m), 1.49-1.37 (2H, m), 1.00 (3H, s), 0.98 (3H, s) |

[Table 15]

**Table 5-6**

| | |
|---|---|
| 45 | DMSO-d₆, 100°C*:12.1 (1H, brs), 7.78-7.72 (1H, m), 7.51 (1H, d, J=9Hz), 7.34-7.24 (1H, m), 7.09 (1H, s), 4.16-4.02 (3H, m), 3.69-3.19 (7H, m), 3.63 (1H, d. J=17Hz), 3.41 (1H, d, J=11Hz). 3.27 (3H, s), 3.22 (3H, s). 3,15 (1H, d, J=12Hz). 2.73 (1H. d, J=12Hz), 2.57-2.40 (2H, m), 1.80-1.68 (2H, m), 1.48-1,37 (2H, m) |
| 46 | DMSO-d₆, 100°C*:12.4 (1H. brs), 7.76-7.71 (1H. m), 7.50 (1H, d, J=9Hz). 7.32-7.24 (1H. m), 7.07 (1H. s). 4.16-4.03 (3H, m), 3.81-3.12 (4H, m), 3.63 (1H, d, J=16Hz), 3.52 (1H, d, J=1 1Hz), 3.42 (1 H, d, J=11Hz), 3.28 (3H, s), 3.17 (1H, d, J=12Hz), 2.79-2.70 (1H, m), 2.32 (3H, s), 1.85-1.75 (2H, m), 1.57-1.46 (2H, m) |
| 47 | DMSO-d₆, 100°C*:12.1 (1H, brs), 7.74 (1H, d, J=2Hz), 7.50 (1H, d, J=9Hz), 7.29 (1H, dd, J=2, 9Hz), 7.10-7.05 (1H, m). 4.15-4.02 (1H, m), 4.10 (1H, d, J=12Hz), 4.07 (1H, d, J=17Hz), 3.71-3.24 (4H, m), 3.61 (1H, d, J=17Hz), 3.51 (1H, d, J=11Hz), 3,40 (1H, d, J=11Hz), 3.27 (3H, s), 3.14 (1H, d, J=12Hz), 2.72 (1H, d, J=12Hz), 1.78-1.68 (2H, m), 1.46-1.37 (2H, m), 1.16 (9H, s) |
| 48 | DMSO-d₆, 100°C:7.77-7.72 (1H, m), 7.51 (1H, d, J=9Hz), 7.30 (1H, dd, J=2, 9Hz), 7.08 (1H, d, J=1Hz), 4.07 (1H, d, J=11Hz), 4.05 (1H, d, J=11Hz), 4.00 (1 H, d, J=16Hz), 3.63 (1 H, d, J=16Hz), 3.54 (1 H, d, J=12Hz), 3.50 (1H, d, J=12Hz), 3.19 (1H, d, J=11Hz), 3.68-3.12 (4H, m), 2.76 (1H, d, J=12Hz), 1.96 (3H, s), 1.88-1.65 (2H, m), 1.51-1.33 (2H, m) |
| 49 | DMSO-d₆, 100°C:12.1 (1H, brs), 8.52-8.45 (1H. m), 7.75 (1H, dt, J=2, 8Hz), 7.72 (1H, d, J=2Hz), 7.49 (1H, d, J=9Hz), 7.37 (1H, d, J=8Hz). 7.31-7.19 (2H, m), 7.08 (1H, s), 4.58 (2H, s), 4.17 (1H, d, J=12Hz). 4.09 (1H, d, J=11 Hz), 4.07 (1H, d, J=17Hz), 3.75-3.11 (4H, m), 3.72 (1H, d, J=11Hz), 3.63 (1 H, d, J=17Hz), 3.61 (1H, d, J=11Hz), 3.17 (1H, d, J=11Hz), 2.78 (1H, d, J=11Hz), 1.94 (3H, s), 1.76-1.68 (2H, m), 1.48-1.40 (2H, m) |
| 50 | DMSO-d₆, 100°C:7.73 (1H, d, J=2Hz), 7.49 (1H, d. J=9Hz), 7.27 (1H, dd, J=2. 9Hz). 7.07-7.04 (1H, m). 4.10-3.94 (3H. m), 3.62 (1H, d, J=16Hz), 3.60-3.33 (3H, m), 3.53 (1H, d, J=12Hz), 3.48 (1H, d, J=12Hz), 3.29-3.12 (1H, m), 3.16 (1H, d, J=12Hz), 2.74 (1H, d, J=12Hz), 1.94 (3H, s), 1.83-1.67 (2H, m). 1.48-1.34 (2H, m) |
| 51 | DMSO-d₆, 100°C:7.74 (1H, d, J=2Hz), 7.49 (7 H, d, J=9Hz), 7.29 (1H, dd. J=2. 9Hz). 7.07 (1H, s), 4.10-3.93 (3H, m). 3.62 (1H, d. J=17Hz), 3.60-3.34 (4H, m), 3.51 (1H, d, J=7Hz), 3.30-3.18 (1H. m), 3.16 (1H, d, J=11Hz), 2.75 (1H, d, J=12Hz), 2.26 (2H, d, J=7Hz), 1.82-1.66 (2H, m). 1.47-1.33 (2H, m), 0.98 (3H, t, J=7Hz) |
| 52 | DMSO-d₆, 100°C:7.74-7.71 (1H, m), 7.49 (1H, d, J=9Hz), 7.27 (1H. dd, J=2. 9Hz). 7.06 (1 H, d, J=1Hz), 4.11-3.95 (3H, m), 3.74-3.45 (4H. m). 3.62 (1H, d, J=16Hz), 3.51 (1H, d, J=7Hz), 3.40-3.30 (1H, m), 3.19 (1H, d, J=5Hz), 3.17 (1H, d, J=1 1 Hz). 2.76 (1 H, d, J=12Hz), 1.90-1.73 (3H, m). 1.47-1.38 (2H. m). 0.74-0.62 (4H, m) |
| 53 | DMSO-d₆, 100°C:7.77-7.67 (2H, m), 7.50 (1H, d, J=9Hz), 7.29 (1H. dd, J=2. 9Hz). 7.08 (1H, s), 6.90-6.84 (1H, m), 6.54 (1H, dd, J=2, 4Hz), 4.13-3.95 (3H, m), 3.86-3.68 (2H, m), 3.62 (1H, d, J=16Hz), 3.61-3.36 (3H. m). 3.53 (1H, d, J=7Hz), 3.19 (1H, d, J=11Hz), 2.77 (1H, d, J=12Hz), 1.92-1.76 (2H, m). 1.57-1.43 (2H, m) |

[Table 16]

**Table 5-7**

| | |
|---|---|
| 54 | DMSO-d₆, 100°C:7.73 (1H, d, J=2Hz), 7.49 (1H, d, J=9Hz), 7.28 (1H, dd. J=2, 9Hz), 7.07 (1H. d, J=1Hz), 4.17-4.01 (5H, m), 3.71-3.18 (7H, m), 3.65 (3H, s), 3.18 (1H, d, J=12Hz), 2.76 (1H, d, J=12Hz), 1.95 (3H, s), 1.81-1.72 (2H, m), 1.48-1.38 (2H. m) |
| 55 | DMSO-d₆, 100°C:12.1 (1H, brs), 8.52-8.45 (1H, m), 7.75 (1H, dt, J=2, 8Hz), 7.72 (1H, d, J=2Hz), 7.49 (1H, d, J=9Hz), 7.37 (1H, d, J=8Hz), 7.31-7.19 (2H, m). 7.08 (1H, s). 4.58 (2H, s), 4.17 (1H, d, J=12Hz), 4.09 (1H, d, J=11 Hz), 4.07 (1 H, d, J=17Hz), 3.75-3.11 (4H, m), 3.72 (1H, d, J=11 Hz), 3.63 (1H, d, J=17Hz), 3.61 (1H, d, J=11Hz) 3.17 (1H, d, J=11Hz). 2.78 (1H, d, J=1 1Hz), 1.94 (3H, s). 1.76-1.68 (2H, m), 1.48-1.40 (2H, m) |
| 56 | DMSO-d₆, 100°C:8.53 (2H, d, J=5Hz), 7.69 (1H, d, J=2Hz), 7.47 (1H. d, J=9Hz). 7.26 (1H, dd, J=2. 9Hz). 7.10 (1H, t. J=5Hz), 7.07 (1H, s), 4.63 (1H, d, J=12Hz), 4.40 (1H. d, J=11 Hz), 4.23 (1 H, d, J=12Hz), 4.14 (1H, d, J=17Hz), 4.13 (1H, d, J=11Hz). 3.68 (1H, d, J=17Hz), 3.57-3.38 (2H. m). 3.32-3.16 (2H, m). 3.23 (1H, d, J=12Hz), 2.88 (1H, d, J=12Hz), 1.93 (3H, s). 1.77-1.67 (2H, m), 1.55-1.38 (2H, m) |
| 57 | DMSO-d₆, 100°C*:7.73 (1H, d, J=2Hz), 7.50 (1H, d, J=9Hz), 7.28 (1H. dd, J=2, 9Hz), 7.07 (1H, s), 4.20 (1H. d, J=12Hz), 4.11-3.98 (1H, m), 4.09 (1H, d, J=12Hz), 3.65-2.60 (4H, m), 3.61 (1H, d, J=16Hz), 3.17 (1H. d, J=12Hz), 2.78 (2H, s), 2.66 (1H, d, J=12Hz), 1.95 (3H, s). 1.80-1.65 (2H, m), 1.50-1.33 (2H, m) |
| 58 | DMSO-d₆, 100°C*:7.74 (1H, d, J=2Hz), 7.50 (1H, d, J=9Hz), 7.29 (1H, dd, J=2,9Hz), 7.10-7.05 (1H, m), 4.24-4.00 (2H, m), 4.09 (1H, d, J=12Hz), 3.67-2.60 (6H, m), 3.61 (1H, d, J=l6Hz), 3.17 (1H, d, J=12Hz), 2.67 (1H, d, J=12Hz), 1.95 (3H, s). 1.79-1.65 (2H, m), 1.47-1.36 (2H, m) |
| 59 | DMSO-d₆, 100°C*:12.2 (1H, brs), 7.79-7.70 (1H, m), 7.49 (1H, d, J=9Hz), 7.33-7.23 (1H, m), 7.07 (1H, s), 4.39-4.29 (1H, m), 4.16-3.99 (2H, m), 3.62-2.32 (8H, m), 3.56 (1H, d, J=16Hz), 2.27 (6H, s), 1.94 (3H, s), 1.80-1.67 (2H, m), 1.48-1.31(2H, m) |
| 60 | OMSO-d₆. 100°C*:12.2 (1H, brs), 7.79-7.70 (1H, m), 7.49 (1H, d, J=9Hz), 7.33-7.23 (1H, m), 7.07 (1H, s), 4.39-4.29 (1H, m), 4.16-3.99 (2H, m), 3.62-2.32 (8H, m), 3.56 (1H, d, J=16Hz). 2.27 (6H, s), 1.94 (3H, s), 1.80-1.67 (2H, m), 1.48-1.31 (2H, m) |
| 61 | DMSO-d₆. 100°C*:7.74 (1H, d. J=2Hz), 7.50 (1H, d, J=9Hz), 7.29 (1H, dd, J=2, 9Hz), 7.10-7.04 (1H, m), 4.20 (1H, d, J=12Hz), 4.09 (1H, d, J=16Hz), 4.05 (1H, d, J=16Hz), 3.68-2.61 (4H, m), 3.61 (1H, d, J=16Hz), 3.17 (1H, d, J=12Hz), 2.78 (2H, s), 2.67 (1H, d, J=12Hz), 2.26 (2H, q. J=7Hz), 1.80-1.64 (2H, m), 1.50-1.31 (2H, m), 0.98 (3H, t, J=7Hz) |
| 62 | DMSO-d₆, 100°C*:7.74 (1H, d, J=2Hz), 7.50 (1H, d, J=9Hz), 7.29 (1H, dd, J=2, 9Hz), 7.10-7.04 (1H. m), 4.20 (1H, d, J=12Hz), 4.09 (1H, d, J=16Hz), 4.05 (1H. d, J=16Hz), 3.68-2.61 (4H, m), 3.61 (1H, d, J=16Hz), 3.17 (1H, d, J=12Hz), 2.78 (2H, s), 2.67 (1H. d, J=12Hz), 2.26 (2H, q. J=7Hz), 1.80-1.64 (2H, m), 1.50-1.31 (2H, m), 0.98 (3H, t, J=7Hz) |

[Table 17]

**Table 5-8**

| | |
|---|---|
| 63 | DMSO-d₆. 100°C:12.2 (1H, brs), 7.77-7.73 (1H, m), 7.51 (1H, d, J=9Hz), 7.34-7.27 (1H, m), 7.09 (1H, s), 4.31 (1H, d, J=12Hz), 4.17-4.03 (4H, m), 3.58 (1H, d, J=17Hz), 3.50-3.21 (4H, m), 3.43 (2H, s), 3.18 (1H, d, J=12Hz), 2.88 (2H, s), 2.58 (1H, d, J=12Hz), 2.50 (3H, s), 2.27 (2H, q, J=7Hz), 1.85-1.67 (2H, m), 1.46-1.34 (2H, m), 1.22 (3H, t, J=7Hz). 0.99 (3H, t, J=7Hz) |
| 64 | DMSO-d₆, 100°C:12.2 (1H, brs), 7.77-7.73 (1H, m), 7.51 (1H, d, J=9Hz), 7.34-7.27 (1H, m), 7.09 (1H, s), 4.31 (1H, d, J=12Hz), 4.17-4.03 (4H, m), 3.58 (1H, d, J=17Hz), 3.50-3.21 (4H, m), 3.43 (2H, s), 3.18 (1H, d, J=12Hz), 2.88 (2H, s), 2.58 (1H, d, J=12Hz), 2.50 (3H, s), 2.27 (2H. q. J=7Hz), 1.85-1,67 (2H. m), 1.46-1.34 (2H, m), 1.22 (3H, t, J=7Hz), 0.99 (3H, t, J=7Hz) |
| 65 | DMSO-d₆, 100°C:7.72 (1H, s), 7.50 (1H, d, J=9Hz), 7.27 (1H, d, J=9Hz), 7.06 (1H, s). 4.32 (1H. d, J=11 Hz). 4.11 (1 H. d, J=12Hz), 4.03 (1H, d, J=16Hz), 3.64-3.12 (7H. m), 3.57 (1H. d, J=16Hz), 2.85 (2H. s), 2.56 (1H, d, J=11Hz), 2.45 (3H, s), 2.25 (2H, q. J=7Hz), 1.80-1.69 (2H. m), 1.45-1.30 (2H. m). 0.97 (3H, t, J=7Hz) |
| 66 | DMSO-d₆, 100°C:7.72 (1H, s), 7.50 (1H. d, J=9Hz), 7.27 (1H, d, J=9Hz), 7.06 (1H. s), 4.32 (1H, d, J=11Hz), 4.1 (1 H, d, J=12Hz), 4.03 (1H, d, J=16Hz), 3,64-3.12 (7H. m), 3.57 (1H, d, J=16Hz), 2.85 (2H, s), 2.56 (1H, d, J=11 Hz), 2.45 (3H, s), 2.25 (2H, q, J=7Hz), 1.80-1.69 (2H, m), 1.45-1.30 (2H, m), 0.97 (3H, t. J=7Hz) |
| 67 | DMSO-d₆, 100°C:7.74 (1H, d, J=2Hz), 7.50 (1H, d, J=9Hz), 7.29 (1H, dd, J=2, 9Hz), 7.08 (1H, s), 4.31 (1H, d, J=11 Hz), 4.15-4.00 (2H, m), 3.60-3.13 (7H, m), 3.55 (1H, d, J=16Hz), 2.90 (1H, d, J=14Hz). 2.84 (1H, d, J=14Hz), 2.56 (1H, d,J=11Hz), 2.49 (3H, s), 1.94 (3H, s), 1.79-1.69 (2H, m), 1.44-1.33 (2H, m) |
| 68 | DMSO-d₆. 100°C*:12.2 (1H, brs), 7.75 (1H, s), 7.50 (1H, d, J=9Hz). 7.35-7.25 (1H, m), 7.06 (1H, s), 4.35 (1H, d, J=14Hz), 3.89 (1H, d, J=16Hz), 3.79 (1H, d, J=13Hz), 3.71 (1H, d, J=11Hz), 3.54-3.12 (5H, m), 3.53 (1H, d, J=16Hz). 3.29 (3H, s), 2.82-2.44 (4H, m), 1.92 (3H, s), 1.40-1.20 (4H, m) |
| 69 | DMSO-d₆, 100°C:12.1 (1H. brs), 7.75 (1H, d, J=2Hz), 7.51 (1H. d, J=9Hz), 7.30 (1H, dd, J=2, 9Hz), 7.09 (1H, s), 4.59 (1H, d, J=12Hz). 4.18-4.03 (2H, m), 3.88-3.48 (3H, m), 3.74 (1H, d, J=9Hz), 3.67 (1 H, d, J=17Hz), 3.29 (3H, s), 3.24 (1H, d, J=13Hz), 3.15-2.90 (3H, m), 1.95 (3H, s), 1.85-1.75 (2H, m), 1.65-1.45 (2H, m) |
| 70 | DMSO-d₆, 100°C:12.1 (1H, brs), 7.75 (1H, d, J=2Hz), 7.51 (1H, d, J=9Hz), 7.30 (1H, dd, J=2, 9Hz), 7.09 (1H, s), 4.59 (1H, d, J=12Hz), 4.18-4.03 (2H, m), 3.88-3.48 (3H, m). 3.74 (1H, d, J=9Hz), 3.67 (1H, d, J=17Hz), 3.29 (3H, s), 3.24 (1H, d, J=13Hz), 3.15-2.90 (3H, m), 1.95 (3H, s). 1.85-1.75 (2H, m), 1.65-1.45 (2H, m) |
| 71 | DMSO-d₆, 100°C:12.2(1H, brs), 7.77 (1H, d. J=2Hz), 7.52 (1H, d, J=9Hz). 7.32 (1H, d, J=2, 9Hz), 7.12 (1H, s), 4.81 (1H, d, J=14Hz), 4.18-4.06 (2H, m), 3.83 (1H, d, J=17Hz), 3.80 (1H, d, J=11Hz), 3.75 (1H, d, J=11Hz), 3.70-3.55 (2H, m), 3.45-3.20 (4H, m), 3.26 (3H, s). 2.02-1.73 (2H, m), 1.97 (3H, s), 1.59-1.47 (2H, m) |

## Claims

1. A compound represented by the following formula (I) or its pharmaceutically acceptable salt: 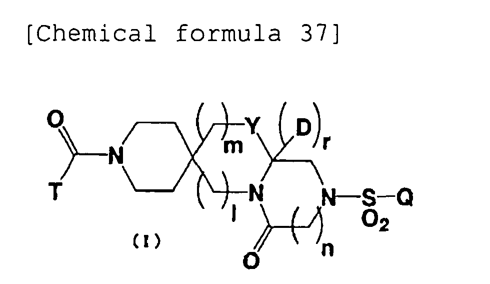 wherein T is an optionally substituted aliphatic hydrocarbon group, an optionally substituted 3- to 4-membered alicyclic hydrocarbon group, an optionally substituted C₂₋₆ alkanoyl group or trifluoromethyl group; D is hydrogen atom, -CO-R⁵ (wherein R⁵ is hydrogen atom or a substituent), or an optionally substituted C₁₋₆ alkyl group; Y is oxygen atom, -S(O)_{y}-, or optionally substituted imino group (-NH-); Q is a hydrocarbon group or a heterocyclic group which optionally has a substituent; 1, m, n, and y are independently an integer selected from 0, 1, and 2 with the proviso that 1 and m are not simultaneously 0; and r is an integer of 0 or 1; and wherein the three rings including the N-containing ring having the T-CO- bonded thereto, the Y-containing ring, and the N-containing ring having the - SO₂-Q bonded thereto are optionally substituted.

2. The following compounds, or a (+) or (-) optical isomer or a pharmaceutically acceptable salt thereof:
(-)-1'-acetyl-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1-methyl-1'-propanoyl-spiro[imidazo[1,2-a]pyrazine-2(3H),4'-piperidin]-5(1H)-one;
(-)-7-[(5-chloro-1H-indol-2-yl)sulfonyl]-1'-cyclobutanecarbonyl-tetrahydro-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1-methyl-1'-propenoyl-spiro[imidazo[1,2-a]pyrazine-2(3H),4'-piperidin]-5(1H)-one;
(-)-7-[(5-chloro-1H-indol-2-yl)sulfonyl]-1'-(trifluoroacetyl)-tetrahydro-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-1'-(methoxyacetyl)-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1-methyl-1'-(dimethylaminoacetyl)-spiro[imidazo[1,2-a]pyrazine-2(3H),4'-piperidin]-5(1H)-one;
(-)-1'-(aminoacetyl)-7-[(5-chloro-1H-indol-2-yl)sulfonyl]tetrahydro-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-1'-(hydroxyacetyl)-8a-(methoxymethyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1'-(2-methoxypropenoyl)-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1'-[(1Z)-(2-methoxypropenoyl)]-1-methyl-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1-methyl-1'-propinoyl-spiro[imidazo[1,2-a]pyrazine-2(3H),4'-piperidin]-5(1H)-one;
(-)-1'-acetyl-7-[[(1E)-2-(5-chloro-2-thienyl)ethenyl]sulfonyl]tetrahydro-8a-(methoxymethyl)-1-methyl-spiro[imidazo[1,2-a]pyrazine-2(3H),4'-piperidin]-5(1H)-one;
(-)-1'-acetyl-7-[(6-chloro-benzo[b]thiophen-2-yl)sulfonyl]tetrahydro-8a-(methoxymethyl)-1-methyl-spiro[imidazo[1,2-a]pyrazine-2(3H),4'-piperidin]-5(1H)-one;
(-)-1'-acetyl-7-[(6-chloronaphthalen-2-yl)sulfonyl]tetrahydro-8a-(methoxymethyl)-1-methyl-spiro[imidazo[1,2-a]pyrazine-2(3H),9'-piperidin]-5(1H)-one;
(-)-7-[(6-chloro-benzo[b]thiophen-2-yl)sulfonyl]tetrahydro-8a-(methoxymethyl)-1-methyl-1'-propinoyl-spiro[imidazo[1,2-a]pyrazine-2(3H),4'-piperidin]-5(1H)-one;
(±)-1'-acetyl-7-[(5-chloro-1H-indol-2-yl)sulfonyl]tetrahydro-8a-(N-methoxycarbonyl-N-methylaminomethyl)-1-methyl-spiro[imidazo[1,2-a]pyrazine-2(3H),4'-piperidin]-5(1H)-5(1H)-one;
(±)-1'-acetyl-7-[(5-chloro-1H-indol-2-yl)sulfonyl]tetrahydro-8a-(N-methanesulfonyl-N-methylaminomethyl)-1-methyl-spiro[imidazo[1,2-a]pyrazine-2(3H),4'-piperidin]-5(1H)-5(1H)-one;
(±)-1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one;
optically active 1'-acetyl-8a-(acetylaminomethyl)-7-[(5-chloro-1H-indol-2-yl)sulfonyl]tetrahydro-1-methyl-spiro[imidazo[1,2-a]pyrazine-2(3H),4'-piperidin]-5(1H)-one;
(-)-1'-acetyl-7-[(5-chloro-1H-indol-2-yl)sulfonyl]tetrahydro-8a-(hydroxymethyl)-1-methyl-spiro[imidazo[1,2-a]pyrazine-2(3H),4'-piperidin]-5(1H)-one;
(-)-1'-acetyl-7-[(5-chloro-1H-indol-2-yl)sulfonyl]tetrahydro-1-ethyl-8a-(methoxymethyl)-spiro[imidazo[1,2-a]pyrazine-2(3H),4'-piperidin]-5(1H)-one;
(±)-1'-acetyl-7-[(5-chloro-1H-indol-2-yl)sulfonyl]tetrahydro-1-(hydroxyethyl)-8a-(methoxymethyl)-spiro[imidazo[1,2-a]pyrazine-2(3H),4'-piperidin]-5(1H)-one;
(-)-1'-acetyl-7-[(5-chloro-1H-indol-2-yl)sulfonyl]tetrahydro-8a-(methoxymethyl)-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1'-propanoyl-spiro[imidazo[1,2-a]pyrazine-2(3H),4'-piperidin]-5(1H)-one;
(-)-1'-acetyl-7-[[(1E)-2-(5-chloro-2-thienyl)ethenyl]sulfonyl]tetrahydro-8a-(methoxymethyl)-spiro[imidazo[1,2-a]pyrazine-2(3H),4'-piperidin]-5(1H)-one;
(-)-1'-acetyl-7-[(6-chloro-benzo[b]thiophen-2-yl)sulfonyl]tetrahydro-8a-(methoxymethyl)-spiro[imidazo[1,2-a]pyrazine-2(3H),4'-piperidin]-5(1H)-one;
(±)-1'-acetyl-7-[(6-chloronaphthalen-2-yl)sulfonyl]tetrahydro-8a-(methoxymethyl)-spiro[imidazo[1,2-a]pyrazine-2(3H),4'-piperidin]-5(1H)-one;
(-)-7-[(5-chloro-1H-indol-2-yl)sulfonyl]tetrahydro-8a-(methoxymethyl)-1'-propanoyl-spiro[5H-oxazolo[3,2-a]pyrazine-2(3H),4'-piperidin]-5-one;
(-)-7-[(6-chloro-benzo[b]thiophen-2-yl)sulfonyl]tetrahydro-8a-(methoxymethyl)-1'-propanoyl-spiro[5H-oxazolo[3,2-a]pyrazine-2(3H),4'-piperidin]-5-one;
(-)-7-[(5-chloro-1H-indol-2-yl)sulfonyl]tetrahydro-8a-(methoxymethyl)-1'-propenoyl-spiro[5H-oxazolo[3,2-a]pyrazine-2(3H),4'-piperidin]-5-one;
(-)-7-[(6-chloro-benzo[b]thiophen-2-yl)sulfonyl]tetrahydro-8a-(methoxymethyl)-1'-propenoyl-spiro[5H-oxazolo[3,2-a]pyrazine-2(3H),4'-piperidin]-5-one;
(-)-1'-acetyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one;
(-)-1'-acetyl-7-[(6-chloro-benzo[b]thiophen-2-yl)sulfonyl]tetrahydro-8a-(methoxymethyl)-spiro[5H-oxazolo[3,2-a]pyrazine-2(3H),4'-piperidin]-5-one;
(-)-1'-acetyl-7-[[(1E)-2-(5-chloro-2-thienyl)ethenyl]sulfonyl]tetrahydro-8a-(methoxymethyl)-spiro[5H-oxazolo[3,2-a]pyrazine-2(3H),4'-piperidin]-5-one;
(-)-1'-acetyl-7-[(6-chloronaphthalen-2-yl)sulfonyl]tetrahydro-8a-(methoxymethyl)-spiro[5H-oxazolo[3,2-a]pyrazine-2(3H),4'-piperidin]-5-one;
(-)-7-[(6-chloro-benzo[b]thiophen-2-yl)sulfonyl]tetrahydro-8a-(methoxymethyl)-1'-propinoyl-spiro[5H-oxazolo[3,2-a]pyrazine-2(3H),4'-piperidin]-5-one;
(-)-1'-n-butanoyl-7-[(5-chloro-1H-indol-2-yl)sulfonyl]tetrahydro-8a-(methoxymethyl)-spiro[5H-oxazolo[3,2-a]pyrazine-2(3H),4'-piperidin]-5-one;
(-)-7-[(5-chloro-1H-indol-2-yl)sulfonyl]tetrahydro-1'-cyclopropanoyl-8a-(methoxymethyl)-spiro[5H-oxazolo[3,2-a]pyrazine-2(3H),4'-piperidin]-5-one;
(-)-7-[(5-chloro-1H-indol-2-yl)sulfonyl]tetrahydro-1'-(methoxyacetyl)-8a-(methoxymethyl)-spiro[5H-oxazolo[3,2-a]pyrazine-2(3H),4'-piperidin]-5-one;
(-)-7-[(5-chloro-1H-indol-2-yl)sulfonyl]tetrahydro-1'-(hydroxyacetyl)-8a-(methoxymethyl)-spiro[5H-oxazolo[3,2-a]pyrazine-2(3H),4'-piperidin]-5-one;
(-)-7-[(5-chloro-1H-indol-2-yl)sulfonyl]tetrahydro-8a-(methoxymethyl)-1'-(2-methylpropanoyl)-spiro[5H-oxazolo[3,2-a]pyrazine-2(3H),4'-piperidin]-5-one;
(-)-7-[(5-chloro-1H-indol-2-yl)sulfonyl]tetrahydro-8a-(methoxymethyl)-1'-(3-methoxypropanoyl)-spiro[5H-oxazolo[3,2-a]pyrazine-2(3H),4'-piperidin]-5-one;
(-)-7-[(5-chloro-1H-indol-2-yl)sulfonyl]tetrahydro-8a-(methoxymethyl)-1'-(2-oxopropanoyl)-spiro[5H-oxazolo[3,2-a]pyrazine-2(3H),4'-piperidin]-5-one;
(-)-7-[(5-chloro-1H-indol-2-yl)sulfonyl]tetrahydro-8a-(methoxymethyl)-1'-(2,2-dimethylpropanoyl)-spiro[5H-oxazolo[3,2-a]pyrazine-2(3H),4'-piperidin]-5-one;
(±)-1'-acetyl-7-[(5-chloro-1H-indol-2-yl)sulfonyl]tetrahydro-8a-(hydroxymethyl)-spiro[5H-oxazolo[3,2-a]pyrazine-2(3H),9'-piperidin]-5-one;
(±)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one;
(-)-1'-acetyl-7-[(5-chloro-1H-indol-2-yl)sulfonyl]tetrahydro-8a-(hydroxymethyl)-spiro[5H-oxazolo[3,2-a]pyrazine-2(3H),4'-piperidin]-5-one;
(-)-7-[(5-chloro-1H-indol-2-yl)sulfonyl]tetrahydro-8a-(hydroxymethyl)-1'-propanoyl-spiro[5H-oxazolo[3,2-a]pyrazine-2(3H),4'-piperidin]-5-one;
(-)-7-[(5-chloro-1H-indol-2-yl)sulfonyl]tetrahydro-1'-cyclopropanoyl-8a-(hydroxymethyl)-spiro[5H-oxazolo[3,2-a]pyrazine-2(3H),4'-piperidin]-5-one;
(-)-1'-acetyl-7-[(5-chloro-1H-indol-2-yl)sulfonyl]tetrahydro-8a-(methoxycarbonylmethoxymethyl)-spiro[5H-oxazolo[3,2-a]pyrazine-2(3H),4'-piperidin]-5-one;
(-)-1'-acetyl-7-[(5-chloro-1H-indol-2-yl)sulfonyl]tetrahydro-8a-(2-pyridylmethoxymethyl)-spiro[5H-oxazolo[3,2-a]pyrazine-2(3H),4'-piperidin]-5-one;
(-)-1'-acetyl-7-[(5-chloro-1H-indol-2-yl)sulfonyl]tetrahydro-8a-(2-pyrimidyloxymethyl)-spiro[5H-oxazolo[3,2-a]pyrazine-2(3H),4'-piperidin]-5-one;
(±)-1'-acetyl-8a-(aminomethyl)-7-[(5-chloro-1H-indol-2-yl)sulfonyl]tetrahydro-spiro[5H-oxazolo[3,2-a]pyrazine-2(3H),4'-piperidin]-5-one;
(-)-1'-acetyl-8a-(aminomethyl)-7-[(5-chloro-1H-indol-2-yl)sulfonyl]tetrahydro-spiro[5H-oxazolo[3,2-a]pyrazine-2(3H),4'-piperidin]-5-one;
(±)-1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),4'-piperidin]-5-one;
optically active 1'-acetyl-7-[(5-chloro-1H-indol-2-yl)sulfonyl]tetrahydro-8a-(N,N-dimethylaminomethyl)-spiro[5H-oxazolo[3,2-a]pyrazine-2(3H),4'-piperidin]-5-one;
(±)-8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one;
optically active 8a-(aminomethyl)-7-[(5-chloro-1H-indol-2-yl)sulfonyl]tetrahydro-1'-propanoyl-spiro[5H-oxazolo[3,2-a]pyrazine-2(3H),4'-piperidin]-5-one;
(±)-7-[(5-chloro-1H-indol-2-yl)sulfonyl]tetrahydro-8a-[(N-ethoxycarboxymethyl)-N-methylaminomethyl]-1'-propanoyl-spiro[5H-ox_azolo[3,2-a]pyrazine-2(3H),4'-piperidin]-5-one;
optically active 7-[(5-chloro-1H-indol-2-yl)sulfonyl]tetrahydro-8a-[(N-ethoxycarboxymethyl)-N-methylaminomethyl]-1'-propanoyl-spiro[5H-oxazolo[3,2-a]pyrazine-2(3H),4'-piperidin]-5-one;
(±)-N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine;
optically active N-[[7-[(5-chloro-1H-indol-2-yl)sulfonyl]tetrahydro-5-oxo-1'-propanoyl-spiro[5H-oxazolo[3,2-a]pyrazine-2(3H),4'-piperidin]-8a-yl]methyl]-N-methylglycine;
(±)-N-[[(1'-acetyl)-7-[(5-chloro-1H-indol-2-yl)sulfonyl]tetrahydro-5-oxo-spiro[5H-oxazolo[3,2-a]pyrazine-2(3H),4'-piperidin]-8a-yl]methyl]-N-methylglycine;
(±)-1-acetyl-8'-[(5-chloro-1H-indol-2-yl)sulfonyl]tetrahydro-9'a-(methoxymethyl)-spiro[piperidine-4,3' (4'H)-[2H]pyrazino[1,2-a] pyrimidin]-6'(7'H)-one;
(±)-1'-acetyl-7-[(5-chloro-1H-indol-2-yl)sulfonyl]tetrahydro-8a-(methoxymethyl)-spiro[5H-thiazolo[3,2-a]pyrazine-2(3H),4'-piperidin]-5-one;
optically active 1'-acetyl-7-[(5-chloro-1H-indol-2-yl)sulfonyl]tetrahydro-8a-(methoxymethyl)-spiro[5H-thiazolo[3,2-a]pyrazine-2(3H),9'-piperidin]-5-one; and
(±)-1'-acetyl-7-[(5-chloro-1H-indol-2-yl)sulfonyl]tetrahydro-8a-(methoxymethyl)-1,1-dioxide-spiro[5H-thiazolo[3,2-a]pyrazine-2(3H),4'-piperidin]-5-one.

3. A pharmaceutical composition comprising a compound represented by the formula (I) as described in calim 1 or its pharmaceutically acceptable salt as its effective component.

4. A FXa inhibitor comprising a compound represented by the formula (I) as described in calim 1 or its pharmaceutically acceptable salt as its effective component.
